# EUROPEAN PATENT APPLICATION

(11) **EP 3 483 181 A1**
(43) Date of publication of application: **15.05.2019**
(21) Application number: 17823636.0
(22) Date of filing: 05.07.2017
(51) Int. Cl.: C07K 16/28, C07K 16/24, A61K 39/00, A61K 39/395, C12N 15/12

(54) **BLYS ANTIBODY, PREPARATION METHOD THEREFOR AND APPLICATION THEREOF**

(30) Priority: 06.07.2016 CN 201610527996
(71) Applicant: Shanghai Pharmaexplorer Co., Ltd., Shanghai 201210 (CN)
(72) Inventor: CAO, Xiaodan, Shanghai 201210 (CN); HU, Yingying, Shanghai 201210 (CN); REN, Fang, Shanghai 201210 (CN); GONG, Shiyong, Shanghai 201210 (CN); GONG, Jing, Shanghai 201210 (CN); LV, Qiang, Shanghai 201210 (CN); GU, Hongzhuan, Shanghai 201210 (CN); SHI, Beilei, Shanghai 201210 (CN); SHAO, Xiaohui, Shanghai 201210 (CN); LV, Xiaofen, Shanghai 201210 (CN); LEUNG, Stewart, Shanghai 201210 (CN); LIU, Lile, Shanghai 201210 (CN)
(74) Representative: Gulde & Partner
(86) International application number: PCT/CN2017/091839
(87) International publication number: WO 2018/006824

(57) **Abstract**

A BLyS antibody, preparation method therefor and application thereof, the BLyS antibody comprising one or more of a heavy chain CDR1, a heavy chain CDR2 and a heavy chain CDR3 of a heavy chain variable region of the BLyS antibody, and/or one or more of a light chain CDR1, a light chain CDR2 and a light chain CDR3 of a light chain variable region of the BLyS antibody. An amino acid sequence of the antibody is shown in a sequence listing. The BLyS antibody has a high affinity, and may observably and effectively seal a BLyS protein at a protein level and a cell level, and prevent the BLyS protein from binding to a receptor. The BLyS antibody lacks a cross-reaction with same protein antigens such as human APRIL, and enjoys good biological activity. The BLyS antibody may inhibit the proliferation of human BLyS-induced mouse B cells and may therefore be used for preparing a drug for preventing or treating diseases associated with BLyS expression or dysfunction.

## Description

This application claims priority of Chinese Patent Application No. 201610527996.0 filed on July 6, 2016. The entire content of the aforementioned application is hereby incorporated by reference.

### Field of invention

The present invention relates to the antibody, and more specifically, relates to BlyS antibody, preparation method therefor and use thereof.

### Prior arts

Autoimmune disease is a disease that the body's own normal organs, tissues and cells are attacked by immune system. As a chronic disease, it is incurable but controllable, and the body will become very sick once people suffering from the disease. This leads to a sharp reduction in the quality of life and the high medical cost will result in a heavy burden on patients and their families, and even the society. Common autoimmune diseases include systemic lupus erythematosus (SLE), rheumatoid arthritis (RA) and the like. SLE may affect various organs, and it is impossible to predict when the disease will onset so far and the natural course of the disease is characterized by the alternation of aggravation and remissions. Global average prevalence rate of SLE is 12-39 patients per hundred thousand people, and the prevalence rate of SLE in China is 30-70 patients per one hundred thousand people, having the second highest rate in the world and next to black people's 100 patients per one hundred thousand people. SLE usually occurs in young women, and 90% of patients are female. RA is a long-term, continuous disease that mainly affects the joints. It usually causes joint fever, swelling and pain, which can lead to the surface erosion and destruction of the joints, and even cause limb deformity in severe cases. According to statistics, the incidence of RA is about 0.3%. In recent years, the incidence of autoimmune disease has gradually increased in China and even in the world. Due to the vast territory and large population in China, the calculated number of patients based on this incidence is quite large. However, there is still a lack of effective intervention and treatment with small side effects and strong specificity for blocking the damage of target organ in early phase and thus improve the prognosis of patients.

Studies have shown that increased expression of B lymphocyte stimulator (BLyS) can be detected in patients with autoimmune diseases and B-cell neoplasms. For instance, the expression of BLyS in serum is elevated in various groups of patients with SLE, and it is associated with the production of autoimmune antibody and disease activity index [Stohl et al. 2003, Arthritis Rheum, 48 (12): 3475; Petri et al. 2008, Arthritis Rheum, 58 (8): 2453]. High expression level of BLyS is also found in synovial fluid of patients with RA [Tan et al. 2003, Arthritis Rheum, 48 (4): 982]. The correlation between BLyS overexpression and the clinical manifestations of these autoimmune diseases suggests that regulation of the expression level of BLyS may be a new approach for the treatment of these diseases.

BLyS, also known as BAFF, THANK, Tall-1, TNFSF13B or zTNF4, is a member of the tumor necrosis factor (TNF) ligand superfamily [Baker et al. 2003, Arthritis Rheum, 48(11):3253]. BLyS is expressed as a membrane-bound isoform with 285 amino acids of type II transmembrane protein or a soluble isoform with 152 amino acids after cleavage. BLyS is expressed on monocytes, macrophages and dendritic cells and is up-regulated by the stimulation of interferon-y and interleukin-10. Recombinant human BLyS enhances B cell proliferation and antibody secretion by binding to the major receptors on the surface of B cells *in vitro.* Recombinant human BLyS leads to splenic proliferation in mice primarily due to an increase in the number of mature B cells *in vivo.* Injection of BLyS into mice also results in an increase of antibody concentration in the serum as well as an increase in T-cell dependent and antigen independent humoral immunity. Overexpression of BLyS can induce abnormally high level of antibodies expression which leading to SLE, RA and other autoimmune diseases.

In recent years, monoclonal antibody drugs have been widely used in the treatment of tumors, autoimmune diseases and the like due to their advantages of strong specificity, high curative effect and small side effects. The annual global sales of monoclonal antibody drugs have been increased from $300 million in 1997 to $66.3 billion in 2012, becoming one of the fastest-growing and most profitable fields in biopharmaceutical industry which has broad space of development. Currently, Belimumab, a monoclonal antibody against BLyS, is the first monoclonal antibody approved by FDA in the past half century, which is of great significance.

Although there are still some problems with Belimumab, as a monoclonal antibody for SLE treatment, the monoclonal antibodies have stronger targeting ability and significantly fewer side effects than immunosuppressants such as cyclophosphamide and hormones. It is estimated that the market has great potential and the sales of SLE drugs will reach $3.9 billion by 2022. Therefore, it is urgent to obtain new, safer and more effective anti-BLyS antibodies for the treatment of autoimmune diseases such as SLE.

### Content of the present invention

Technical problem to be solved herein is to provide a high-affinity and high-specificity humanized or fully human BLyS antibody, a preparation method and a use thereof for overcoming the lack of effective and safe BLyS antibodies at current stage. The BLyS antibodies of the present invention have high affinity for BLyS, which can inhibit the binding of BLyS to its receptor and inhibit the proliferation of BLyS-induced mouse B lymphocytes. The BLyS antibodies herein lacks a cross-reaction with homologous protein antigens of BLyS such as human APRIL and thus they can be used in manufacturing a medicament for preventing or treating autoimmune diseases or tumors, which are associated with abnormal expression or dysfunction of BLyS.

The inventors obtained the lead antibody of BLyS by employing phage display and hybridoma technique. After preliminary production, purification and identification of the lead antibody, the BLyS antibody which comprises outstanding bioactive properties such as high affinity (affinity KD<5×10⁻⁹M), effectively blocking the binding of BLyS to its receptor, and lacking a cross-reaction with BLyS homologous protein such as human APRIL, can be obtained. Subsequently the amino acid sequences of the heavy chain variable region and the light chain variable region of BLyS antibody can be obtained by using molecular biological methods.

The present invention provides an isolated protein, which comprises complementary determining regions (CDR or CDRs) of BLyS antibody: one or more of the heavy chain CDR1, heavy chain CDR2 and heavy chain CDR3, and/or one or more of the light chain CDR1, light chain CDR2, and light chain CDR3, wherein the heavy chain CDR1 contains amino acid sequences represented by SEQ ID No.2, SEQ ID No.10, SEQ ID No.18, SEQ ID No.26, SEQ ID No.34, SEQ ID No.42, SEQ ID No.50, SEQ ID No.58, SEQ ID No.66, SEQ ID No.74, SEQ ID No.82, SEQ ID No.90 or SEQ ID No.98; the heavy chain CDR2 contains amino acid sequences represented by SEQ ID No.3, SEQ ID No.11, SEQ ID No.19, SEQ ID No.27, SEQ ID No.35, SEQ ID No.43, SEQ ID No.51, SEQ ID No.59, SEQ ID No.67, SEQ ID No.75, SEQ ID No.83, SEQ ID No.91 or SEQ ID No.99; the heavy chain CDR3 contains amino acid sequences represented by SEQ ID No.4, SEQ ID No.12, SEQ ID No.20, SEQ ID No.28, SEQ ID No.36, SEQ ID No.44, SEQ ID No.52, SEQ ID No.60, SEQ ID No.68, SEQ ID No.76, SEQ ID No.84, SEQ ID No.92 or SEQ ID No.100; the light chain CDR1 contains amino acid sequences represented by SEQ ID No.6, SEQ ID No.14, SEQ ID No.22, SEQ ID No.30, SEQ ID No.38, SEQ ID No.46, SEQ ID No.54, SEQ ID No.62, SEQ ID No.70, SEQ ID No.78, SEQ ID No.86, SEQ ID No.94 or SEQ ID No.102; the light chain CDR2 contains amino acid sequences represented by SEQ ID No.7, SEQ ID No.15, SEQ ID No.23, SEQ ID No.31, SEQ ID No.39, SEQ ID No.47, SEQ ID No.55, SEQ ID No.63, SEQ ID No.71, SEQ ID No.79, SEQ ID No.87, SEQ ID No.95 or SEQ ID No.103; the light chain CDR3 contains amino acid sequences represented by SEQ ID No.8, SEQ ID No.16, SEQ ID No.24, SEQ ID No.32, SEQ ID No.40, SEQ ID No.48, SEQ ID No.56, SEQ ID No.64, SEQ ID No.72, SEQ ID No.80, SEQ ID No.88, SEQ ID No.96 or SEQ ID No.104;
Or, the amino acid sequences of the heavy chain CDR1 is at least 80% identical to the amino acid sequences represented by SEQ ID No.2, SEQ ID No.10, SEQ ID No.18, SEQ ID No.26, SEQ ID No.34, SEQ ID No.42, SEQ ID No.50, SEQ ID No.58, SEQ ID No.66, SEQ ID No.74, SEQ ID No.82, SEQ ID No.90 or SEQ ID No.98; the amino acid sequences of the heavy chain CDR2 is at least 80% identical to the amino acid sequences represented by SEQ ID No.3, SEQ ID No.11, SEQ ID No.19, SEQ ID No.27, SEQ ID No.35, SEQ ID No.43, SEQ ID No.51, SEQ ID No.59, SEQ ID No.67, SEQ ID No.75, SEQ ID No.83, SEQ ID No.91 or SEQ ID No.99; the amino acid sequences of the heavy chain CDR3 is at least 80% identical to the amino acid sequences represented by SEQ ID No.4, SEQ ID No.12, SEQ ID No.20, SEQ ID No.28, SEQ ID No.36, SEQ ID No.44, SEQ ID No.52, SEQ ID No.60, SEQ ID No.68, SEQ ID No.76, SEQ ID No.84, SEQ ID No.92 or SEQ ID No.100; the amino acid sequences of the light chain CDR1 is at least 80% identical to the amino acid sequences represented by SEQ ID No.6, SEQ ID No.14, SEQ ID No.22, SEQ ID No.30, SEQ ID No.38, SEQ ID No.46, SEQ ID No.54, SEQ ID No.62, SEQ ID No.70, SEQ ID No.78, SEQ ID No.86, SEQ ID No.94 or SEQ ID No.102; the amino acid sequences of the light chain CDR2 is at least 80% identical to the amino acid sequences represented by SEQ ID No.7, SEQ ID No.15, SEQ ID No.23, SEQ ID No.31, SEQ ID No.39, SEQ ID No.47, SEQ ID No.55, SEQ ID No.63, SEQ ID No.71, SEQ ID No.79, SEQ ID No.87, SEQ ID No.95 or SEQ ID No.103; the amino acid sequences of the light chain CDR3 is at least 80% identical to the amino acid sequences represented by SEQ ID No.8, SEQ ID No.16, SEQ ID No.24, SEQ ID No.32, SEQ ID No.40, SEQ ID No.48, SEQ ID No.56, SEQ ID No.64, SEQ ID No.72, SEQ ID No.80, SEQ ID No.88, SEQ ID No.96 or SEQ ID No.104.

Preferably, the heavy chain CDR1 comprises amino acid sequence of SEQ ID No.2, the heavy chain CDR2 comprises amino acid sequence of SEQ ID No.3 and the heavy chain CDR3 comprises amino acid sequence of SEQ ID No.4; the heavy chain heavy chain CDR1 comprises amino acid sequence of SEQ ID No.10, the heavy chain CDR2 comprises amino acid sequence of SEQ ID No.11 and a CDR3 comprises amino acid sequence of SEQ ID No.12; the heavy chain CDR1 comprises amino acid sequence of SEQ ID No.18, the heavy chain CDR2 comprises amino acid sequence of SEQ ID No.19 and the heavy chain CDR3 comprises amino acid sequence of SEQ ID No.20; the heavy chain CDR1 comprises amino acid sequence of SEQ ID No.26, the heavy chain CDR2 comprises amino acid sequence of SEQ ID No.27 and the heavy chain CDR3 comprises amino acid sequence of SEQ ID No.28; the heavy chain CDR1 comprises amino acid sequence of SEQ ID No.34, the heavy chain CDR2 comprises amino acid sequence of SEQ ID No.35 and a CDR3 comprises amino acid sequence of SEQ ID No.36; the heavy chain CDR1 comprises amino acid sequence of SEQ ID No.42, the heavy chain CDR2 comprises amino acid sequence of SEQ ID No.43 and the heavy chain CDR3 comprises amino acid sequence of SEQ ID No.44; the heavy chain CDR1 comprises amino acid sequence of SEQ ID No.50, the heavy chain CDR2 comprises amino acid sequence of SEQ ID No.51 and the heavy chain CDR3 comprises amino acid sequence of SEQ ID No.52; the heavy chain CDR1 comprises amino acid sequence of SEQ ID No.58, the heavy chain CDR2 comprises amino acid sequence of SEQ ID No.59 and the heavy chain CDR3 comprises amino acid sequence of SEQ ID No.60; the heavy chain CDR1 comprises amino acid sequence of SEQ ID No.66, the heavy chain CDR2 comprises amino acid sequence of SEQ ID No.67 and the heavy chain CDR3 comprises amino acid sequence of SEQ ID No.68; the heavy chain CDR1 comprises amino acid sequence of SEQ ID No.74, the heavy chain CDR2 comprises amino acid sequence of SEQ ID No.75 and the heavy chain CDR3 comprises amino acid sequence of SEQ ID No.76; the heavy chain CDR1 comprises amino acid sequence of SEQ ID No.82, the heavy chain CDR2 comprises amino acid sequence of SEQ ID No.83 and the heavy chain CDR3 comprises amino acid sequence of SEQ ID No.84; the heavy chain CDR1 comprises amino acid sequence of SEQ ID No.90, the heavy chain CDR2 comprises amino acid sequence of SEQ ID No.91 and the heavy chain CDR3 comprises amino acid sequence of SEQ ID No.92; the heavy chain CDR1 comprises amino acid sequence of SEQ ID No.98, a CDR2 comprises amino acid sequence of SEQ ID No.99 and the heavy chain CDR3 comprises amino acid sequence of SEQ ID No.100;
The light chain CDR1 comprises amino acid sequence of SEQ ID No.6, the light chain CDR2 comprises amino acid sequence of SEQ ID No.7 and the light chain CDR3 comprises amino acid sequence of SEQ ID No.8; the light chain CDR1 comprises amino acid sequence of SEQ ID No.14, the light chain CDR2 comprises amino acid sequence of SEQ ID No.15 and the light chain CDR3 comprises amino acid sequence of SEQ ID No.16; the light chain CDR1 comprises amino acid sequence of SEQ ID No.22, the light chain CDR2 comprises amino acid sequence of SEQ ID No.23 and the light chain CDR3 comprises amino acid sequence of SEQ ID No.24; the light chain CDR1 comprises amino acid sequence of SEQ ID No.30, the light chain CDR2 comprises amino acid sequence of SEQ ID No.31 and the light chain CDR3 comprises amino acid sequence of SEQ ID No.32; the light chain CDR1 comprises amino acid sequence of SEQ ID No.38, the light chain CDR2 comprises amino acid sequence of SEQ ID No.39 and the light chain CDR3 comprises amino acid sequence of SEQ ID No.40; the light chain CDR1 comprises amino acid sequence of SEQ ID No.46, the light chain CDR2 comprises amino acid sequence of SEQ ID No.47 and the light chain CDR3 comprises amino acid sequence of SEQ ID No.48; the light chain CDR1 comprises amino acid sequence of SEQ ID No.54, the light chain CDR2 comprises amino acid sequence of SEQ ID No.55 and the light chain CDR3 comprises amino acid sequence of SEQ ID No.56; the light chain CDR1 comprises amino acid sequence of SEQ ID No.62, the light chain CDR2 comprises amino acid sequence of SEQ ID No.63 and the light chain CDR3 comprises amino acid sequence of SEQ ID No.64; the light chain CDR1 comprises amino acid sequence of SEQ ID No.70, the light chain CDR2 comprises amino acid sequence of SEQ ID No.71 and the light chain CDR3 comprises amino acid sequence of SEQ ID No.72; the light chain CDR1 comprises amino acid sequence of SEQ ID No.78, the light chain CDR2 comprises amino acid sequence of SEQ ID No.79 and the light chain CDR3 comprises amino acid sequence of SEQ ID No.80; the light chain CDR1 comprises amino acid sequence of SEQ ID No.86, the light chain CDR2 comprises amino acid sequence of SEQ ID No.87 and the light chain CDR3 comprises amino acid sequence of SEQ ID No.88; the light chain CDR1 comprises amino acid sequence of SEQ ID No.94, the light chain CDR2 comprises amino acid sequence of SEQ ID No.95 and a CDR3 comprises amino acid sequence of SEQ ID No.96; or, the light chain light chain CDR1 comprises amino acid sequence of SEQ ID No.102, the light chain CDR2 comprises amino acid sequence of SEQ ID No.103 and the light chain CDR3 comprises amino acid sequence of SEQ ID No.104. Preferably, aforesaid protein comprises the variable region of BLyS antibody heavy chain and/or variable region of BLyS antibody light chain forming by CDR and framework, the amino acid sequences of heavy chain variable region are represented by SEQ ID No.1, SEQ ID No.9, SEQ ID No.17, SEQ ID No.25, SEQ ID No.33, SEQ ID No.41, SEQ ID No.49, SEQ ID No.57, SEQ ID No.65, SEQ ID No.73, SEQ ID No.81, SEQ ID No.89 or SEQ ID No.97; the amino acid sequences of light chain variable region are represented by SEQ ID No.5, SEQ ID No.13, SEQ ID No.21, SEQ ID No.29, SEQ ID No.37, SEQ ID No.45, SEQ ID No.53, SEQ ID No.61, SEQ ID No.69, SEQ ID No.77, SEQ ID No.85, SEQ ID No.93 or SEQ ID No.101.

The invention further provides an isolated protein, comprising a variable region of BLyS antibody heavy chain and/or variable region of BLyS antibody light chain, the amino acid sequences of heavy chain variable region are represented by SEQ ID No.1, SEQ ID No.9, SEQ ID No.17, SEQ ID No.25, SEQ ID No.33, SEQ ID No.41, SEQ ID No.49, SEQ ID No.57, SEQ ID No.65, SEQ ID No.73, SEQ ID No.81, SEQ ID No.89 or SEQ ID No.97; the amino acid sequences of light chain variable region are represented by SEQ ID No.5, SEQ ID No.13, SEQ ID No.21, SEQ ID No.29, SEQ ID No.37, SEQ ID No.45, SEQ ID No.53, SEQ ID No.61, SEQ ID No.69, SEQ ID No.77, SEQ ID No.85, SEQ ID No.93 or SEQ ID No.101.

Preferably, an amino acid sequence of the heavy chain variable region is shown in SEQ ID No.1 and an amino acid sequence of the light chain variable region is shown in SEQ ID No.5; an amino acid sequence of the heavy chain variable region is shown in SEQ ID No.9 and an amino acid sequence of the light chain variable region is shown in SEQ ID No.13; an amino acid sequence of the heavy chain variable region is shown in SEQ ID No.17 and an amino acid sequence of the light chain variable region is shown in SEQ ID No.21; an amino acid sequence of the heavy chain variable region is shown in SEQ ID No.25 and an amino acid sequence of the light chain variable region is shown in SEQ ID No.29; an amino acid sequence of the heavy chain variable region is shown in SEQ ID No.33 and an amino acid sequence of the light chain variable region is shown in SEQ ID No.37; an amino acid sequence of the heavy chain variable region is shown in SEQ ID No.41 and an amino acid sequence of the light chain variable region is shown in SEQ ID No.45; an amino acid sequence of the heavy chain variable region is shown in SEQ ID No.49 and an amino acid sequence of the light chain variable region is shown in SEQ ID No.53; an amino acid sequence of the heavy chain variable region is shown in SEQ ID No.57 and an amino acid sequence of the light chain variable region is shown in SEQ ID No.61; an amino acid sequence of the heavy chain variable region is shown in SEQ ID No.65 and an amino acid sequence of the light chain variable region is shown in SEQ ID No.69; an amino acid sequence of the heavy chain variable region is shown in SEQ ID No.73 and an amino acid sequence of the light chain variable region is shown in SEQ ID No.77; an amino acid sequence of the heavy chain variable region is shown in SEQ ID No.81 and an amino acid sequence of the light chain variable region is shown in SEQ ID No.85; an amino acid sequence of the heavy chain variable region is shown in SEQ ID No.89 and an amino acid sequence of the light chain variable region is shown in SEQ ID No.93; or, an amino acid sequence of the heavy chain variable region is shown in SEQ ID No.97 and an amino acid sequence of the light chain variable region is shown in SEQ ID No.101.

In this art, antibody-antigen binding domain each contains a light chain variable region and a heavy chain variable region, meanwhile each variable region contains three domains, CDR1, CDR2 and CDR3.

In summary, the SEQ ID numbers of the above-mentioned amino acid sequences are shown in Table 1:

**Table 1 BLyS Antibody Amino Acid SEQ ID NO.**

| Clone No. | Heavy Chain | | | | Light Chain | | | |
|---|---|---|---|---|---|---|---|---|
| | Variable Region | CDR1 | CDR2 | CDR3 | Variable Region | CDR1 | CDR2 | CDR3 |
| 2-1G11 | 1 | 2 | 3 | 4 | 5 | 6 | 7 | 8 |
| L9G7 | 9 | 10 | 11 | 12 | 13 | 14 | 15 | 16 |
| L1D12 | 17 | 18 | 19 | 20 | 21 | 22 | 23 | 24 |
| 35E6F7C3 | 25 | 26 | 27 | 28 | 29 | 30 | 31 | 32 |
| 8E7D9C7F5 | 33 | 34 | 35 | 36 | 37 | 38 | 39 | 40 |
| 20D1B6E9E5 | 41 | 42 | 43 | 44 | 45 | 46 | 47 | 48 |
| 78C11D2D12 | 49 | 50 | 51 | 52 | 53 | 54 | 55 | 56 |
| 89A2G5E7 | 57 | 58 | 59 | 60 | 61 | 62 | 63 | 64 |
| 97E7B3F2 | 65 | 66 | 67 | 68 | 69 | 70 | 71 | 72 |
| 97A3C2H4 | 73 | 74 | 75 | 76 | 77 | 78 | 79 | 80 |
| 67A2E1D10 | 81 | 82 | 83 | 84 | 85 | 86 | 87 | 88 |
| 111D10D6G3 | 89 | 90 | 91 | 92 | 93 | 94 | 95 | 96 |
| 93C6F10D3 | 97 | 98 | 99 | 100 | 101 | 102 | 103 | 104 |

Where the numbers in Table 1 are the sequence numbers in the sequence listing, for example, the amino acid sequence of heavy chain variable region of 2-1G11 is shown in SEQ ID No. 1, and the amino acid sequence of heavy chain CDR1 of 2-1G11 is shown in SEQ ID No. 2.

The isolated protein in the present invention further comprises a framework region (or framework or FR) of BLyS antibody, and the framework region comprises a framework region of heavy chain and/or a framework region of light chain; and preferably, the framework region of heavy chain is a framework region of human or mouse antibody heavy chain, and/or the framework region of light chain is a framework region of human or mouse antibody light chain.

More preferably, when the isolated protein in the present invention is humanized antibody or fully human antibody, the framework region of heavy chain is a framework region of human antibody heavy chain, and the framework residues of human antibody heavy chain may contain germlines DP4, DP7, DP8, DP9, DP10, DP14(VH1-18), DP31, DP33, DP35(VH3-11), DP45, DP46, DP47, DP48, DP49(VH3-30), DP50, DP51(VH3-48), DP53, DP54(VH3-7), DP65, DP66, DP67, DP68 and DP69, especially the FR1, FR2, FR3 of these germlines; as well as JH fragments JH-1, JH-2, JH-3, JH-4, JH-4b, JH-5 and JH-6, especially the sequences encoded by FR4 of these germlines, or the consensus sequences of the heavy chain framework regions. The framework region of light chain is a framework region of human antibody light chain, and the framework residues of human antibody light chain may contain germlines O2, 012, DPK1(O18), DPK2, DPK3, DPK4, DPK5, DPK6, DPK7, DPK8, DPK9, DPK10, DPK12(A2), DPK13, DPK15, DPK16, DPKI8, DPK19, DPK20, DPK21, DPK22, DPK23, DPK24(B3), DPK25, DPK26(A10) and DPK 28, especially the FR1, FR2, FR3 of these germlines; as well as Jk fragments Jk1, Jk2, Jk3, Jk4 and Jk5, especially the sequences encoded by FR4 of these germlines. Such sequences of framework regions may be obtained from public DNA databases including germline antibody gene sequences or published references. For example, germline DNA sequences of variable region genes of human heavy and light chain are available in the "VBase" human germline sequence database (http://www2.mrc-lmb.cam.ac.uk/vbase/) and the Kabat (E.A et al., 1991 Sequences of Proteins of Immunological Interest, 5th edition). In a better embodiment of the humanized antibody of the present invention, the framework residues of heavy chain is preferably V_{H}1-18 of V_{H} exon, V_{H}3-7 of V_{H} exon or J_{H}-6 of J_{H} exon from a heavy chain of human germline antibody, and the framework residues of light chain is preferably B3 of V_{K} exon, J_{K}-4 of the J_{K} exon or A10 of the V_{K} exon from a light chain of human germline antibody.

Preferably, the protein further comprises heavy chain constant region of antibody and/or light chain constant region of antibody, wherein the heavy chain constant region of antibody is known in the art, preferably is a heavy chain constant region of mouse antibody or a heavy chain constant region of human antibody, more preferably is a heavy chain constant region of human antibody. The light chain constant region of antibody is known in the art, preferably is a light chain constant region of mouse antibody or a light chain constant region of human antibody, more preferably is a light chain constant region of human antibody.

Where the mouse heavy chain constant region and mouse light chain constant region, or human heavy chain constant region and human light chain constant region can form murine BLyS antibody or BLyS chimeric antibody with the heavy chain variable regions having amino acid sequence shown in SEQ ID NO.25, 33, 41, 49, 57, 65, 73, 81, 89 or 97 and the light chain variable regions having amino acid sequence shown in SEQ ID NO. 29, 37, 45, 53, 61, 69, 77, 85, 93 or 101, respectively.

Further, the heavy chain CDRs sequences shown in SEQ ID NO. 26-28, 34-36, 42-44, 50-52, 58-60, 66-68, 74-76, 82-84, 90-92, or 98-100 determined by the amino acid sequence of the heavy chain variable region of SEQ ID NO. 25, 33, 41, 49, 57, 65, 73, 81, 89 or 97 according to Kabat definition in aforementioned chimeric antibody, and the light chain CDRs sequences of SEQ ID NO. 30-32, 38-40, 46-48, 54-56, 62-64, 70-72, 78-80, 86-88, 94-96 or 102-104 determined by aforementioned amino acid sequence of the light chain variable region of SEQ ID NO. 29, 37, 45, 53, 61, 69, 77, 85, 93 or 101 according to Kabat definition, were transplanted into the selected templates of human germline to replace the CDR region in the templates of human germline and then the humanized antibodies were constructed, respectively. The light chain framework region and heavy chain framework region in the germline templates are as described above, preferably selecting from V_{H}1-18 of V_{H} exon, V_{H}3-7 of V_{H} exon or J_{H}-6 of J_{H} exon in a heavy chain of human germline antibody, and B3 of V_{K} exon, J_{K}-4 of the J_{K} exon or A10 of the V_{K} exon in a light chain of human germline antibody. Optionally, in order to ensure the activity of antibodies, the buried residues, the residues that have direct interaction with the CDR regions, and the framework residues that have a significant influence on the conformations of VH and VL were back-mutated based on the three-dimensional structure of mouse antibody.

Preferably, the amino sequences of the heavy chain variable region, in which its CDR regions were transplanted into the selected human germline templates and the residues of framework regions were back-mutated, preferably are shown in SEQ ID NO. 140, SEQ ID NO. 141, SEQ ID NO. 142, SEQ ID NO. 143, SEQ ID NO. 144, SEQ ID NO. 145, SEQ ID NO. 146, SEQ ID NO. 149, SEQ ID NO. 150, SEQ ID NO. 151 or SEQ ID NO. 152. The amino acid sequences of the light chain variable region preferably are shown in SEQ ID No. 147, SEQ ID No. 148, SEQ ID No. 153, SEQ ID No. 154, SEQ ID No. 155, SEQ ID No. 156 or SEQ ID No.157.

Sequence of the heavy chain variable region shown in SEQ ID NO. 1, 9 or 17 and sequence of the light chain variable region shown in SEQ ID NO. 5, 13 or 21 can form human BLyS antibody with constant region of humanized antibody heavy chain as well as constant region of humanized antibody light chain.

The protein is antibody protein, preferably one or more of full-length antibody protein, antibody-antigen binding domain protein fragment, bispecific antibody, multispecific antibody, single-chain antibody fragment (scFv), single-domain antibody (sdAb) or single-region antibody, as well as the monoclonal antibody or polyclonal antibody produced by aforesaid antibodies. The monoclonal antibody can be developed using a variety of routes and technologies such as hybridoma technology, phage display technology, single lymphocyte gene cloning technology and the like, and the production of monoclonal antibody from wild-type or transgenic mice by hybridoma technology is current mainstream technology.

The full-length antibody protein is a conventional full-length antibody protein known in the art, which comprises heavy chain variable region, light chain variable region, heavy chain constant region and light chain constant region. The heavy chain variable region and light chain variable region of the protein form human full-length antibody protein with human heavy chain constant region and human light chain constant region. Preferably, the full-length antibody protein is IgG1, IgG2, IgG3 or IgG4.

The single-chain antibody is a conventional single-chain antibody known in the art, which comprises heavy chain variable region, light chain variable region and short peptide of 15-20 amino acids.

The protein fragment of antibody-antigen binding domain is a conventional protein fragment of antibody-antigen binding domain known in the art, which comprises light chain variable region, light chain constant region and Fd fragment of heavy chain constant region. Preferably, the protein fragment of antibody-antigen binding domain is Fab and F(ab')2.

The single-domain antibody is a conventional single-domain antibody known in the art, which comprises heavy chain variable region and heavy chain constant region.

The single-region antibody is a conventional single-region antibody known in the art, which only comprises heavy chain variable region.

The preparation method of the protein is a conventional preparation method known in the art. The preparation method is preferably: obtain the protein by isolating the protein from an expression transformant that recombinantly expresses the protein or by artificial synthesis of protein sequences. Preferably, the method for isolating the protein from the expression transformant that recombinantly expresses the protein includes: cloning a nucleic acid that encodes a protein and comprises a point mutation into a recombinant vector, transforming the recombinant vector into the transformant to obtain a recombinant expression transformant, and isolating and purifying the protein from the culture of the recombinant expression transformant.

The present invention further provides a nucleic acid encoding aforesaid protein.

Preferably, the nucleotide sequences of the nucleic acid encoding the heavy chain variable region are shown in SEQ ID No.105, SEQ ID No.107, SEQ ID No.109, SEQ ID No.111, SEQ ID No.113, SEQ ID No.115, SEQ ID No.117, SEQ ID No.119, SEQ ID No.121, SEQ ID No.123, SEQ ID No.125, SEQ ID No.127 or SEQ ID No.129; and/or, the nucleotide sequences of the nucleic acid encoding the light chain variable region are shown in SEQ ID No.106, SEQ ID No.108, SEQ ID No.110, SEQ ID No.112, SEQ ID No.114, SEQ ID No.116, SEQ ID No.118, SEQ ID No.120, SEQ ID No.122, SEQ ID No.124, SEQ ID No.126, SEQ ID No.128 or SEQ ID No.130.

More preferably, a nucleotide sequence of the nucleic acid encoding the heavy chain variable region is shown in SEQ ID No.105, and a nucleotide sequence of the nucleic acid encoding the light chain variable region is shown in SEQ ID No.106; a nucleotide sequence of the nucleic acid encoding the heavy chain variable region is shown in SEQ ID No.107, and a nucleotide sequence of the nucleic acid encoding the light chain variable region is shown in SEQ ID No.108; a nucleotide sequence of the nucleic acid encoding the heavy chain variable region is shown in SEQ ID No.109, and a nucleotide sequence of the nucleic acid encoding the light chain variable region is shown in SEQ ID No.110; a nucleotide sequence of the nucleic acid encoding the heavy chain variable region is shown in SEQ ID No.111, and a nucleotide sequence of the nucleic acid encoding the light chain variable region is shown in SEQ ID No.112; a nucleotide sequence of the nucleic acid encoding the heavy chain variable region is shown in SEQ ID No.113, and a nucleotide sequence of the nucleic acid encoding the light chain variable region is shown in SEQ ID No.114; a nucleotide sequence of the nucleic acid encoding the heavy chain variable region is shown in SEQ ID No.115, and a nucleotide sequence of the nucleic acid encoding the light chain variable region is shown in SEQ ID No.116; a nucleotide sequence of the nucleic acid encoding the heavy chain variable region is shown in SEQ ID No.117, and a nucleotide sequence of the nucleic acid encoding the light chain variable region is shown in SEQ ID No.118; a nucleotide sequence of the nucleic acid encoding the heavy chain variable region is shown in SEQ ID No.119, and a nucleotide sequence of the nucleic acid encoding the light chain variable region is shown in SEQ ID No.120; a nucleotide sequence of the nucleic acid encoding the heavy chain variable region is shown in SEQ ID No.121, and a nucleotide sequence of the nucleic acid encoding the light chain variable region is shown in SEQ ID No.122; a nucleotide sequence of the nucleic acid encoding the heavy chain variable region is shown in SEQ ID No.123, and a nucleotide sequence of the nucleic acid encoding the light chain variable region is shown in SEQ ID No.124; a nucleotide sequence of the nucleic acid encoding the heavy chain variable region is shown in SEQ ID No.125, and a nucleotide sequence of the nucleic acid encoding the light chain variable region is shown in SEQ ID No.126; a nucleotide sequence of the nucleic acid encoding the heavy chain variable region is shown in SEQ ID No.127, and a nucleotide sequence of the nucleic acid encoding the light chain variable region is shown in SEQ ID No.128; a nucleotide sequence of the nucleic acid encoding the heavy chain variable region is shown in SEQ ID No.129, and a nucleotide sequence of the nucleic acid encoding the light chain variable region is shown in SEQ ID No.130.

In summary, the sequence ID numbers of the aforementioned nucleotide sequences are shown in Table 2:

**Table 2 Nucleotide SEQ ID NO of BLyS Antibody Gene.**

| Clone No. | Heavy chain variable region | Light chain variable region |
|---|---|---|
| 2-1G11 | 105 | 106 |
| L9G7 | 107 | 108 |
| L1D12 | 109 | 110 |
| 35E6F7C3 | 111 | 112 |
| 8E7D9C7F5 | 113 | 114 |
| 20D1B6E9E5 | 115 | 116 |
| 78C11D2D12 | 117 | 118 |
| 89A2G5E7 | 119 | 120 |
| 97E7B3F2 | 121 | 122 |
| 97A3C2H4 | 123 | 124 |
| 67A2E1D10 | 125 | 126 |
| 111D10D6G3 | 127 | 128 |
| 93C6F10D3 | 129 | 130 |

As used herein, the numbers in Table 2 are the sequence ID numbers in the Sequence Listing, for example, the nucleotide sequence of the amino acid encoding the heavy chain variable region of 2-1G11 is shown in SEQ ID No.105, and the nucleotide sequence of the amino acid encoding the light chain variable region of 2-1G11 is shown in SEQ ID No.106.

The nucleotide sequence encoding the heavy chain CDR1 of 2-1G11 is the sequence from 76th to 105th base shown in SEQ ID No.105 of the Sequence Listing.

The nucleotide sequence encoding the heavy chain CDR2 of 2-1G11 is the sequence from 148th to 198th base shown in SEQ ID No.105 of the Sequence Listing.

The nucleotide sequence encoding the heavy chain CDR3 of 2-1G11 is the sequence from 295th to 342nd base shown in SEQ ID No.105 of the Sequence Listing.

The nucleotide sequence encoding the light chain CDR1 of 2-1G11 is the sequence from 70th to 102nd base shown in SEQ ID No.106 of the Sequence Listing.

The nucleotide sequence encoding the light chain CDR2 of 2-1G11 is the sequence from 148th to 168th base shown in SEQ ID No.106 of the Sequence Listing.

The nucleotide sequence encoding the light chain CDR3 of 2-1G11 is the sequence from 265th to 291th base shown in SEQ ID No.106 of the Sequence Listing.

The nucleotide sequence encoding the heavy chain CDR1 of L9G7 is the sequence from 76th to 105th base shown in SEQ ID No.107 of the Sequence Listing.

The nucleotide sequence encoding the heavy chain CDR2 of L9G7 is the sequence from 148th to 198th base shown in SEQ ID No.107 of the Sequence Listing.

The nucleotide sequence encoding the heavy chain CDR3 of L9G7 is the sequence from 295th to 342nd base shown in SEQ ID No.107 of the Sequence Listing.

The nucleotide sequence encoding the light chain CDR1 of L9G7 is the sequence from 70th to 102nd base shown in SEQ ID No.108 of the Sequence Listing.

The nucleotide sequence encoding the light chain CDR2 of L9G7 is the sequence from 148th to 168th base shown in SEQ ID No.108 of the Sequence Listing.

The nucleotide sequence encoding the light chain CDR3 of L9G7 is the sequence from 265th to 291nd base shown in SEQ ID No.108 of the Sequence Listing.

The nucleotide sequence encoding the heavy chain CDR1 of L1D12 is the sequence from 76th to 105th base shown in SEQ ID No.109 of the Sequence Listing.

The nucleotide sequence encoding the heavy chain CDR2 of L1D12 is the sequence from 148th to 195th base shown in SEQ ID No.109 of the Sequence Listing.

The nucleotide sequence encoding the heavy chain CDR3 of L1D12 is the sequence from 292nd to 330nd base shown in SEQ ID No.1 09 of the Sequence Listing.

The nucleotide sequence encoding the light chain CDR1 of L1D12 is the sequence from 67th to 99th base shown in SEQ ID No.110 of the Sequence Listing.

The nucleotide sequence encoding the light chain CDR2 of L1D12 is the sequence from 145th to 165th base shown in SEQ ID No.110 of the Sequence Listing.

The nucleotide sequence encoding the light chain CDR3 of L1D12 is the sequence from 262nd to 297th base shown in SEQ ID No.110 of the Sequence Listing.

The nucleotide sequence encoding the heavy chain CDR1 of 35E6F7C3 is the sequence from 76th to 105th base shown in SEQ ID No.111 of the Sequence Listing.

The nucleotide sequence encoding the heavy chain CDR2 of 35E6F7C3 is the sequence from 148th to 198th base shown in SEQ ID No.111.

The nucleotide sequence encoding the heavy chain CDR3 of 35E6F7C3 is the sequence from 295nd to 321th base shown in SEQ ID No.111 of the Sequence Listing.

The nucleotide sequence encoding the light chain CDR1 of 35E6F7C3 is the sequence from 70th to 99th base shown in SEQ ID No.112 of the Sequence Listing.

The nucleotide sequence encoding the light chain CDR2 of 35E6F7C3 is the sequence from 145th to 165th base shown in SEQ ID No.112 of the Sequence Listing.

The nucleotide sequence encoding the light chain CDR3 of 35E6F7C3 is the sequence from 262nd to 288th base shown in SEQ ID No.112 of the Sequence Listing.

The nucleotide sequence encoding the heavy chain CDR1 of 8E7D9C7F5 is the sequence from 76th to 105th base shown in SEQ ID No.113 of the Sequence Listing.

The nucleotide sequence encoding the heavy chain CDR2 of 8E7D9C7F5 is the sequence from 148th to 198th base shown in SEQ ID No.113 of the Sequence Listing.

The nucleotide sequence encoding the heavy chain CDR3 of 8E7D9C7F5 is the sequence from 295nd to 342nd base shown in SEQ ID No.113 of the Sequence Listing.

The nucleotide sequence encoding the light chain CDR1 of 8E7D9C7F5 is the sequence from 70th to 114th base shown in SEQ ID No.114 of the Sequence Listing.

The nucleotide sequence encoding the light chain CDR2 of 8E7D9C7F5 is the sequence from 160th to 180th base shown in SEQ ID No.114 of the Sequence Listing.

The nucleotide sequence encoding the h light chain CDR3 of 8E7D9C7F5 is the sequence from 277nd to 303th base shown in SEQ ID No.114 of the Sequence Listing.

The nucleotide sequence encoding the heavy chain CDR1 of 20D1B6E9E5 is the sequence from 76th to 105th base shown in SEQ ID No.115 of the Sequence Listing.

The nucleotide sequence encoding the heavy chain CDR2 of 20D1B6E9E5 is the sequence from 148th to 198th base shown in SEQ ID No.115 of the Sequence Listing.

The nucleotide sequence encoding the heavy chain CDR3 of 20D1B6E9E5 is the sequence from 295nd to 339th base shown in SEQ ID No.115 of the Sequence Listing.

The nucleotide sequence encoding the light chain CDR1 of 20D1B6E9E5 is the sequence from 70th to 117th base shown in SEQ ID No.116 of the Sequence Listing.

The nucleotide sequence encoding the light chain CDR2 of 20D1B6E9E5 is the sequence from 163th to 183th base shown in SEQ ID No.116 of the Sequence Listing.

The nucleotide sequence encoding the light chain CDR3 of 20D1B6E9E5 is the sequence from 280nd to 306th base shown in SEQ ID No.116 of the Sequence Listing.

The nucleotide sequence encoding the heavy chain CDR1 of 78C11D2D12 is the sequence from 76th to 105th base shown in SEQ ID No.117 of the Sequence Listing.

The nucleotide sequence encoding the heavy chain CDR2 of 78C11D2D12 is the sequence from 148th to 198th base shown in SEQ ID No.117 of the Sequence Listing.

The nucleotide sequence encoding the heavy chain CDR3 of 78C11D2D12 is the sequence from 295nd to 315th base shown in SEQ ID No.117 of the Sequence Listing.

The nucleotide sequence encoding the light chain CDR1 of 78C11D2D12 is the sequence from 70th to 99th base shown in SEQ ID No.118 of the Sequence Listing.

The nucleotide sequence encoding the light chain CDR2 of 78C11D2D12 is the sequence from 145th to 165th base shown in SEQ ID No.118 of the Sequence Listing.

The nucleotide sequence encoding the light chain CDR3 of 78C11D2D12 is the sequence from 262nd to 288th base shown in SEQ ID No.118 of the Sequence Listing.

The nucleotide sequence encoding the heavy chain CDR1 of 89A2G5E7 is the sequence from 76th to 105th base shown in SEQ ID No.119 of the Sequence Listing.

The nucleotide sequence encoding the heavy chain CDR2 of 89A2G5E7 is the sequence from 148th to 195th base shown in SEQ ID No.119 of the Sequence Listing.

The nucleotide sequence encoding the heavy chain CDR3 of 89A2G5E7 is the sequence from 292nd to 327th base shown in SEQ ID No.119 of the Sequence Listing.

The nucleotide sequence encoding the light chain CDR1 of 89A2G5E7 is the sequence from 70th to 105th base shown in SEQ ID No.120 of the Sequence Listing.

The nucleotide sequence encoding the light chain CDR2 of 89A2G5E7 is the sequence from 151th to 171th base shown in SEQ ID No.120 of the Sequence Listing.

The nucleotide sequence encoding the light chain CDR3 of 89A2G5E7 is the sequence from 268nd to 294th base shown in SEQ ID No.120 of the Sequence Listing.

The nucleotide sequence encoding the heavy chain CDR1 of 97E7B3F2 is the sequence from 76th to 105th base shown in SEQ ID No.121 of the Sequence Listing.

The nucleotide sequence encoding the heavy chain CDR2 of 97E7B3F2 is the sequence from 148th to 198th base shown in SEQ ID No.121 of the Sequence Listing.

The nucleotide sequence encoding the heavy chain CDR3 of 97E7B3F2 is the sequence from 295nd to 321th base shown in SEQ ID No.121 of the Sequence Listing.

The nucleotide sequence encoding the light chain CDR1 of 97E7B3F2 is the sequence from 70th to 117th base shown in SEQ ID No.122 of the Sequence Listing.

The nucleotide sequence encoding the light chain CDR2 of 97E7B3F2 is the sequence from 163th to 183th base shown in SEQ ID No.122 of the Sequence Listing.

The nucleotide sequence encoding the light chain CDR3 of 97E7B3F2 is the sequence from 280nd to 306th base shown in SEQ ID No.122 of the Sequence Listing.

The nucleotide sequence encoding the heavy chain CDR1 of 97A3C2H4 is the sequence from 76th to 105th base shown in SEQ ID No.123 of the Sequence Listing.

The nucleotide sequence encoding the heavy chain CDR2 of 97A3C2H4 is the sequence from 148th to 198th base shown in SEQ ID No.123 of the Sequence Listing.

The nucleotide sequence encoding the heavy chain CDR3 of 97A3C2H4 is the sequence from 295nd to 327th base shown in SEQ ID No.123 of the Sequence Listing.

The nucleotide sequence encoding the light chain CDR1 of 97A3C2H4 is the sequence from 70th to 102th base shown in SEQ ID No.124 of the Sequence Listing.

The nucleotide sequence encoding the light chain CDR2 of 97A3C2H4 is the sequence from 148th to 168th base shown in SEQ ID No.124 of the Sequence Listing.

The nucleotide sequence encoding the light chain CDR3 of 97A3C2H4 is the sequence from 265nd to 291th base shown in SEQ ID No.124 of the Sequence Listing.

The nucleotide sequence encoding the heavy chain CDR1 of 67A2E1D10 is the sequence from 76th to 105th base shown in SEQ ID No.125 of the Sequence Listing.

The nucleotide sequence encoding the heavy chain CDR2 of 67A2E1D10 is the sequence from 148th to 198th base shown in SEQ ID No.125 of the Sequence Listing.

The nucleotide sequence encoding the heavy chain CDR3 of 67A2E1D10 is the sequence from 295nd to 333th base shown in SEQ ID No.125 of the Sequence Listing.

The nucleotide sequence encoding the light chain CDR1 of 67A2E1D10 is the sequence from 70th to 102nd base shown in SEQ ID No.126 of the Sequence Listing.

The nucleotide sequence encoding the light chain CDR2 of 67A2E1D10 is the sequence from 148th to 168th base shown in SEQ ID No.126 of the Sequence Listing.

The nucleotide sequence encoding the light chain CDR3 of 67A2E1D10 is the sequence from 265nd to 291th base shown in SEQ ID No.126 of the Sequence Listing.

The nucleotide sequence encoding the heavy chain CDR1 of 111D10D6G3 is the sequence from 76th to 105th base shown in SEQ ID No.127 of the Sequence Listing.

The nucleotide sequence encoding the heavy chain CDR2 of 111D10D6G3 is the sequence from 148th to 198th base shown in SEQ ID No.127 of the Sequence Listing.

The nucleotide sequence encoding the heavy chain CDR3 of 111D10D6G3 is the sequence from 295nd to 309th base shown in SEQ ID No.127 of the Sequence Listing.

The nucleotide sequence encoding the light chain CDR1 of 111D10D6G3 is the sequence from 70th to 102th base shown in SEQ ID No.128 of the Sequence Listing.

The nucleotide sequence encoding the light chain CDR2 of 111D10D6G3 is the sequence from 148th to 168th base shown in SEQ ID No.128 of the Sequence Listing.

The nucleotide sequence encoding the light chain CDR3 of 111D10D6G3 is the sequence from 265nd to 291th base shown in SEQ ID No.128 of the Sequence Listing.

The nucleotide sequence encoding the heavy chain CDR1 of 93C6F10D3 is the sequence from 76th to 105th base shown in SEQ ID No.129 of the Sequence Listing.

The nucleotide sequence encoding the heavy chain CDR2 of 93C6F10D3 is the sequence from 148th to 198th base shown in SEQ ID No.129 of the Sequence Listing.

The nucleotide sequence encoding the heavy chain CDR3 of 93C6F10D3 is the sequence from 295nd to 336th base shown in SEQ ID No.129 of the Sequence Listing.

The nucleotide sequence encoding the light chain CDR1 of 93C6F10D3 is the sequence from 70th to 102th base shown in SEQ ID No.130 of the Sequence Listing.

The nucleotide sequence encoding the light chain CDR2 of 93C6F10D3 is the sequence from 148th to 168th base shown in SEQ ID No.130 of the Sequence Listing.

The nucleotide sequence encoding the light chain CDR3 of 93C6F10D3 is the sequence from 265nd to 291th base shown in SEQ ID No.130 of the Sequence Listing.

In a preferable embodiment of the present invention, the nucleotide sequence of the nucleic acid encoding the heavy chain variable region of the humanized antibody of the present invention, which is derived from the human framework region, is shown in SEQ ID NO. 158, SEQ ID NO. 159, SEQ ID NO. 160, SEQ ID NO. 161, SEQ ID NO. 162, SEQ ID NO. 163, SEQ ID NO. 164, SEQ ID NO. 167, SEQ ID NO. 168, SEQ ID NO. 169 or SEQ ID NO. 170 of the Sequencing Listing; and/or, the nucleotide sequence of the nucleic acid encoding the light chain variable region is shown in SEQ ID No.165, SEQ ID No.166, SEQ ID No.171, SEQ ID No.172, SEQ ID No.173, SEQ ID No.174, or SEQ ID No.175 of the Sequencing Listing.

The preparation method of the nucleic acid is a conventional preparation method known in the art, preferably comprising following steps: the nucleic acid molecules encoding aforesaid proteins are obtained by gene cloning technology, or the nucleic acid molecules encoding aforesaid proteins are obtained by the method of artificial full sequence synthesis.

Person skilled in the art knows that the substitution, deletion, alteration, insertion or addition can be introduced into the base sequence encoding the amino acid sequence of aforesaid protein as appropriate to provide a homologue of polynucleotide. A homologue of polynucleotide in the present invention can be prepared by substituting, deleting or adding one or more bases of a gene encoding the protein sequence while the activity of the antibody is maintained.

The present invention further provides a recombinant expression vector comprising the nucleic acid.

As used herein, the recombinant expression vector can be obtained by the conventional method known in the art, i.e, constructing the nucleic acid molecule of the present invention to various expression vectors. The expression vectors are a variety of vectors that are conventional in the art, as long as the vectors are able to accommodate aforesaid nucleic acid molecule. The vectors preferably include various plasmids, cosmids, phages or viral vectors.

The present invention further provides a recombinant expression transformant comprising the recombinant expression vector.

As used herein, the method for preparing the recombinant expression transformant is a conventional preparation method known in the art, preferably transforming the recombinant expression vector into host cells. The host cells are conventional various host cells known in the art, as long as they are able to stably self-replicate aforesaid recombinant expression vectors and efficiently express the carried nucleic acid. Preferably, the host cells are *E. coli* TG1 or *E. coli* BL21 cells (expressing single chain antibody or Fab antibody), or HEK293 or CHO-K1 cells (expressing full-length IgG antibody). The preferred recombinant expression transformant of the present invention can be obtained by transforming aforesaid recombinant expression plasmids into host cells. As used herein, the transformation method is a conventional method known in the art, preferably chemical transformation method, heat shock method or electrotransformation method.

The present invention further provides a method for preparing a BLyS antibody, which comprises following steps: culturing the recombinant expression transformant, and obtaining BLyS antibody from the culture.

The present invention further provides a method for detecting cells that overexpressing BLyS protein, which comprises following steps: contacting the protein with a sample to be tested *in vitro,* and detecting the combination between the protein and the sample to be tested.

The definition of overexpression is conventional in the art, which refers to the overexpression of RNA or protein of BLyS in the sample to be tested (due to increased transcription, post-transcriptional processing, translation, post-translational processing and the alternation in protein degradation), and local over-expression and increased functional activity (e.g. in the case of increased enzymatic hydrolysis of the substrate) resulting from changes in protein transporting patterns (increased nuclear localization).

As used herein, the method for detecting aforesaid combination is the conventional method known in the art, preferably the detection by fluorescence activated cell sorter (FACS).

The present invention provides a composition for detecting cells that overexpress BLyS protein, which comprises aforesaid protein as an active ingredient. Preferably, it further comprises compound consisting of functional fragments of aforesaid protein as an active ingredient.

The present invention provides a use of aforesaid protein in the preparation of medicament.

Preferably, the medicament is a medicament for preventing or treating diseases associated with abnormal expression or dysfunction of BLyS.

As used herein, the diseases associated with abnormal expression or dysfunction of BLyS is conventional diseases associated with abnormal expression or dysfunction of BLyS known in the art. Preferably autoimmune diseases, inflammatory diseases, infectious diseases or proliferative diseases. In the present invention, the autoimmune diseases are conventional autoimmune diseases known in the art, preferably systemic lupus erythematosus (SLE), rheumatoid arthritis (RA), Sjogren's syndrome (SS), multiple sclerosis (MS), myasthenia gravis (MG), chronic thyroiditis or immunodeficiency syndrome. In the present invention, the inflammatory diseases are conventional inflammatory diseases known in the art, preferably asthma or allergic diseases. In the present invention, the infectious diseases are conventional infectious diseases known in the art. Preferably acquired immune deficiency syndrome (AIDS). In the present invention, the proliferative diseases are conventional proliferative diseases known in the art, preferably leukemia, tumor or lymphoma.

The present invention further provides a pharmaceutical composition, comprising aforesaid protein as an active ingredient.

Preferably, the pharmaceutical composition is a pharmaceutical composition for preventing or treating diseases associated with abnormal expression or dysfunction of BLyS.

The administration route of the pharmaceutical composition in the present invention is preferably administered by injection or administered orally. The injection administration preferably includes intravenous injection, intramuscular injection, intraperitoneal injection, intradermal injection or subcutaneous injection. The pharmaceutical composition is in the form of various forms conventionally known in the art, preferably in solid, semisolid or liquid form, and may be aqueous solutions, non-aqueous solutions or suspensions, more preferably tablets, capsules, granules, injections or infusions and the like.

As used herein, preferably, the pharmaceutical composition of the present invention further comprises one or more pharmaceutical carriers. The pharmaceutical carrier is the conventional pharmaceutical carrier known in the art, and the pharmaceutical carrier can be any suitable physiologically or pharmaceutically acceptable pharmaceutical adjuvant. The pharmaceutical adjuvant is t conventional pharmaceutical adjuvant known in the art, preferably includes pharmaceutically acceptable excipients, fillers, diluents and the like. More preferably, the pharmaceutical composition comprises 0.01-99.99% of aforesaid protein and 0.01-99.99% of the pharmaceutical carrier, and the percentage is the mass percentage of the pharmaceutical composition.

As used herein, preferably, the pharmaceutical composition is administered in an amount effective to alleviate or delay the progression of a disease, degenerative or traumatic disorder. The effective amount can be determined on an individual basis and will be based in part on the consideration of the symptoms to be treated and the outcome sought. The effective amount can be determined by person skilled in the art using aforesaid factors such as individual difference of subjects and conventional experimentation.

The present invention provides the use of aforesaid protein in preparing medicament for preventing or treating diseases associated with abnormal expression or dysfunction of BLyS; preferably, the diseases associated with abnormal expression or dysfunction of BLyS are autoimmune diseases, inflammatory diseases, infectious diseases or proliferative diseases.

The present invention provides the use of aforesaid pharmaceutical composition in preparing drugs for preventing or treating diseases associated with abnormal expression or dysfunction of BLyS; preferably, the diseases associated with abnormal expression or dysfunction of BLyS are autoimmune diseases, inflammatory diseases, infectious diseases or proliferative diseases.

Based on the common knowledge in the art, aforesaid preferred conditions can be combined arbitrarily to obtain the preferable embodiments of the present invention.

The reagents and raw materials used in the present invention are commercially available.

The advantage of the present invention is that the protein of the present invention is a humanized or human BLyS antibody, which has a high affinity for BLyS protein (affinity KD <5×10⁻⁹M), can significantly block BLyS activities at the protein level and cellular level, and prevent the binding of BLyS to its receptors. The BLyS antibody lacks a cross-reaction with homologous protein antigen such as human APRIL. It was shown in the B-cell proliferation experiments that the BLyS antibody enjoys good biological activity which can inhibit the proliferation of mouse B lymphocytes induced by human BLyS. *In vivo* detection in mouse demonstrated that the BLyS antibody was able to reduce the increase of B lymphocytes in mouse splenocytes which is caused by BLyS protein. Therefore, the BLyS antibody can be used alone or in combination with other drugs in detecting, diagnosing, treating or screening diseases associated with abnormal expression or dysfunction of BLyS, such as autoimmune diseases, inflammatory diseases, infectious diseases, or proliferative diseases.

### Brief description of the drawings

The features and advantages of the present invention are described below with reference to the drawings.
Fig. 1 shows the detection result of the biological activity of purified hBLyS-ECD. As used herein, RLU represents the relative light unit.
Fig. 2A and Fig. 2B show the detection of expression level of BLyS protein in CHO-K1 recombinant cell line by using flow cytometry analysis method. Among them, 2C4 represents the clone number; 4C4 represents the clone number; antibody refers to the BLyS antibody (purchased from eBioscience); negative control refers to the isotype antibody control.
Fig. 3 shows the serum antibody titer of BALB/c and SJL mice after immunization with immunogen in ELISA detection assay.
Fig. 4 shows the activity of biotinylated hBLyS-ECD in ELISA detection assay
Fig. 5A and Fig. 5B show the reactivity of the purified BLyS antibody with biotinylated hBLyS-ECD in ELISA assay.
Fig. 6A and Fig. 6B show the results of enzyme-linked immunosorbent assay for detecting the reaction of the purified BLyS antibody and the BLyS homologous protein APRIL.
Fig.7 shows the reactivity of purified BLyS antibodies and human BLyS recombinant cells detected by flow cytometry analysis.
Fig.8 shows the reactivity of purified BLyS antibodies and cynomolgus BLyS recombinant cells using flow cytometry analysis.
Fig. 9A and Fig. 9B show the binding activity of purified BLyS antibody-blocked BLyS receptor BAFF R with biotinylated hBLyS-ECD.
Fig. 10 shows the inhibitory effect of purified BLyS antibodies on the proliferation of mouse B lymphocytes stimulated with hBLyS-ECD.
Fig. 11 shows the inhibitory effect of purified BLyS antibodies on the proliferation of mouse B lymphocytes stimulated with mouse BLyS-ECD.
Fig. 12 shows the reactivity of BLyS chimeric antibody with biotinylated hBLyS-ECD in ELISA assay.
Fig. 13 shows the binding activity of BLyS chimeric antibody-blocked BLyS receptor BAFF R with biotinylated hBLyS-ECD.
Fig. 14 shows the inhibitory effect of BLyS chimeric antibody on the proliferation of mouse B lymphocytes stimulated with hBLyS-ECD.
Fig. 15A-15C shows the effect of BLyS chimeric antibody and human antibody on the proportion of mouse B lymphocytes in splenocytes after hBLyS-ECD stimulation.
Fig. 16 shows the reactivity of BLyS humanized antibody with the biotinylated hBLyS-ECD in ELISA assay.
Fig. 17 shows the binding activity of BLyS humanized antibody-blocked BLyS receptor BAFF R with biotinylated hBLyS-ECD.
Fig. 18 shows the inhibitory effect of BLyS humanized antibody on the proliferation of mouse B lymphocytes stimulated with hBLyS-ECD.
Fig. 19A and Fig. 19B show the effect of BLyS humanized antibody on the proportion of mouse B lymphocytes in splenocytes and the level of IgA in serum after stimulation with hBLyS-ECD.

### Detailed description of the preferred embodiment

The following examples further illustrate the present invention and should not be construed to limit the present invention. Experimental methods without indicating specific conditions in the embodiments below can make reference to routine methods and conditions, or make choice according to product instructions.

The term room "temperature" as used in any of embodiment refers to the temperature of operating chamber in which the experiments is conducted, generally 15-30°C.

### Embodiment 1 Preparation of BLyS antibody by hybridoma technology

### (i) Preparation of immunogen

The nucleotide sequence (as shown in SEQ ID No. 131 of Sequence Listing) encoding the amino acid sequence of Ala134-Leu285 in the extracellular domain of human BLyS protein (BLyS-ECD) was cloned into a His-tagged pCPC vector (purchased from Invitrogen, V044-50) and plasmid was prepared according to established standard molecular biology methods as described in [Sambrook, J., Fritsch, E. F., and Maniatis, T. (1989). Molecular Cloning: A Laboratory Manual, Second Edition (Plainview, New York: Cold Spring Harbor Laboratory Press)]. HEK293 cells (purchased from Invitrogen) were transiently transfected (PEI, Polysciences) and expanded at 37 °C using FreeStyle™ 293 (purchased from Invitrogen). After 7 days, the cell culture liquid was collected and the cell components were removed by centrifugation to obtain a culture supernatant containing the extracellular domain of human BLyS protein. The culture supernatant was loaded on a Ni affinity column (purchased from GE Healthcare) while the changes in UV absorbance (A₂₈₀ nm) were monitored with a UV detector. After loading, the Ni affinity column was equilibrated with phosphate buffer (pH 7.4) containing 5% (v/v) sucrose and 0.01% (v/v) Tween-80 until the UV absorbance returned to baseline, and then gradient elution was carried out with 0-500 mM imidazole. The His-tagged extracellular domain of the human BLyS protein eluted from the Ni affinity column was collected, dialyzed and concentrated in an ultrafiltration tube at 4 °C using phosphate buffer (pH 7.4) containing 5% (v/v) sucrose and 0.01% (v/v) Tween-80. The dialyzed protein was sterile-filtered by 0.22 µm and aliquoted and stored at -80 °C to obtain the purified extracellular domain of human BLyS protein as immunogen (i.e., hBLyS-ECD). Before being used, the immunogen is subjected to a series of quality control tests, such as detection of concentration, purity, molecular weight, biological activity of the protein and the like.

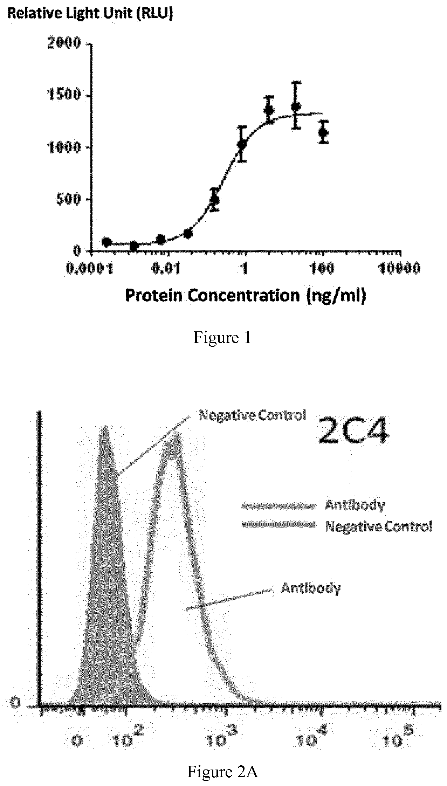

As used herein, the biological activity of the immunogen was tested by B cell proliferation assay (see Embodiment 5 for method). It was shown in Figure 1 and Table 3 that the immunogen can stimulate the proliferation of mouse B cells.

**Table 3 Biological Activity Detection of Immunogen**

| Relative light unit (RLU) | Protein Concentration (ng/mL) | | | | | | | | | |
|---|---|---|---|---|---|---|---|---|---|---|
| | 0 | 0.000256 | 0.00128 | 0.00640 | 0.0320 | 0.160 | 0.800 | 4.00 | 20.0 | 100 |
| | 63.64 | 92.57 | 56.70 | 118.02 | 173.56 | 497.54 | 1032.11 | 1365.34 | 1402.37 | 1147.81 |

### (ii) Construction of stable cell lines expressing human- or cyno-BLyS

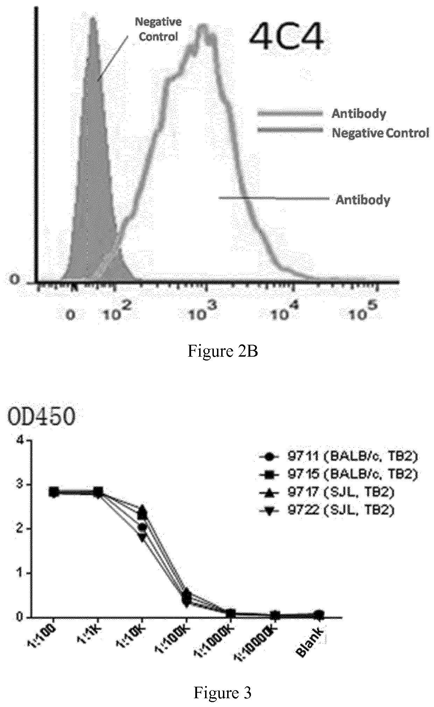

The amino acid sequence of human or cynomolgus BLyS (i.e., hBLyS or cyno BLyS) was mutated from arginine to histidine at position (Genebank accession numbers in NCBI are Q9Y275 and EHH58704.1, respectively), so that the constructed stable cell lines express complete human or cynomolgus BLyS without cleavage. The transfected cells were obtained by cloning the mutated nucleotide sequence (as shown in SEQ ID No. 132 and SEQ ID No. 133) containing full-length amino acid sequence of human or cynomolgus BLyS into the pLVX vector (purchased from Clontech), and then packaging lentivirus and subsequently infecting CHO-K1 cell line (purchased from Invitrogen) by Shanghai GenePharma Co., Ltd. After 72 hours, detection was performed using known BLyS antibody (purchased from eBioscience) by flow cytometry [see Manetta J et al., 2014, J Inflamm Res, 20 (7): 121-131]. When the transfected cells began to express human or cynomolgus BLyS, the cells were subcloned in 96-well plates by limited dilution method and incubated at 37 °C and 5% (v/v) CO₂ for about 2 weeks, and some wells of the monoclonal cells were selected and enlarged into 6-well plates. The amplified clones were further screened by flow cytometry using known BLyS antibody (purchased from eBioscience). Monoclonal cell lines with better growth and higher intensity of fluorescence were selected to expand the culture and cryopreserved in liquid nitrogen, and then human or cynomolgus BLyS stable cell lines were established. The results of specific selection are shown in Table 4 and Fig. 2A-2B, and the positive cell (%) in Table 4 refers to the percentage of positive cells in the total number of cells. It was shown in Table 4 that a series of BLyS-positive CHO-K1 cell lines were constructed.

**Table 4 FACS screening test results of CHO-K1 cells expressing human or cynomolgus BLyS protein**

| No. | Clone No. of transfected cell | BLyS Antibody | | IgG Subtype Control | |
|---|---|---|---|---|---|
| | | Positive Cell (%) | Mean fluorescence intensity | Positive Cell (%) | Mean fluorescence intensity |
| 1 | CHO-K1 hBLyS 1B3 | 89.15 | 365 | 0.83 | 31 |
| 2 | CHO-K1 hBLyS 1B4 | 84.87 | 239 | 0.36 | 23 |
| 3 | CHO-K1 hBLyS 2A3 | 77.50 | 198 | 0.18 | 19 |
| 4 | CHO-K1 hBLyS 2C4 | 91.65 | 295 | 0.84 | 39 |
| 5 | CHO-K1 hBLyS 2B3 | 72.46 | 189 | 1.07 | 23 |
| 6 | CHO-K1 hBLyS 3B2 | 87.47 | 295 | 0.24 | 29 |
| 7 | CHO-K1 hBLyS 4B3 | 86.85 | 262 | 0.21 | 27 |
| 8 | CHO-K1 hBLyS 4C2 | 90.78 | 299 | 0.79 | 31 |
| 9 | CHO-K1 cynoBLyS 1A4 | 82.04 | 412 | 0.22 | 31 |
| 10 | CHO-K1 cynoBLyS 1B2 | 87.49 | 412 | 0.10 | 19 |
| 11 | CHO-K1 cynoBLyS 2B3 | 94.66 | 442 | 0.20 | 16 |
| 12 | CHO-K1 cynoBLyS 3B2 | 89.91 | 422 | 0.17 | 19 |
| 13 | CHO-K1 cynoBLyS 4B1 | 92.28 | 742 | 0.71 | 26 |
| 14 | CHO-K1 cynoBLyS 4C2 | 95.00 | 676 | 0.28 | 24 |
| 15 | CHO-K1 cynoBLyS 4C4 | 94.25 | 705 | 0.20 | 27 |

### (iii) Preparation of hybridoma cells and screening of antibody

6-8 weeks old female BALB/c and SJL mice (purchased from Shanghai SLAC Laboratory Animal Co., Ltd.) were feed under SPF conditions. At the time of initial immunization, 0.2 mL of the immunogen (i.e., hBLyS-ECD) collected at step (i) was emulsified in Freund's complete adjuvant and then intraperitoneally injected, that is, each mouse was injected with 100 µg of immunogen. While booster, 0.2 mL of the immunogen was emulsified in incomplete Freund's adjuvant and then injected intraperitoneally, that is, each mouse was injected with 50 µg of immunogen. The interval between the initial immunization and first booster is two weeks and the interval between each booster is three weeks. Blood was collected 1 week after each booster, and the antibody titer and specificity of the immunogen in serum were tested by ELISA. The results were shown in Fig. 3 and Table 5. It was shown in Table 5 that the serum of mice immunized with immunogen bound to the immunogen to a different extent and presented an antigen-antibody reaction, wherein the highest dilution is about 1:1 million. The blank control was 1% (w/w) BSA, wherein the batch refers to the serum of mice on the seventh day after the second booster and the data in the table refers to OD₄₅₀ nm values.

**Table 5 Serum antibody titers of BALB/c and SJL mice after immunized with immunogen detected by ELISA**

| OD₄₅₀ₙₘ | Serum Dilution | | | | | | |
|---|---|---|---|---|---|---|---|
| Batch | 1:100 | 1:10³ | 1:10⁴ | 1:10⁵ | 1:10⁶ | 1:10⁷ | Blank control |
| 9711 (BALB/c, TB2) | 2.8110 | 2.8547 | 2.0439 | 0.3713 | 0.0994 | 0.0561 | 0.0879 |
| 9715 (BALB/c, TB2) | 2.8624 | 2.8593 | 2.3021 | 0.4848 | 0.0968 | 0.0526 | 0.0488 |
| 9717 (SJL, TB2) | 2.8312 | 2.8201 | 2.4538 | 0.5821 | 0.1084 | 0.0553 | 0.0501 |
| 9722 (SJL, TB2) | 2.7962 | 2.7751 | 1.8081 | 0.3297 | 0.0822 | 0.0516 | 0.0468 |

B. Female BALB/c and SJL mice at 6-8 weeks of age (purchased from Shanghai SLAC Laboratory Animal Co., Ltd.) were feed under SPF conditions. At the time of initial immunization, 25µL of immunogen (hBLyS-ECD), oligonucleotide (CpG) and GERBU adjuvant were mixed and then injected in tarsal joints of mice, that is, each mouse was injected with 10 µg of immunogen. Upon booster, 25µL of the immunogen and GERBU adjuvant were mixed and then injected in tarsal joints of mice, that is, each mouse was injected with 5 µg of immunogen. The interval between the initial immunization and first booster is 3-4 days. Blood was collected 1 week after the second booster, and the antibody titer and specificity to the immunogen in serum were measured by ELISA. After the second booster, the serum antibody titer detected by ELISA reached 1: 10000 or more.

Before the completion of steps A and B, 50 µg or 5 µg of immunogen were injected into the peritoneal cavity or tarsal joints of each selected last immunized mouse, respectively, and the mice were sacrificed 5 days later and the splenocytes were harvested. NH₄OH was added by a final concentration of 1% (w/w) to lyse the red blood cells in the splenocytes to prepare a splenocyte suspension. The cells were washed three times with centrifugation at 1000 rpm in DMEM basal medium (purchased from Invitrogen), then the viable cells were mixed with mouse myeloma cells SP2/0 (purchased from ATCC) at a ratio of 5:1, and a high-efficiency electrofusion method was used for cell infusion (See METHODS IN ENZYMOLOGY, VOL. 220). The fused cells were diluted into DMEM medium containing 20% (w/w) fetal bovine serum and 1×HAT. Then 1×10⁵ cells in 200µL DMEM medium was aliquoted into target well of 96-well cell culture plates and incubated in a 5% (v/v) CO₂ incubator at 37 °C. After 14 days, the supernatant of the cell fusion plates was screened by indirect ELISA. Positive clones with OD₄₅₀ₙₘ> 1.0 in ELISA assay were enlarged to 24-well plates and cultured in HT DMEM containing 10% (w/w) fetal bovine serum at 37 °C under a condition of 5% (v/v) CO₂. After incubating for 3 days, the culture medium enlarged in 24-well plates was centrifuged, the supernatant was collected and subject to the antibody subtype analysis. Among them, the binding activity to hBLyS-ECD was determined by ELISA (see Embodiment 3A and Embodiment 3B respectively for the method of detecting the binding activity), and the blocking activity of the antibody sample on BLyS receptor was determined by ligand-receptor binding assay (see Embodiment 4 for the detection method of blocking activity).

According to the result of 24-well plate screening, hybridoma cells with OD₄₅₀ₙₘ> 1.0 in ELISA assay and the rate of BLyS receptor blocking by hybridoma cell culture supernatant reaching 60% in ligand-receptor binding assay were selected as positive clones that meet the requirements herein. Selected hybridoma cells were subcloned in a 96-well plate by limited dilution method and cultured in DMEM medium (purchased from Invitrogen) containing 10% (w/w) FBS at 37 °C under a condition of 5% (v/v) CO₂. 10 days after subcloning, ELISA was used for preliminary screening, and single positive monoclone was selected and expanded into 24-well plate for further culture. After 3 days, the biological activity was evaluated using receptor-ligand binding assay. Where the evaluation criteria is that OD₄₅₀ₙₘ should higher than 1.0 in ELISA assay, and the rate of BLyS receptor block-inhibited by hybridoma cell culture supernatant should reach 60% in ligand-receptor binding assay.

According to the detection results of 24-well plate samples, the optimal clones were selected and expanded in DMEM culture medium (purchased from Invitrogen) containing 10% (w/w) FBS at 37 °Cunder a condition of 5% (v/v) CO₂. The optimal clones were obtained and cryopreserved in liquid nitrogen, and the optimal hybridoma cells were used for subsequent production and purification of antibody.

### (iv) Production and purification of the lead antibody

Since hybridoma cells produce lower concentration of antibody, only about 1-10 µg/mL, and the concentration of antibody varies greatly and many biological activity analysis methods were interfered in different level by various proteins produced by cells in culture medium and components of fetal bovine serum in culture medium, so it is necessary to carry out small-scale (1-5 mg) of antibody production and purification.

The hybridoma cells harvested at step (iii) were inoculated into T-75 cell culture flasks and acclimated for 3 generations using a production medium (Hybridoma serum free medium, purchased from Invitrogen). When they were in healthy condition, the cells were inoculated into roller bottle. 500 mL of production medium was poured to each 2-liter roller bottle at a cell density of 1.0×10⁵ cells/mL. The bottle caps were covered and the roller bottles were placed on a rotary machine in a 37°C incubator at a 3 rpm/min of rotating speed. After 14 days of continuous rotation culture, the cell culture was collected and the cells were removed by filtration through a 0.45 µm filter until the culture supernatant were clarified. The clarified culture supernatant can be purified immediately or stored at -30 °C.

Monoclonal antibodies in the 300 mL of clarified culture supernatant of hybridoma cells was purified with a 2 mL of Protein G column (purchased from GE Healthcare). The Protein G column was first equilibrated with equilibration buffer (PBS phosphate buffer, pH 7.2) and then the clarified culture supernatant was loaded onto the Protein G column at a flow rate of 3 mL/min. After loading, the Protein G column was washed with equilibration buffer, and the volume of the equilibration buffer was 4 times of the bed volume of Protein G column. The monoclonal antibody bound to Protein G column was eluted with elution buffer (0.1M glycine hydrochloride buffer, pH 2.5) and the elution was monitored by an ultraviolet detector (A280 ultraviolet absorption peak). Eluted monoclonal antibody was collected and the pH was neutralized by adding 10% (v/v) of 1.0 M Tris-HCl buffer. Then elution was dialyzed overnight in PBS phosphate buffer immediately. The next day the buffer was changed once and dialysis was continued for 3 hours. Dialyzed monoclonal antibody was collected and sterile filtered using a 0.22 µm filter, and sterile stored the harvested purified BLyS antibody as a lead antibody.

The protein concentration (A280/1.4), purity, endotoxicity (Lonza kit) and the like of purified BLyS antibody was tested and the results were shown in Table 6. It was shown in the result that the lead antibody contained 1.0 EU/mg concentration of endotoxin or less.

**Table 6 Detection and Analysis of Purified BLyS Antibody**

| Clone No. | Antibody Purification | Protein Conc. (mg/mL) | Endotoxin (EU/mg) |
|---|---|---|---|
| 35E6F7C3 | > 90% | 0.86 | 0.16 |
| 8E7D9C7F5 | > 90% | 1.5 | 0.05 |
| 20D1B6E9E5 | > 90% | 0.73 | 0.13 |
| 78C11D2D12 | > 90% | 0.956 | 0.26 |
| 89A2G5E7 | > 90% | 0.734 | 0.23 |
| 97E7B3F2 | > 90% | 0.583 | 0.93 |
| 97A3C2H4 | > 90% | 0.504 | 0.30 |
| 67A2E1D10 | > 90% | 0.5 | 0.16 |
| 111D10D6G3 | > 90% | 0.25 | 0.50 |
| 93C6F10D3 | > 90% | 0.485 | 0.66 |

### Embodiment 2 Preparation of BLyS antibody by phage display technology

### (i) Biotinylation of BLyS protein

Biotin-X-X-NHS (purchased from Sigma Aldrich) and the immunogen prepared in embodiment 1 (hBLyS-ECD) were mixed at a molar ratio of 7: 1 and stood for 30 minutes at room temperature before addition of 50 mM final concentration of 1 M NH₄Cl to terminate the reaction. Biotinylated immunogens (i.e., biotinylated hBLyS-ECDs) were then obtained by dialysis overnight in PBS phosphate buffer to remove free biotin. The concentration of biotinylated hBLyS-ECD was determined using BCA protein assay kit (purchased from Pierce).

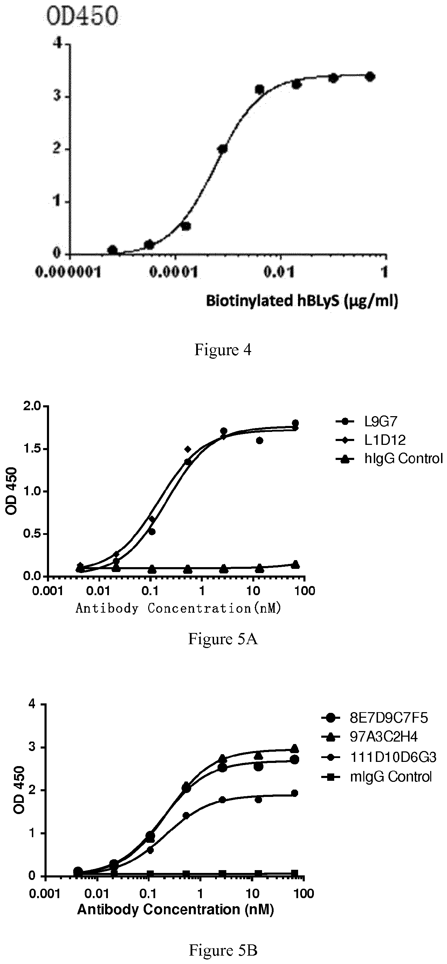

The activity of biotinylated hBLyS-ECD was determined by ELISA. The BLyS antibody (purchased from GSK) was diluted to 1 µg/mL with PBS, 50 µL/well of diluted antibody was aliquoted into the ELISA microplate and incubated overnight at 4 °C. After blocking with ELISA blocking solution [PBS phosphate buffer containing 1% (w/v) BSA and 0.05% (v/v) Tween-20, pH 7.4] for 2 hours at 37 °C, gradient dilutions of biotinylated hBLyS-ECD were added in and incubated for 1 hour at 37 °C. Streptavidin-labeled horseradish peroxidase (purchased from Sigma, product number S5512) was pipetted in and incubated for 30 minutes at room temperature. A 100 µL/well of TMB color developing solution was added in and incubated for 15 minutes at room temperature, then 5 µL of IN hydrochloric acid was used to stop the color reaction and the value at OD₄₅₀ₙₘ was read by ELISA plate reader. As shown in Figure 4 and Table 7, the results demonstrated that the biotinylated hBLyS-ECD could bind to BLyS antibodies.

**Table 7 ELISA detection of the binding of Biotinylated Immunogen to BLyS Antibodies**

| OD₄₅₀ₙₘ | Concentration of Biotinylated Immunogen(ng/mL) | | | | | | | |
|---|---|---|---|---|---|---|---|---|
| | 0.000006 | 0.000032 | 0.00016 | 0.0008 | 0.004 | 0.02 | 0.1 | 0.5 |
| | 0.08 | 0.19 | 0.54 | 2.01 | 3.14 | 3.24 | 3.36 | 3.39 |

### (ii) BLyS antibody identified by phage display technology

Lead antibody was identified from human naive single-chain variable fragment (ScFv) phage display library (constructed by Shanghai ChemPartner. Co., Ltd.). Antibody binding to BLyS was obtained by four rounds of biopanning. The detailed process is as follows:
Streptavidin diluted in PBS to a final concentration of 12.5 µg/mL was aliquoted into immuno tubes at 1 mL/tube, and incubated overnight at 4 °C. After washing the immuno tubes three times with PBS, the biotinylated hBLyS-ECD prepared at step (i) was aliquoted into a half of the immuno tubes with 50 µg per tube, and shaken at room temperature for 1 hour. After washing three times with PBS buffer, the immuno tubes were blocked for 2 hours at room temperature with 10 mL of 2% (w/v) blocking solution [PBS buffer containing 2% (w/v) skimmed milk powder]. Meanwhile, 1 mL of phage ScFv antibody library and blocking solution were added and shaken for 2 hours at room temperature in the other half of immuno tubes. And the control tube was set by adding the same volume of blocking solution. The blocking solution in control tubes and the immuno tubes adding with biotinylated hBLyS-ECD were removed, and the blocked phage ScFv antibody library was added and the tubes were oscillated at room temperature for 2 hours. Immuno tubes and control tubes were rinsed 5 times with PBST [PBS buffer containing 0.1% (v/v) Tween-20] first and then rinsed 5 times with PBS buffer. Where the time of rinse increased by 5 in each round. After rinsing, 1 mL of 10 µg/mL trypsin was aliquoted into each tube, and the phage bound to the biotinylated hBLyS-ECD was eluted by incubation at 37 °C for 30 minutes. 1 mL of trypsin solution was pipetted into 4 mL of *E. coli* TG1 (purchased from LUCIGEN) in logarithmic growth phase and allowed for incubating at 37°C for 30 minutes to obtain a culture solution of TG1. The culture solution of TG1 was serially diluted, plated, and cultured overnight at 37°C. The clones obtained from immuno tubes bound to the biotinylated hBLyS-ECD and control tubes were calculated, and 20-30 clones were selected for sequencing.

Meanwhile, the clones on the plates were washed with 2YT medium (2YT medium was prepared by adding 10 g of yeast extract, 16 g of tryptone and 5 g of NaCl to 1 L of water, and adjusting the pH to 7.0 with NaOH and autoclaving) and collected, then inoculated into fresh medium and cultured untill logarithmic phase at 37°C. Helper phage M13K07 (purchased from NEB, Cat. No. N0315S) was added, mixed and stood at 37°C for 30 minutes. Then, the solution was incubated with shaking at 37°C for 30 minutes and centrifuged at 4000 rpm for 10 minutes, and the cells were collected and added with fresh medium, then incubated with shaking at 30°C for 4 hours. After centrifugation at 4000 rpm for 30 minutes, the supernatant was collected, and 1/4 volume of 2.5 M NaCl solution containing 5×PEG was added and allowed for placing on ice overnight. After centrifugating at 4000 rpm for 30 minutes at 4°C, the phage precipitation was collected and dissolved in PBS buffer. Residual cell fragments were removed by centrifugating at 10,000 rpm for 10 minutes and the supernatant was collected for biopanning in the next round.

The binding activity of screened scFv antibody with BLyS protein was determined by ELISA. Clones with OD₄₅₀ₙₘ higher than 1.0 were selected for sequencing to get clones with various CDRH3 sequences. Then according to the results of FACS and ligand-receptor binding assay, clones with MFI value higher than 30 in FACS assay and blocking inhibitory rate of cell lysis supernatant on BLyS receptors reaching 60% in ligand-receptor binding assay were selected as the positive clones that meet the criteria.

### (iii) Production and purification of antibody

Based on the sequencing results of the positive clones (see details in Embodiment 7 below), primers (detailed primer sequences are shown in Table 8) were designed to amplify light chain and heavy chain variable regions by PCR, respectively. A 50 µL of reaction system including 0.5 µL of plasmid extracting from *E. coli* TG1 transfected with positive clones), 10 pmol of each primer, 0.5 µL of DNA polymerase and a suitable buffer system was set up. The PCR program consisted of pre-denaturation at 95 °C for 2 minutes, 30 cycles of denaturation at 95 °C for 15 seconds, annealing at 55 °C for 30 seconds and extension at 68 °C for 45 seconds, followed by 10 minutes extension at 68 °C to obtain the PCR products. The DNA polymerase used in PCR was purchased from Invitrogen, with Cat. No. 12344, while the buffer system is a buffer system purchased and used with the DNA polymerase as a set. 5 µL of PCR products were used for agarose gel electrophoresis, and positive samples were purified by column recovery kit NucleoSpin® Gel & PCR Clean-up, which is purchased from MACHEREY-NAGEL with Cat. No. 740609. Ligation was carried out as follows: 3 µL of inserted fragment, 0.5 µL of digested expression vector, 0.5 µL of recombinase Exnase and 2 µL of buffer in 10µL of reaction system reacted at 37 °C for half an hour to obtain the ligated products, namely the constructed recombinant vector. The recombinase was purchased from Vazyme with Cat. No. C112-01/02; the buffer was purchased for using with the recombinase as a set; heavy chain variable region was directionally cloned into the expression vector containing the signal peptide and heavy chain IgG1 constant region of human antibody (the expression vector was purchased from Invitrogen and the recombination process was performed by Shanghai ChemPartner Co., Ltd.), light chain variable region was directionally cloned into the expression vector containing the signal peptide and light chain kappa or lambda (only for the following light chain of the L1D12 antibody) constant region of human antibody (the expression vector was purchased from Invitrogen and the recombination was performed by Shanghai ChemPartner Co., Ltd.). 10 µL of the ligation products were aliquoted into 50 µL of competent cells (Ecos 101competent cells, purchased from Yeastern, Cat. No. FYE607) and placed in an ice bath for 30 minutes. After that, above competent cells were heat shocked at 42 °C for 1 minute in water bath and placed on ice for 2 minutes, then mixed with 500 µL of antibiotic-free 2YT medium and resuscitated at 200 RPM for 45 minutes on a shaker at 37 °C, and 200 µL of above mix was pipetted to spread on LB solid medium containing 100µg/mL of ampicillin and overnight incubated in 37 °C incubator. On the next day, colony PCR was performed in a 30 µL of PCR system using primers pTT-EF1a-F and pSV40 (whose nucleotide sequences are shown in SEQ ID No. 134-135, respectively) specific for the expression vector. The reaction system of colony PCR comprises: 1 µL of each primer, 10 µL of PCR pre-mixture (purchased from Novoprotein) and replenish to a total volume of 20 µL with water. Bacterial colony was picked into the PCR system by a pipette tip with continuously pipetting for several times, and then 0.5 µL of which was picked onto another LB solid plate containing 100 µg/mL ampicillin for the preservation of the bacterial strain. After the PCR reaction, 5 µL of products were picked out for detection by agarose gel electrophoresis and the positive samples were sequenced and analyzed [see Kabat, "Sequences of Proteins of Immunological Interest," National Institutes of Health, Bethesda, Md. (1991)].

**Table 8 Primer Sequence NO. of Positive Clones**

| Clone No. | | Forward Primer | Reverse Primer |
|---|---|---|---|
| 2-1G11 | V_{H} | 176 | 177 |
| | V_{L} | 178 | 179 |
| L9G7 | V_{H} | 180 | 181 |
| | V_{L} | 182 | 183 |
| L1D12 | V_{H} | 184 | 185 |
| | V_{L} | 186 | 187 |

After validating by colony PCR, expression vectors containing the aforesaid recombinant antibody light chain and heavy chain with correct sequences were transiently transfected into FreeStyle™ 293-F cells (purchased from Invitrogen) to produce antibodies. At the time of transfection, the density of 293-F cells should among 1-1.5×10⁶ cells/mL, and 100 µg of cells require 100 µg of aforesaid constructed DNA vectors (the mass ratio of the recombinant light chain vector to the recombinant heavy chain vector is 3:2) and 200 µg of transfection reagent polyethyleneimine (PEI). The DNA vectors and PEI were pipetted into the culture medium respectively, and allowed to stand at room temperature for 5 minutes before filtering through a 0.22-µm of membrane filter, and then the mixture was obtained by mixing DNA vectors and PEI and allowed to stand at room temperature for 15 minutes. Then the mixture was aliquoted into the cells slowly and cultured at 37 °C in 8% (v/v) CO₂ incubator at 120 rpm. The day after transfection, 0.5% (v/v) of peptone was pipetted into the cell culture medium. After 6-7 days, the cell culture medium was centrifuged at 3500g for 30 minutes. The supernatant was collected and filtered through a 0.22µm filter.

Monoclonal antibody in 200 mL of clarified supernatant was purified using 1 mL of Protein A column (purchased from GE Healthcare). The Protein A column was first equilibrated with equilibration buffer (PBS phosphate buffer, pH 7.2) and the clarified supernatant was loaded onto the Protein A column at a flow rate of 3 mL/min. After loading, the Protein A column was washed with equilibration buffer, where the volume of the equilibration buffer was 5 times bed volume of Protein A column. BLyS antibody bound to Protein A column was eluted with elution buffer (0.1M glycine hydrochloride buffer, pH 2.5) and the elution was monitored by an ultraviolet detector (A280 ultraviolet absorption peak). Eluted antibody was collected and the pH was neutralized by adding 10% (v/v) of 1.0 M Tris-HCl buffer. Then dialysis was performed immediately in PBS phosphate buffer overnight. The next day the buffer was changed once and dialysis was continued for 3 hours. Dialyzed BLyS antibody was collected and sterile filtered using a 0.22 µm filter, and stored the purified BLyS antibody sterilely as a lead antibody.

The lead antibody was tested for concentration (A280/1.4), purity, and endotoxicity (Lonza kit) of protein, etc, the results were shown in Table 9. It was found that the endotoxin concentration of the lead antibody was 1.0 EU/mg or less.

**Table 9 Analysis of purified BLyS Antibody**

| Clone No. | Antibody Conc. | Protein Conc. (mg/mL) | Endotoxin (EU/mg) |
|---|---|---|---|
| 2-1G11 | >90% | 1.65 | 0.16 |
| L9G7 | >90% | 2.36 | 0.26 |
| L1D12 | >90% | 2.73 | 0.34 |

### Embodiment 3 Detection of the lead antibody

### A. Detection of the binding of antibody to BLyS protein by Enzyme-linked immunosorbent assay (ELISA)

To determine the binding activity of the lead antibodies harvested from Embodiment 1 and 2 to human BLyS protein and human APRIL protein of the BLyS protein family, respectively, ELISA was performed.

Streptavidin was first diluted to a final concentration of 1.0 µg/mL with PBS, and then 50 µL of diluted streptavidin per well was aliquoted into 96-well ELISA plate, which was subsequently sealed with plastic film and incubated at 4 °C overnight. On the next day the plate was washed four times with the plate washing buffer [PBS buffer containing 0.05% (v/v) Tween20] and added with the blocking solution [PBS buffer containing 0.05% (v/v) Tween20 and 1% (w/w) BSA] and blocked for 2 hours at 37 °C. The blocking solution was removed, the biotinylated hBLyS-ECD prepared in Embodiment 2 was diluted with PBS to a final concentration of 50 ng/mL and then aliquoted into the 96-well ELISA plate at 50 µL per well and incubated for 1 hour at 37 °C. After rinsing the plate four times with the plate washing buffer [PBS buffer containing 0.05% (v/v) Tween20], 100 µl of the purified lead antibody harvested in Embodiment 1 and 2 was pipetted into each well. After incubation for 1 hour at 37 °C, the plate was rinsed four times with the plate washing buffer [PBS buffer containing 0.05% (v/v) Tween20]. HRP-labeled (horseradish peroxidase) secondary antibody (purchased from Sigma) was added in and incubated at 37 °C for 1 hour. The plate was then rinsed four times with the plate washing buffer [PBS buffer containing 0.05% (v/v) Tween20]. 100 µL of TMB substrate was aliquoted into each well and after incubation for 5 minutes at room temperature, 100 µL of stop solution (1.0 N HCl) was aliquoted into each well. A₄₅₀ₙₘ values were read via an ELISA plate reader (SpectraMax M5e purchased from Molecular Devices) and the results were shown in Figures 5A-5B and Table 10. Table 10 showed that the purified antibody can bind to the BLyS recombinant protein in ELISA assay. Data shown in the table is OD number at 450 nm.

**Table 10 ELISA detection of the binding of purified BLyS antibody with biotinylated hBLyS-ECD**

| OD₄₅₀nm | Antibody Cone. (nM) | | | | | | | |
|---|---|---|---|---|---|---|---|---|
| Clone No. | 66.7 | 13.3 | 2.67 | 0.534 | 0.107 | 0.0213 | 0.00427 | 0 |
| L9G7 | 1.8062 | 1.6016 | 1.7138 | 1.3508 | 0.5280 | 0.1814 | 0.0853 | 0.0710 |
| L1D12 | 1.7492 | 1.6062 | 1.6476 | 1.4971 | 0.6781 | 0.2618 | 0.1349 | 0.1099 |
| hIgG Control | 0.1483 | 0.1016 | 0.0956 | 0.0916 | 0.0905 | 0.1106 | 0.1082 | 0.1027 |
| 8E7D9C7F5 | 2.7224 | 2.5619 | 2.5304 | 2.0564 | 0.9458 | 0.2909 | 0.1151 | 0.0564 |
| 97A3C2H4 | 2.9812 | 2.8238 | 2.7511 | 2.1167 | 0.8988 | 0.2826 | 0.1053 | 0.0603 |
| 111D10D6G3 | 1.9376 | 1.7802 | 1.7823 | 1.4143 | 0.6127 | 0.1935 | 0.0820 | 0.0617 |
| mIgG Control | 0.0708 | 0.0627 | 0.0659 | 0.0594 | 0.0562 | 0.0595 | 0.0695 | 0.0574 |

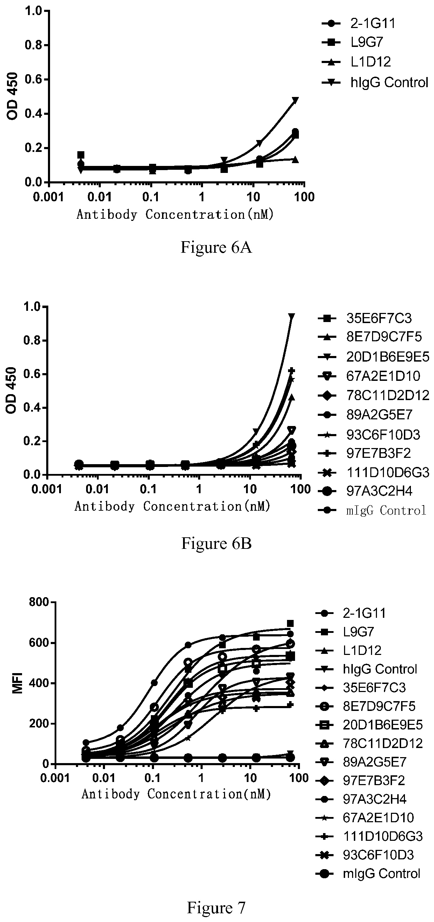

To test whether the BLyS antibody cross-reacted with human APRIL protein, APRIL protein (purchased from R & D Systems) was first diluted to a final concentration of 1.0 µg/mL with PBS, and then 50 µL of which per well was aliquoted into a 96-well ELISA plate, and the plate was subsequently sealed with plastic film and incubated at 4 °C overnight. On the next day the plate was rinsed four times with the plate washing buffer [PBS buffer containing 0.05% (v/v) Tween20] and a blocking solution [PBS buffer containing 0.05% (v/v) Tween20 and 1% (w/w) BSA] was added, then the protein was blocked at 37 °C for 2 hours. The blocking solution was removed and 100 µL per well of the lead antibodies prepared in Embodiment 1 and 2 were added in. After incubation for 1 hour at 37 °C, the plate was rinsed four times with the plate washing buffer [PBS buffer containing 0.05% (v/v) Tween20]. HRP-labeled (horseradish peroxidase) secondary antibody (purchased from Sigma) was added in and incubated at 37 °C for 1 hour. The plate was then rinsed four times with the plate washing buffer [PBS buffer containing 0.05% (v/v) Tween20]. 100 µL of TMB substrate was aliquoted into each well and after incubation at room temperature for 5 minutes, 100 µL of stop solution (1.0 N HCl) was aliquoted into each well. A₄₅₀ₙₘ values were read via an ELISA plate reader (SpectraMax M5e purchased from Molecular Devices) and the results were shown in Figures 6A-6B and Table 11. Table 11 showed that the lead antibodies didn't bind to the recombinant APRIL protein which is the most homologous to BLyS protein. As used herein, the IgG control is human IgG and the data in the table is OD number at 450 nm.

**Table 11-1 ELISA detection of the binding of BLyS antibodies with APRIL proteins**

| OD₄₅₀ₙₘ | Antibody Cone. (nM) | | | | | | | |
|---|---|---|---|---|---|---|---|---|
| Clone No. | 66.7 | 13.3 | 2.67 | 0.534 | 0.107 | 0.0213 | 0.00427 | 0 |
| 2-1G11 | 0.2964 | 0.1374 | 0.09015 | 0.0668 | 0.0699 | 0.07335 | 0.1070 | 0.0847 |
| L9G7 | 0.2755 | 0.1072 | 0.0751 | 0.07405 | 0.0861 | 0.0777 | 0.1606 | 0.0638 |
| L1D12 | 0.1334 | 0.1240 | 0.1019 | 0.0757 | 0.06785 | 0.0854 | 0.1215 | 0.0625 |
| hIgG Control | 0.4768 | 0.2263 | 0.1284 | 0.0793 | 0.0722 | 0.0804 | 0.0693 | 0.0711 |

**Table 11-2 ELISA detection of the binding of BLyS antibodies with APRIL proteins**

| OD₄₅₀ₙₘ | Antibody Conc. (nM) | | | | | | | |
|---|---|---|---|---|---|---|---|---|
| Clone No. | 66.7 | 13.3 | 2.67 | 0.534 | 0.107 | 0.0213 | 0.00427 | 0 |
| 35E6F7C3 | 0.07025 | 0.0567 | 0.05605 | 0.05435 | 0.05365 | 0.05315 | 0.0553 | 0.05425 |
| 8E7D9C7F5 | 0.46405 | 0.1156 | 0.07025 | 0.0599 | 0.05575 | 0.0565 | 0.05715 | 0.05515 |
| 20D1B6E9E5 | 0.942 | 0.25655 | 0.07755 | 0.0611 | 0.0543 | 0.05465 | 0.0535 | 0.055 |
| 67A2E1D10 | 0.26415 | 0.0898 | 0.0673 | 0.05805 | 0.0588 | 0.05625 | 0.0553 | 0.05305 |
| 78C11D2D12 | 0.13785 | 0.0703 | 0.0613 | 0.05525 | 0.05485 | 0.05345 | 0.0564 | 0.05645 |
| 89A2G5E7 | 0.19595 | 0.0973 | 0.0614 | 0.0565 | 0.0548 | 0.0547 | 0.0578 | 0.06555 |
| 93C6F10D3 | 0.56985 | 0.1712 | 0.07455 | 0.0584 | 0.05505 | 0.0568 | 0.0546 | 0.0568 |
| 97E7B3F2 | 0.6204 | 0.182 | 0.0707 | 0.0556 | 0.0705 | 0.05375 | 0.0535 | 0.0533 |
| 111D10D6G3 | 0.0681 | 0.05725 | 0.05835 | 0.0525 | 0.05355 | 0.05485 | 0.0538 | 0.0547 |
| 97A3C2H4 | 0.17275 | 0.0848 | 0.06165 | 0.05895 | 0.05615 | 0.0559 | 0.0625 | 0.0566 |
| mIgG Control | 0.102 | 0.06245 | 0.05575 | 0.0536 | 0.0533 | 0.05365 | 0.05865 | 0.0716 |

### B. Detection of the binding of antibody with BLyS-expressing cells by fluorescence-activated cell sorting assay (FACS)

The CHO-K1-hBLyS stable cell line and the CHO-K1-cynoBLyS stable cell line obtained from step (ii) of Embodiment 1 were enlarged and cultured to 90% confluency in T-175 cell culture flask. The medium was aspirated completely, and the cells were rinsed once with PBS buffer (purchased from Invitrogen) and then treated with cell dispersion solution (TrypLE™ Express Enzyme, purchased from Life technology) and collected. The detailed steps are as follows: the cells were rinsed once with PBS buffer, after counting, the cells were diluted to 2×10⁶ cells/ml with PBS buffer, blocked with 2% (w/w) fetal bovine serum and incubated on ice for 30 minutes. 200 µL of which per well was aliquoted into a 96-well FACS plate and centrifuged at 2000 rpm at 4°C, then each 100 µL of the lead antibodies obtained from Embodiment 1 and 2 were aliquoted into each well and incubated on ice for 1 hour. The cells were washed twice with FACS buffer [PBS buffer containing 2% (w/w) FBS], and then mixed with 100 µL per well of fluorescence (Alexa 488) labeled secondary antibody (purchased from Invitrogen) on ice for 1 hour. After centrifuged and rinsed 3 times with FACS buffer, cells were suspended with 200µL of FACS buffer and the results were detected and analyzed by FACS (FACS Calibur, purchased from BD). The results were shown in Figures 7-8 and Tables 12-13. It was shown in Tables 12-13 that the lead antibody could bind to human BLyS protein on the cell surface and the lead antibody could cross-react with cynomolgus BLyS. The data in the table is MFI, the average fluorescence intensity of the cell population.

**Table 12 Detection of the binding of BLyS antibody to human BLyS recombinant cells (CHO-K1 hBLyS) by flow cytometry**

| MFI | Antibody Conc. (nM) | | | | | | | |
|---|---|---|---|---|---|---|---|---|
| Clone No. | 66.7 | 13.3 | 2.67 | 0.534 | 0.107 | 0.0213 | 0.00427 | 0 |
| 2-1G11 | 645.0 | 632.9 | 627.1 | 590.6 | 402.7 | 180.5 | 106.8 | 185.5 |
| L9G7 | 697.0 | 629.6 | 590.7 | 469.3 | 226.4 | 93.0 | 51.6 | 101.3 |
| L1D12 | 549.9 | 522.4 | 503.3 | 413.1 | 205.1 | 89.5 | 54.4 | 101.2 |
| hIgG Control | 52.0 | 35.1 | 33.1 | 33.4 | 32.9 | 33.6 | 32.3 | 27.2 |
| 35E6F7C3 | 608.6 | 528.3 | 437.4 | 247.3 | 110.4 | 54.9 | 39.2 | 52.4 |
| 8E7D9C7F5 | 596.2 | 569.5 | 532.1 | 504.5 | 282.3 | 122.0 | 67.4 | 151.6 |
| 20D1B6E9E5 | 534.7 | 498.0 | 473.9 | 401.5 | 193.5 | 83.6 | 46.8 | 31.8 |
| 78C11D2D12 | 355.5 | 336.0 | 299.5 | 248.9 | 132.0 | 61.3 | 39.5 | 31.2 |
| 89A2G5E7 | 431.1 | 405.8 | 373.9 | 192.4 | 92.1 | 48.1 | 37.0 | 31.0 |
| 97E7B3F2 | 406.0 | 349.2 | 332.0 | 318.2 | 174.0 | 78.7 | 48.2 | 32.4 |
| 97A3C2H4 | 525.0 | 460.1 | 448.0 | 340.3 | 158.0 | 69.8 | 44.0 | 31.2 |
| 67A2E1D10 | 432.4 | 371.2 | 279.0 | 128.6 | 60.0 | 37.1 | 33.2 | 31.2 |
| 111D10D6G3 | 295.5 | 276.0 | 264.9 | 246.6 | 150.3 | 65.5 | 41.2 | 32.4 |
| 93C6F10D3 | 391.6 | 327.0 | 316.7 | 306.0 | 174.6 | 76.6 | 44.9 | 31.6 |
| mIgG Control | 33.6 | 31.7 | 31.0 | 30.7 | 31.6 | 32.7 | 30.9 | 30.3 |

**Table 13 Detection of the binding of BLyS antibody to cynomolgus BLyS recombinant cells (CHO-K1 cynoBLyS) by flow cytometry**

| MFI | Antibody Conc. (nM) | | | | | | | |
|---|---|---|---|---|---|---|---|---|
| Clone No. | 66.7 | 13.3 | 2.67 | 0.534 | 0.107 | 0.0213 | 0.00427 | 0 |
| 2-1G11 | 1456.4 | 1449.6 | 1480.8 | 1400.5 | 816.2 | 331.8 | 175.6 | 21.2 |
| L9G7 | 1342.8 | 1282.4 | 1276.1 | 1009.5 | 386.8 | 143.3 | 63.5 | 20.6 |
| L1D12 | 1175.8 | 1133.8 | 1166.9 | 900.2 | 363.7 | 134.7 | 66.7 | 21.2 |
| hIgG Control | 44.2 | 26.7 | 22.7 | 22.6 | 22.8 | 22.4 | 22.0 | 20.7 |
| 35E6F7C3 | 1131.2 | 1080.3 | 953.1 | 501.0 | 176.9 | 66.3 | 32.6 | 20.3 |
| 8E7D9C7F5 | 1173.8 | 1167.6 | 1128.8 | 1051.3 | 476.6 | 164.9 | 74.4 | 19.8 |
| 20D1B6E9E5 | 1034.3 | 978.3 | 933.0 | 808.3 | 349.1 | 118.1 | 50.4 | 19.8 |
| 78C11D2D12 | 664.0 | 635.8 | 594.2 | 480.3 | 234.8 | 106.0 | 37.7 | 20.2 |
| 89A2G5E7 | 884.0 | 828.6 | 725.8 | 355.2 | 124.6 | 53.6 | 32.1 | 21.2 |
| 97E7B3F2 | 770.2 | 709.9 | 704.6 | 687.6 | 288.3 | 112.0 | 53.0 | 22.8 |
| 97A3C2H4 | 987.3 | 943.1 | 955.6 | 681.8 | 242.7 | 120.4 | 44.3 | 20.0 |
| 67A2E1D10 | 842.1 | 738.2 | 517.7 | 213.4 | 76.6 | 35.3 | 24.7 | 20.3 |
| 111D10D6G3 | 539.0 | 524.1 | 510.4 | 477.9 | 221.7 | 108.0 | 38.8 | 20.4 |
| 93C6F10D3 | 784.9 | 714.2 | 729.4 | 705.8 | 308.3 | 112.4 | 46.9 | 21.9 |
| mIgG Control | 21.6 | 20.7 | 21.2 | 20.0 | 19.7 | 19.8 | 20.4 | 19.8 |

### C. Determination of affinity constant of BLyS antibody

Affinity constants were determined using a Biacore X100 instrument (purchased from GE Healthcare). Detailed operations and methods referred to the instrument manuals and detailed methods provided by manufacturers, specifically: determining affinity constant using a CM5 chip (Sensor Chip CM5, purchased from GE Healthcare). Firstly, the CM5 chip was activated with 50 mM NaOH, 50 mM NHS and 200 mM EDC mixed by volume ratio of 1:1 sequentially, then anti-human Fc fragment antibody (purchased from Genway) was diluted to 16.4 µg/ml with 10 mM sodium acetate buffer (pH5.0) and coupled reacted with CM5 chip. Then 1 M ethanolamine was injected in chip to block the remaining activation sites. After the antibody to be tested (i.e., the human BLyS antibody prepared by Embodiment 2) was captured by chip, 7 different gradient of immunogen dilutions (i.e., hBLyS-ECD) prepared by Embodiment 1 were injected and the binding and dissociation of antigen with antibody was detected by a Biacore instrument. The dissociation constant and binding constant were then fitted by Biacore X100 Evaluation Software 2.0, while the affinity constant was the ratio of dissociation constant to binding constant. The results were shown in Table 14, indicating that the BLyS antibody has high affinity with hBLyS-ECD.

**Table 14 Affinity constant of BLyS antibody- hBLyS-ECD**

| Clone No. | Affinity Constant K_{D} (nM) | Binding Constant kₐ (1/Ms) | Dissociation Constant k_{d} (1/s) |
|---|---|---|---|
| 2-1G11 | 0.637 | 1.485×10⁶ | 9.464×10⁻⁴ |
| L9G7 | 0.525 | 4.647×10⁵ | 2.439×10⁻⁴ |
| L1D12 | 2.420 | 5.610×10⁵ | 1.358×10⁻³ |

### Embodiment 4 Detection of purified BLyS antibody blocking the binding of the BLyS protein to its receptor BAFF R

BLyS antibody blocking the binding of the BLyS protein to its receptor BAFF R was detected by the receptor-ligand binding assay of BLyS protein.

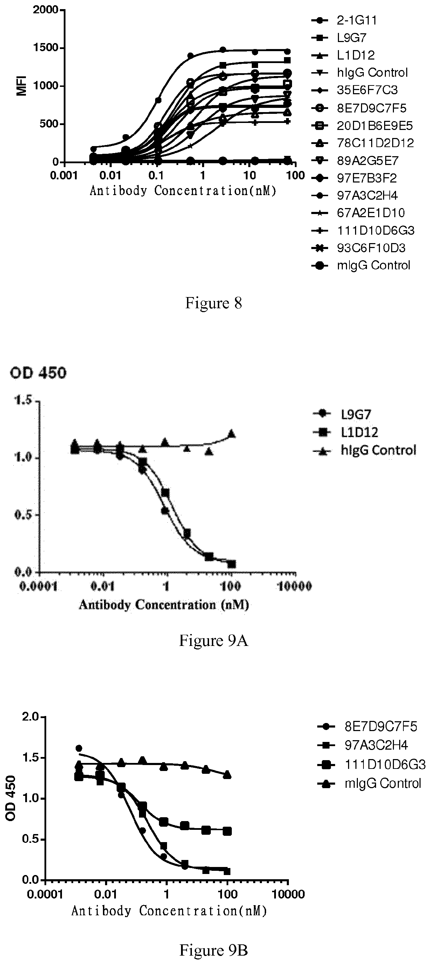

BAFF R (purchased from R & D Systems) was aliquoted into 96-well ELISA plate at 50 ng per well, and the plate was sealed with plastic film and incubated overnight at 4 °C. On the next day the plate was washed four times with the plate washing buffer [PBS buffer containing 0.05% (v/v) Tween20] and added with blocking solution [PBS buffer containing 0.05% (v/v) Tween20 and 1% (w/w) BSA], then blocked for 1 hour at room temperature. The blocking solution was removed and 50 µL per well of the lead antibodies prepared in Embodiment 1 and 2 were added followed by the addition of 50 µL per well of the biotinylated hBLyS-ECD prepared in Embodiment 2. After incubation for 1 hour at 37 °C, the plate was washed four times with the plate washing buffer [PBS buffer containing 0.05% (v/v) Tween20]. 100 µL per well of HRP-labeled (horseradish peroxidase) avidin (purchased from Sigma) was added and incubated at 37 °C for 1 hour. The plate was then washed four times with the plate washing buffer [PBS buffer containing 0.05% (v/v) Tween20]. 100 µL of TMB substrate was aliquoted into each well and after incubation for 5 minutes at room temperature, then 100 µL of stop solution (1.0 N HCl) was aliquoted into each well. A₄₅₀ₙₘ values were read with an ELISA plate reader (SpectraMax 384plus, Molecular Device) and the results were shown in Figures 9A-9B and Table 15. It was shown in Table 15 that the binding of BLyS protein to its receptor BAFF R could be blocked by the BLyS antibody.

**Table 15-1 The binding of BLyS protein to its receptor BAFF R blocked by BLyS antibody**

| OD₄₅₀ₙₘ | Antibody Conc. (nM) | | | | | | | |
|---|---|---|---|---|---|---|---|---|
| Clone No. | 100 | 20 | 4 | 0.8 | 0.16 | 0.032 | 0.0064 | 0.00128 |
| L9G7 | 0.07055 | 0.13375 | 0.32685 | 0.54205 | 0.8991 | 1.0243 | 1.0739 | 1.0749 |
| L1D12 | 0.0755 | 0.14205 | 0.35175 | 0.70175 | 0.97195 | 1.08705 | 1.08145 | 1.0776 |
| hIgG Control | 1.22145 | 1.06255 | 1.08975 | 1.1444 | 1.08315 | 1.11255 | 1.1394 | 1.13265 |

**Table 15-2 The binding of BLyS protein to its receptor BAFF R blocked by BLyS antibody**

| OD₄₅₀ₙₘ | Antibody Conc. (nM) | | | | | | | |
|---|---|---|---|---|---|---|---|---|
| Clone No. | 100 | 20 | 4 | 0.8 | 0.16 | 0.032 | 0.0064 | 0.00128 |
| 8E7D9C7F5 | 0.1098 | 0.1334 | 0.1733 | 0.2944 | 0.6103 | 1.0464 | 1.3822 | 1.6167 |
| 97A3C2H4 | 0.1073 | 0.1246 | 0.2077 | 0.4236 | 0.8155 | 1.1209 | 1.2067 | 1.3356 |
| 111D10D6G3 | 0.6047 | 0.6158 | 0.6699 | 0.7171 | 0.9112 | 1.1410 | 1.2852 | 1.2729 |
| mIgG Control | 1.2984 | 1.3641 | 1.4120 | 1.3967 | 1.4740 | 1.4478 | 1.4018 | 1.4197 |

### Embodiment 5 The effect of human or mouse BLyS protein blocking by purified BLyS antibody on B lymphocyte proliferation detected by mouse B lymphocyte proliferation assay

### (i) B lymphocytes isolated from mice spleen

Freshly harvested mouse spleen was ground, resuspended in RPMI 1640 medium (purchased from Invitrogen, Cat. No.: A10491) containing 10% (w/w) fetal bovine serum and filtered with a 70 µm cell strainer (purchased from BD), and then centrifuged at 1,500 rpm at 4 °C for 5 minutes. The supernatant was removed and the cell pellets were put on vortex for 15 seconds. Red blood cell lysis buffer (purchased from Sigma) was added in and the mixture was allowed to stand at room temperature for 5 minutes. The mixture was replenished to 15 mL with RPMI 1640 medium containing 10% (w/w) fetal bovine serum and centrifuged at 1500 rpm at 4 °C for 5 minutes to obtain the supernatant. The supernatant was filtered with a 40 µm cell strainer (purchased from BD) to obtain a filtered supernatant, and cell counting was followed. Mouse B lymphocytes were isolated using a kit (purchased from Miltenyi Biotec), and the experimental procedures were performed in strict accordance with the instructions of kit. Specific description of the experiment is summarized briefly as follows: The filtered supernatant was centrifuged at 300g at 4 °C for 10 minutes and the supernatant was removed. 40 µL of buffer [PBS buffer containing 0.5% (w/v) BSA and 2 mM EDTA] and 10 µL of biotinylated antibody mixture that binding with non-B lymphocytes from the kit were aliquoted into each of 10⁷ cells, and incubated at 4 °C for 5 minutes after mixing. 30 µL of buffer [PBS buffer containing 0.5% (w/v) BSA and 2 mM EDTA] and 20 µL of avidin-labeled magnetic beads (purchased from Miltenyi Biotec) were further aliquoted into each of 10⁷ cells, and mixed and further incubated at 4 °C for 10 minutes to obtain cells suspension. The volume of above mixture was replenished to 500 µL with buffer. LS Separation Columns (purchased from Miltenyi Biotec) were placed on a magnetic stand, rinsed with 3 mL of buffer and then the cell suspension was loaded onto column, and flow-through solution, which is the suspensions containing unlabeled enriched B lymphocytes, was collected. 3 mL of buffer was further loaded onto the column and the flow-through was collected and poured together with the flow-through solution collected by the previous step, then the B lymphocytes isolated from mouse spleen were obtained.

### (ii) Proliferation assay of mouse B lymphocytes

50 µl of the B lymphocytes obtained in the step (i) of Embodiment 5 were plated in 96-well cell culture plate at 5×10⁴ cells per well, then 2 µg/ml of F(ab')2-goat anti-mouse IgM secondary antibody (purchased from Jackson ImmunoResearch) and 10 ng/ml of the immunogen (hBLyS-ECD) prepared in Embodiment 1 or mouse BLyS protein (purchased from R & D Systems), and different concentrations of the lead antibody prepared in Embodiment 1 or 2 were added, and the volume of each reaction well was adjusted to 100 µL. The reaction plate was incubated at 37 °C in a 5% (v/v) CO₂ incubator for 72 hours and the number of viable cells were detected. The number of viable cells was detected by using CellTiter-Glo® Luminescent Cell Viability Assay Kit (purchased from Promega), and the procedure was performed in strict accordance with the kit's instructions. Specific description of the experiment is briefed as follows: the 96-well plate was equilibrated for 30 minutes at room temperature before detection, an equal volume of CellTiter-Glo® reagent was aliquoted into the cell culture medium, and the mixture was placed in a shaker for 2 minutes to induce cell lysis, then the luminescence signal is stabilized by placing the plate at room temperature for 10 minutes, and the values was read via a plate reader (SpectraMax 384plus, Molecular Device).

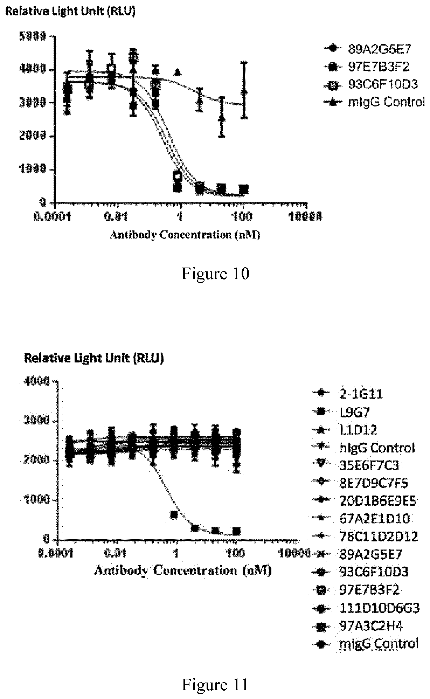

The effect of BLyS antibody on the proliferation of mouse B lymphocyte was detected in the experiment described in step (ii) of Embodiment 5. The results were shown in Figures 10-11 and Tables 16-17, and Figure 10 showed that the antibody to be tested could inhibit mouse B lymphocyte proliferation which was induced by hBLyS-ECD stimulation. It was shown in Figure 11 that there lacked a cross reaction between mouse BLyS and BLyS antibodies to be tested except antibody L9G7, which could inhibit mouse B lymphocyte proliferation inducing by mouse BLyS.

**Table 16 Inhibition of BLyS antibody on mouse B lmphocyte proliferation stimulated by hBLyS-ECD**

| OD₄₅₀ₙₘ | Antibody Concentration (nM) | | | | | | | | | |
|---|---|---|---|---|---|---|---|---|---|---|
| Clone No. | 100 | 20 | 4 | 0.8 | 0.16 | 0.032 | 0.0064 | 0.00128 | 0.000256 | 0 |
| 89A2G5E7 | 413.95 | 433.49 | 431.19 | 530.08 | 3267.88 | 3363.31 | 3697.92 | 3704.82 | 3123.00 | 3440.35 |
| 97E7B3F2 | 377.15 | 382.91 | 364.46 | 438.27 | 2992.95 | 2937.59 | 3773.77 | 3758.77 | 3380.47 | 2956.04 |
| 93C6F10D3 | 416.18 | 466.03 | 513.55 | 798.73 | 3528.81 | 4364.64 | 4049.32 | 3546.19 | 3418.68 | 3028.00 |
| mIgG Control | 3395.07 | 2590.61 | 3103.90 | 3945.19 | 4015.39 | 4014.24 | 3696.60 | 3878.43 | 3134.97 | 2733.32 |

**Table 17 Inhibition of BLyS antibody on mouse B lmphocyte proliferation stimulated by mBLyS**

| OD₄₅₀ₙₘ | Antibody Concentration (nM) | | | | | | | | | |
|---|---|---|---|---|---|---|---|---|---|---|
| Clone No. | 100 | 20 | 4 | 0.8 | 0.16 | 0.032 | 0.0064 | 0.00128 | 0.000256 | 0 |
| 2-1G11 | 2491.49 | 2681.59 | 2461.12 | 2824.44 | 2747.95 | 2473.49 | 2610.72 | 2556.73 | 2453.25 | 2318.27 |
| L9G7 | 220.90 | 243.44 | 307.69 | 640.17 | 2263.13 | 2342.03 | 2461.50 | 2475.02 | 2429.94 | 2104.22 |
| L1D12 | 2454.73 | 2463.75 | 2557.30 | 2409.65 | 2515.59 | 2461.50 | 2504.32 | 2609.14 | 2446.84 | 2343.15 |
| hIgG Control | 2666.96 | 2601.72 | 2584.85 | 2511.74 | 2437.50 | 2501.61 | 2464.49 | 2244.03 | 2224.91 | 2224.91 |
| 35E6F7C3 | 2485.27 | 2563.79 | 2522.83 | 2525.10 | 2406.76 | 2431.79 | 2520.55 | 2339.62 | 2163.23 | 2157.54 |
| 8E7D9C7F5 | 2359.58 | 2347.02 | 2444.10 | 2301.34 | 2352.73 | 2316.18 | 2245.37 | 2244.23 | 2222.53 | 2100.33 |
| 20D1B6E9E5 | 1919.32 | 2239.20 | 2199.22 | 2483.69 | 2116.96 | 2378.58 | 2183.22 | 2174.09 | 2090.69 | 2259.77 |
| 67A2E1D10 | 2067.84 | 2240.35 | 2407.15 | 2489.40 | 2331.74 | 2263.20 | 2111.25 | 2195.79 | 2392.29 | 2121.53 |
| 78C11D2D12 | 2479.25 | 2468.98 | 2338.85 | 2507.78 | 2409.62 | 2389.07 | 2244.11 | 2384.51 | 2205.30 | 1991.85 |
| 89A2G5E7 | 2478.80 | 2636.99 | 2481.07 | 2644.96 | 2368.40 | 2393.44 | 2166.96 | 2272.80 | 2166.96 | 2087.29 |
| 93C6F10D3 | 2742.84 | 2617.65 | 2638.13 | 2527.74 | 2542.53 | 2544.81 | 2509.53 | 2293.29 | 2313.77 | 2311.50 |
| 97E7B3F2 | 2307.96 | 2289.69 | 2413.03 | 2307.96 | 2360.50 | 2575.19 | 2311.39 | 2152.65 | 2197.19 | 2129.81 |
| 111D10D6G3 | 2355.34 | 2538.19 | 2714.19 | 2518.77 | 2290.20 | 2318.77 | 2130.21 | 2151.92 | 2303.92 | 2115.35 |
| 97A3C2H4 | 2322.00 | 2396.38 | 2434.15 | 2414.69 | 2299.11 | 2367.77 | 2251.04 | 2199.55 | 2206.41 | 2162.93 |
| mIgG Control | 2354.26 | 2361.11 | 2437.57 | 2520.88 | 2326.87 | 2320.03 | 2415.89 | 2272.10 | 2112.33 | 2192.21 |

### Embodiment 6 Identification of BLyS antibody binding epitopes

The purified human BLyS antibody and hybridoma antibody were subjected to competitive enzyme-linked immunosorbent assay and the epitopes binding by different antibodies were analyzed.

Purified BLyS antibody was first diluted to a final concentration of 1.0 µg/mL with PBS, then aliquoted into a 96-well ELISA plate at 50 µL per well, and the plate was subsequently sealed with plastic film and incubated at 4 °C overnight. On the next day the plate was rinsed four times with the plate washing buffer [PBS buffer containing 0.05% (v/v) Tween20] and mixed with blocking solution [PBS buffer containing 0.05% (v/v) Tween20 and 1% (w/w) BSA], and then blocked at 37 °C for 1 hour. The blocking solution was removed, and the competitive antibody and IgG control were diluted to a final concentration of 40 µg/mL with PBS and then aliquoted into the 96-well ELISA plate at 50 µL per well. The biotinylated hBLyS-ECD was then diluted with PBS to a final concentration of 2 ng/mL and pipetted into the 96-well ELISA plate at 50 µL per well. The final concentration of competitive antibody and hBLyS-ECD was 20 µg/mL and 1 ng/mL, respectively. After incubation at 37 °C for 1 hour, the plate was rinsed four times with the plate washing buffer [PBS buffer containing 0.05% (v/v) Tween20]. 100 µL per well of HRP-labeled (horseradish peroxidase) streptavidin (purchased from Sigma) was added into the plate and the plate was incubated at 37 °C for 1 hour. The plate was then washed four times with the plate washing buffer [PBS buffer containing 0.05% (v/v) Tween20]. 100 µL of TMB substrate was aliquoted into each well and after incubation at room temperature for 5 minutes, 100 µL of stop solution (1.0 N HCl) was aliquoted into each well. A₄₅₀ₙₘ values were read by an ELISA plate reader (SpectraMax M5e, purchased from Molecular Device) and the results were shown in Tables 18A-18B. It was shown in the tables that the BLyS antibodies were competitive to each other in various degrees, indicating that the binding of antibodies to different epitopes. The data shown in the table is the inhibitory rate (%) of the binding level of the original antibody to hBLyS-ECD after addition of the competitive antibody.

**Table 18A BLyS human antibody compete to bind with hBLyS-ECD**

| Clone No. | 2-1G11 | L1D12 | L9G7 |
|---|---|---|---|
| 2-1G11 | 82 | 91 | 79 |
| L1D12 | 94 | 94 | 90 |
| L9G7 | 96 | 97 | 95 |

**Table 18B Purified BLyS antibody compete to bind with hBLyS-ECD**

| Clone No. | 8E7D9C7F5 | 93C6F10D3 | 97A3C2H4 | 67A2E1D10 | 78C11D2D12 | 111D10D6G3 | 35E6F7C3 |
|---|---|---|---|---|---|---|---|
| 8E7D9C7F5 | 92 | 85 | 89 | 96 | 94 | 96 | 40 |
| 93C6F10D3 | 94 | 79 | 90 | 97 | 92 | 95 | 42 |
| 97A3C2H4 | 93 | 93 | 89 | 96 | 96 | 93 | 55 |
| 67A2E1D10 | 63 | 44 | 75 | 93 | 80 | 86 | 18 |
| 78C11D2D12 | 37 | 58 | 46 | 95 | 88 | 95 | 29 |
| 111D10D6G3 | 27 | 41 | 26 | 96 | 86 | 95 | 22 |
| 35E6F7C3 | 27 | 33 | 26 | 33 | 36 | 38 | 91 |

### Embodiment 7 Sequencing of light chain and heavy chain variable region and preparation of mouse-human chimeric antibody

Total RNA isolation: after the culture supernatant of subclones corresponding to the lead antibodies selected by Embodiment 1 were tested for antigen binding (i.e., after completing the detection and activity determination of Embodiments 3-5), 5×10⁷ hybridoma cells were collected by centrifugation, mixed with 1mL of Trizol and transferred to 1.5mL centrifuge tube, and allowed to stand at room temperature for 5 minutes; subsequently 0.2mL of chloroform was added and oscillated for 15 seconds, the tubes were centrifuged at 12000g and 4 °C for 5 minutes after standing for 2 minutes, and supernatant was transferred to a new 1.5mL of centrifuge tube; 0.5mL of isopropanol was added, the liquid in the tube was mixed gently and centrifuged at 12000g and 4 °C for 15 minutes after standing at room temperature for 10 minutes, and the supernatant was removed; then 1mL of 75% (v/v) ethanol was added in, the pellets were rinsed gently and centrifuged at 12000g and 4 °C for 5 minutes, the supernatant was removed, the pellets were air dried and dissolved in DEPC-treated H₂O (placed in a 55 °C of water bath for 10 minutes to promote dissolution), then the total RNA was obtained.

Reverse transcription and PCR: 1µg of total RNA was took and the reverse transcriptase was added, a 20µL system was set up and reacted at 42 °C for 60 minutes, the reaction was terminated at 85 °C for 10 minutes. A 50 µL PCR system was set with 1 µL of cDNA, 25 pmol of each primer, 1 µL of DNA polymerase, 250 µmol of dNTPs and a compatible buffer system. PCR program consisted of pre-denaturing at 95 °C for 3 minutes, 35 cycles of denaturing at 95 °C for 30 seconds, annealing at 55 °C for 30 seconds and extending at 72 °C for 35 seconds, followed by extension at 72 °C for 5 minutes to obtain a PCR product. The kit used for reverse transcription was PrimeScript RT Master Mix purchased from Takara, with Cat. No. RR036; the kit used for PCR including the Q5 high-fidelity enzyme was purchased from NEB, with Cat. No. M0492.

Cloning and sequencing: 5 µL of PCR product was used to test by agarose gel electrophoresis, and positive samples were purified by column recovery kit NucleoSpin® Gel & PCR Clean-up, purchased from MACHEREY-NAGEL with a Cat. No. of 740609. Ligation was carried out by using 50 ng of sample, 50 ng of T vector, 0.5 µL of ligase, 1 µL of buffer being brought to a final reaction system volume of 10µL, and reacted at 16 °C for half an hour to obtain the ligated product, wherein the ligation kit is T4 DNA ligase purchased from NEB with a Cat. No. of M0402; 5 µL of the ligation product was pipetted into 100 µL of competent cells (Ecos 101competent cells, purchased from Yeastern, Cat. No. FYE607) and ice-cooled for 5 minutes. Subsequently the competent cells were heat shocked at 42 °C for 1 minute in water bath and placed on ice for 1 minutes, then 650 µL of antibiotic-free SOC medium was added and the competent cells were resuscitated at 200 RPM on a shaker at 37 °C for 30 minutes, 200 µL of cells suspension was pipetted and spread on LB solid medium containing antibiotic and cultured overnight in 37 °C incubator. On the next day, colony PCR was performed in a 30 µL PCR system using primers M13F and M13R specifically designed for T vector, bacterial colonies were picked by a tip and pipetted into the PCR system and mixed, and 0.5 µL of suspension was picked onto another LB solid plate containing 100 µg/mL ampicillin to preserve the bacterial strain; 5 µL of product was subjected to the detection of agarose gel electrophoresis when the PCR reaction is completed, and the positive samples were sequenced and analyzed [see Kabat, "Sequences of Proteins of Immunological Interest," National Institutes of Health, Bethesda, Md. (1991)]. The sequencing results were shown in Tables 19-20.

Cloning and sequencing of the human BLyS antibodies prepared by phage display of the present invention could be found in section (iii) of Embodiment 2, and these sequencing results were also included in Tables 19-20.

**Table 19 BLyS Antibody Amino Acid SEQ ID NO.**

| Clone No. | Heavy Chain Protein | | | | Light Chain Protein | | | |
|---|---|---|---|---|---|---|---|---|
| | Variable Region | CDR1 | CDR2 | CDR3 | Variable Region | CDR1 | CDR2 | CDR3 |
| 2-1G11 | 1 | 2 | 3 | 4 | 5 | 6 | 7 | 8 |
| L9G7 | 9 | 10 | 11 | 12 | 13 | 14 | 15 | 16 |
| L1D12 | 17 | 18 | 19 | 20 | 21 | 22 | 23 | 24 |
| 35E6F7C3 | 25 | 26 | 27 | 28 | 29 | 30 | 31 | 32 |
| 8E7D9C7F5 | 33 | 34 | 35 | 36 | 37 | 38 | 39 | 40 |
| 20D1B6E9E5 | 41 | 42 | 43 | 44 | 45 | 46 | 47 | 48 |
| 78C11D2D12 | 49 | 50 | 51 | 52 | 53 | 54 | 55 | 56 |
| 89A2G5E7 | 57 | 58 | 59 | 60 | 61 | 62 | 63 | 64 |
| 97E7B3F2 | 65 | 66 | 67 | 68 | 69 | 70 | 71 | 72 |
| 97A3C2H4 | 73 | 74 | 75 | 76 | 77 | 78 | 79 | 80 |
| 67A2E1D10 | 81 | 82 | 83 | 84 | 85 | 86 | 87 | 88 |
| 111D10D6G3 | 89 | 90 | 91 | 92 | 93 | 94 | 95 | 96 |
| 93C6F10D3 | 97 | 98 | 99 | 100 | 101 | 102 | 103 | 104 |

As used herein, the numbers in Table 19 are the sequence numbers in the sequence listing, for example, the amino acid sequence of heavy chain variable region of 2-1G11 is shown in SEQ ID No. 1, and the amino acid sequence of heavy chain CDR1 of 2-1G11 is shown in SEQ ID No. 2.

**Table 20 Nucleotide SEQ ID NO. of BLyS Antibody Gene**

| Clone No. | Heavy Chain variable region | Light Chain variable region |
|---|---|---|
| 2-1G11 | 105 | 106 |
| L9G7 | 107 | 108 |
| L1D12 | 109 | 110 |
| 35E6F7C3 | 111 | 112 |
| 8E7D9C7F5 | 113 | 114 |
| 20D1B6E9E5 | 115 | 116 |
| 78C11D2D12 | 117 | 118 |
| 89A2G5E7 | 119 | 120 |
| 97E7B3F2 | 121 | 122 |
| 97A3C2H4 | 123 | 124 |
| 67A2E1D10 | 125 | 126 |
| 111D10D6G3 | 127 | 128 |
| 93C6F10D3 | 129 | 130 |

As used herein, the numbers in Table 20 are the sequence numbers in the sequence listing, for example, the nucleotide sequence of the heavy chain variable region of 2-1G11 is shown in SEQ ID No. 105, and the nucleotide sequence of the light chain variable region of 2-1G11 is shown in SEQ ID No.106.

The nucleotide sequence encoding the heavy chain CDR1 of 2-1G11 is the sequence from 76th to 105th base shown in SEQ ID No.105 of the Sequence Listing.

The nucleotide sequence encoding the heavy chain CDR2 of 2-1G11 is the sequence from 148th to 198th base shown in SEQ ID No.105 of the Sequence Listing.

The nucleotide sequence encoding the heavy chain CDR3 of 2-1G11 is the sequence from 295th to 342nd base shown in SEQ ID No.105 of the Sequence Listing.

The nucleotide sequence encoding the light chain CDR1 of 2-1G11 is the sequence from 70th to 102nd base shown in SEQ ID No.106 of the Sequence Listing.

The nucleotide sequence encoding the light chain CDR2 of 2-1G11 is the sequence from 148th to 168th base shown in SEQ ID No.106 of the Sequence Listing.

The nucleotide sequence encoding the light chain CDR3 of 2-1G11 is the sequence from 265th to 291th base shown in SEQ ID No.106 of the Sequence Listing.

The nucleotide sequence encoding the heavy chain CDR1 of L9G7 is the sequence from 76th to 105th base shown in SEQ ID No.107 of the Sequence Listing.

The nucleotide sequence encoding the heavy chain CDR2 of L9G7 is the sequence from 148th to 198th base shown in SEQ ID No.107 of the Sequence Listing.

The nucleotide sequence encoding the heavy chain CDR3 of L9G7 is the sequence from 295th to 342nd base shown in SEQ ID No.107 of the Sequence Listing.

The nucleotide sequence encoding the light chain CDR1 of L9G7 is the sequence from 70th to 102nd base shown in SEQ ID No.108 of the Sequence Listing.

The nucleotide sequence encoding the light chain CDR2 of L9G7 is the sequence from 148th to 168th base shown in SEQ ID No.108 of the Sequence Listing.

The nucleotide sequence encoding the light chain CDR3 of L9G7 is the sequence from 265th to 291nd base shown in SEQ ID No.108 of the Sequence Listing.

The nucleotide sequence encoding the heavy chain CDR1 of L1D12 is the sequence from 76th to 105th base shown in SEQ ID No.109 of the Sequence Listing.

The nucleotide sequence encoding the heavy chain CDR2 of L1D12 is the sequence from 148th to 195th base shown in SEQ ID No.109 of the Sequence Listing.

The nucleotide sequence encoding the heavy chain CDR3 of L1D12 is the sequence from 292nd to 330nd base shown in SEQ ID No.109 of the Sequence Listing.

The nucleotide sequence encoding the light chain CDR1 of L1D12 is the sequence from 67th to 99th base shown in SEQ ID No.110 of the Sequence Listing.

The nucleotide sequence encoding the light chain CDR2 of L1D12 is the sequence from 145th to 165th base shown in SEQ ID No.110 of the Sequence Listing.

The nucleotide sequence encoding the light chain CDR3 of L1D12 is the sequence from 262nd to 297th base shown in SEQ ID No.110 of the Sequence Listing.

The nucleotide sequence encoding the heavy chain CDR1 of 35E6F7C3 is the sequence from 76th to 105th base shown in SEQ ID No.111 of the Sequence Listing.

The nucleotide sequence encoding the heavy chain CDR2 of 35E6F7C3 is the sequence from 148th to 198th base shown in SEQ ID No.111.

The nucleotide sequence encoding the heavy chain CDR3 of 35E6F7C3 is the sequence from 295nd to 321th base shown in SEQ ID No.111 of the Sequence Listing.

The nucleotide sequence encoding the light chain CDR1 of 35E6F7C3 is the sequence from 70th to 99th base shown in SEQ ID No.112 of the Sequence Listing .

The nucleotide sequence encoding the light chain CDR2 of 35E6F7C3 is the sequence from 145th to 165th base shown in SEQ ID No.112 of the Sequence Listing.

The nucleotide sequence encoding the light chain CDR3 of 35E6F7C3 is the sequence from 262nd to 288th base shown in SEQ ID No.112 of the Sequence Listing.

The nucleotide sequence encoding the heavy chain CDR1 of 8E7D9C7F5 is the sequence from 76th to 105th base shown in SEQ ID No.113 of the Sequence Listing.

The nucleotide sequence encoding the heavy chain CDR2 of 8E7D9C7F5 is the sequence from 148th to 198th base shown in SEQ ID No.113 of the Sequence Listing.

The nucleotide sequence encoding the heavy chain CDR3 of 8E7D9C7F5 is the sequence from 295nd to 342nd base shown in SEQ ID No.113 of the Sequence Listing.

The nucleotide sequence encoding the light chain CDR1 of 8E7D9C7F5 is the sequence from 70th to 114th base shown in SEQ ID No.114 of the Sequence Listing.

The nucleotide sequence encoding the light chain CDR2 of 8E7D9C7F5 is the sequence from 160th to 180th base shown in SEQ ID No.114 of the Sequence Listing.

The nucleotide sequence encoding the light chain CDR3 of 8E7D9C7F5 is the sequence from 277nd to 303th base shown in SEQ ID No.114 of the Sequence Listing.

The nucleotide sequence encoding the heavy chain CDR1 of 20D1B6E9E5 is the sequence from 76th to 105th base shown in SEQ ID No.115 of the Sequence Listing.

The nucleotide sequence encoding the heavy chain CDR2 of 20D1B6E9E5 is the sequence from 148th to 198th base shown in SEQ ID No.115 of the Sequence Listing.

The nucleotide sequence encoding the heavy chain CDR3 of 20D1B6E9E5 is the sequence from 295nd to 339th base shown in SEQ ID No.115 of the Sequence Listing.

The nucleotide sequence encoding the light chain CDR1 of 20D1B6E9E5 is the sequence from 70th to 117th base shown in SEQ ID No.116 of the Sequence Listing.

The nucleotide sequence encoding the light chain CDR2 of 20D1B6E9E5 is the sequence from 163th to 183th base shown in SEQ ID No.116 of the Sequence Listing.

The nucleotide sequence encoding the light chain CDR3 of 20D1B6E9E5 is the sequence from 280nd to 306th base shown in SEQ ID No.116 of the Sequence Listing.

The nucleotide sequence encoding the heavy chain CDR1 of 78C11D2D12 is the sequence from 76th to 105th base shown in SEQ ID No.117 of the Sequence Listing.

The nucleotide sequence encoding the heavy chain CDR2 of 78C11D2D12 is the sequence from 148th to 198th base shown in SEQ ID No.117 of the Sequence Listing.

The nucleotide sequence encoding the heavy chain CDR3 of 78C11D2D12 is the sequence from 295nd to 315th base shown in SEQ ID No.117 of the Sequence Listing.

The nucleotide sequence encoding the light chain CDR1 of 78C11D2D12 is the sequence from 70th to 99th base shown in SEQ ID No.118 of the Sequence Listing.

The nucleotide sequence encoding the light chain CDR2 of 78C11D2D12 is the sequence from 145th to 165th base shown in SEQ ID No.118 of the Sequence Listing.

The nucleotide sequence encoding the light chain CDR3 of 78C11D2D12 is the sequence from 262nd to 288th base shown in SEQ ID No.118 of the Sequence Listing.

The nucleotide sequence encoding the heavy chain CDR1 of 89A2G5E7 is the sequence from 76th to 105th base shown in SEQ ID No.119 of the Sequence Listing.

The nucleotide sequence encoding the heavy chain CDR2 of 89A2G5E7 is the sequence from 148th to 195th base shown in SEQ ID No.119 of the Sequence Listing.

The nucleotide sequence encoding the heavy chain CDR3 of 89A2G5E7 is the sequence from 292nd to 327th base shown in SEQ ID No.119 of the Sequence Listing.

The nucleotide sequence encoding the light chain CDR1 of 89A2G5E7 is the sequence from 70th to 105th base shown in SEQ ID No.120 of the Sequence Listing.

The nucleotide sequence encoding the light chain CDR2 of 89A2G5E7 is the sequence from 151th to 171th base shown in SEQ ID No.120 of the Sequence Listing.

The nucleotide sequence encoding the light chain CDR3 of 89A2G5E7 is the sequence from 268nd to 294th base shown in SEQ ID No.120 of the Sequence Listing.

The nucleotide sequence encoding the heavy chain CDR1 of 97E7B3F2 is the sequence from 76th to 105th base shown in SEQ ID No.121 of the Sequence Listing.

The nucleotide sequence encoding the heavy chain CDR2 of 97E7B3F2 is the sequence from 148th to 198th base shown in SEQ ID No.121 of the Sequence Listing.

The nucleotide sequence encoding the heavy chain CDR3 of 97E7B3F2 is the sequence from 295nd to 321th base shown in SEQ ID No.121 of the Sequence Listing.

The nucleotide sequence encoding the light chain CDR1 of 97E7B3F2 is the sequence from 70th to 117th base shown in SEQ ID No.122 of the Sequence Listing.

The nucleotide sequence encoding the light chain CDR2 of 97E7B3F2 is the sequence from 163th to 183th base shown in SEQ ID No.122 of the Sequence Listing.

The nucleotide sequence encoding the light chain CDR3 of 97E7B3F2 is the sequence from 280nd to 306th base shown in SEQ ID No.122 of the Sequence Listing.

The nucleotide sequence encoding the heavy chain CDR1 of 97A3C2H4 is the sequence from 76th to 105th base shown in SEQ ID No.123 of the Sequence Listing.

The nucleotide sequence encoding the heavy chain CDR2 of 97A3C2H4 is the sequence from 148th to 198th base shown in SEQ ID No.123 of the Sequence Listing.

The nucleotide sequence encoding the heavy chain CDR3 of 97A3C2H4 is the sequence from 295nd to 327th base shown in SEQ ID No.123 of the Sequence Listing.

The nucleotide sequence encoding the light chain CDR1 of 97A3C2H4 is the sequence from 70th to 102th base shown in SEQ ID No.124 of the Sequence Listing.

The nucleotide sequence encoding the light chain CDR2 of 97A3C2H4 is the sequence from 148th to 168th base shown in SEQ ID No.124 of the Sequence Listing.

The nucleotide sequence encoding the light chain CDR3 of 97A3C2H4 is the sequence from 265nd to 291th base shown in SEQ ID No.124 of the Sequence Listing.

The nucleotide sequence encoding the heavy chain CDR1 of 67A2E1D10 is the sequence from 76th to 105th base shown in SEQ ID No.125 of the Sequence Listing.

The nucleotide sequence encoding the heavy chain CDR2 of 67A2E1D10 is the sequence from 148th to 198th base shown in SEQ ID No.125 of the Sequence Listing.

The nucleotide sequence encoding the heavy chain CDR3 of 67A2E1D10 is the sequence from 295nd to 333th base shown in SEQ ID No.125 of the Sequence Listing.

The nucleotide sequence encoding the light chain CDR1 of 67A2E1D10 is the sequence from 70th to 102nd base shown in SEQ ID No.126 of the Sequence Listing.

The nucleotide sequence encoding the light chain CDR2 of 67A2E1D10 is the sequence from 148th to 168th base shown in SEQ ID No.126 of the Sequence Listing.

The nucleotide sequence encoding the light chain CDR3 of 67A2E1D10 is the sequence from 265nd to 291th base shown in SEQ ID No.126 of the Sequence Listing.

The nucleotide sequence encoding the heavy chain CDR1 of 111D10D6G3 is the sequence from 76th to 105th base shown in SEQ ID No.127 of the Sequence Listing.

The nucleotide sequence encoding the heavy chain CDR2 of 111D10D6G3 is the sequence from 148th to 198th base shown in SEQ ID No.127 of the Sequence Listing.

The nucleotide sequence encoding the heavy chain CDR3 of 111D10D6G3 is the sequence from 295nd to 309th base shown in SEQ ID No.127 of the Sequence Listing.

The nucleotide sequence encoding the light chain CDR1 of 111D10D6G3 is the sequence from 70th to 102th base shown in SEQ ID No.128 of the Sequence Listing.

The nucleotide sequence encoding the light chain CDR2 of 111D10D6G3 is the sequence from 148th to 168th base shown in SEQ ID No.128 of the Sequence Listing.

The nucleotide sequence encoding the light chain CDR3 of 111D10D6G3 is the sequence from 265nd to 291th base shown in SEQ ID No.128 of the Sequence Listing.

The nucleotide sequence encoding the heavy chain CDR1 of 93C6F10D3 is the sequence from 76th to 105th base shown in SEQ ID No.129 of the Sequence Listing.

The nucleotide sequence encoding the heavy chain CDR2 of 93C6F10D3 is the sequence from 148th to 198th base shown in SEQ ID No.129 of the Sequence Listing.

The nucleotide sequence encoding the heavy chain CDR3 of 93C6F10D3 is the sequence from 295nd to 336th base shown in SEQ ID No.129 of the Sequence Listing.

The nucleotide sequence encoding the light chain CDR1 of 93C6F10D3 is the sequence from 70th to 102th base shown in SEQ ID No.130 of the Sequence Listing.

The nucleotide sequence encoding the light chain CDR2 of 93C6F10D3 is the sequence from 148th to 168th base shown in SEQ ID No.130 of the Sequence Listing.

The nucleotide sequence encoding the light chain CDR3 of 93C6F10D3 is the sequence from 265nd to 291th base shown in SEQ ID No.130 of the Sequence Listing.
Preparation of mouse-human chimeric BLyS antibody: According to the sequencing results of above steps, the antibody heavy chain variable region and light chain variable region sequences were obtained. Production and preparation of mouse-human chimeric BLyS antibody referred to step (iii) of Embodiment 2, including: 1. the preparation of the recombinant vector: the heavy chain variable region was directionally cloned into the expression vector containing the signal peptide and the heavy chain IgG1 constant region of human antibody (wherein the expression vector was purchased from Invitrogen and the recombination process was performed by Shanghai ChemPartner Co., Ltd.), the light chain variable region was directionally cloned into the expression vector containing the signal peptide and the light chain kappa constant region of human antibody (wherein the expression vector was purchased from Invitrogen and the recombination process was performed by Shanghai ChemPartner Co., Ltd.); 2, cell transfection; 3, antibody purification. The harvested BLyS chimeric antibody was characterized (methods referred to Embodiments 3-5). Each harvested chimeric antibody was named with the initial "c" before the clone number of corresponding lead antibody, for example, the chimeric antibody c8E7D9C7F5 corresponds to the lead antibody 8E7D9C7F5.

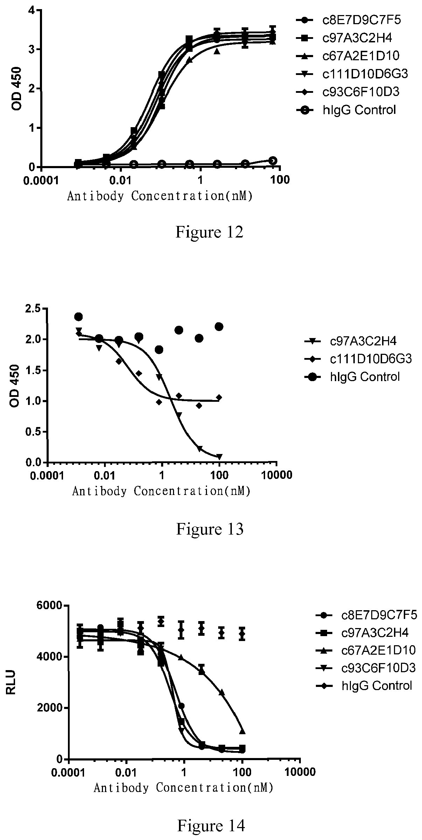

The results were shown in Figure 12 and Table 21, and it was shown in Table 21 that the chimeric antibody could bind to BLyS recombinant protein in ELISA assay. Data shown in the table is the OD value at 450 nm.

**Table 21 ELISA detection of the binding of BLyS chimeric antibody to biotinylated hBLyS-ECD**

| OD₄₅₀ₙₘ | Antibody Cone. (nM) | | | | | | | |
|---|---|---|---|---|---|---|---|---|
| Clone No. | 66.7 | 13.3 | 2.67 | 0.534 | 0.107 | 0.0213 | 0.00427 | 0 |
| c8E7D9C7F5 | 3.3060 | 3.3214 | 3.2359 | 3.0718 | 1.6993 | 0.5743 | 0.1761 | 0.0918 |
| c97A3C2H4 | 3.3024 | 3.3311 | 3.3218 | 3.2608 | 2.3489 | 0.9358 | 0.2502 | 0.1218 |
| c67A2E1D10 | 3.1888 | 3.2390 | 2.9446 | 2.7309 | 1.5835 | 0.5226 | 0.1428 | 0.0848 |
| c111D10D6G3 | 3.4282 | 3.3866 | 3.4048 | 3.1580 | 2.1245 | 0.7471 | 0.2222 | 0.1048 |
| hIgG Control | 0.1436 | 0.0720 | 0.0652 | 0.0620 | 0.0662 | 0.0699 | 0.0798 | 0.0731 |

The binding and dissociation between antibody and antigen was detected via Biacore. The results were shown in Table 22, indicating that the BLyS chimeric antibody has high affinity for hBLyS-ECD.

**Table 22 Affinity constant of BLyS chimeric antibody-hBLyS-ECD**

| Clone No. | Affinity Constant K_{D} (nM) | Binding Constant kₐ (1/MS) | Dissociation Constant k_{d} (1/s) |
|---|---|---|---|
| c35E6F7C3 | 1.454 | 1.905×10⁵ | 2.770×10⁻⁴ |
| c8E7D9C7F5 | 2.383 | 4.565×10⁵ | 1.088×10⁻³ |
| c20D1B6E9E5 | 0.642 | 3.294×10⁵ | 2.113×10⁻⁴ |
| c89A2G5E7 | 0.0857 | 2.128×10⁵ | 1.823×10⁻⁵ |
| c97E7B3F2 | 0.147 | 3.335×10⁵ | 4.892×10⁻⁵ |
| c97A3C2H4 | 0.446 | 1.647×10⁵ | 7.340×10⁻⁵ |
| c67A2F1D10 | 4.990 | 3.195×10⁴ | 1.594×10⁻⁴ |
| c111D10D6G3 | 0.297 | 1.004×10⁵ | 2.984×10⁻⁵ |
| c93C6F10D3 | 0.386 | 5.489×10⁵ | 2.118×10⁻⁴ |

The results were shown in Figures 13 and Table 23, and it was shown in Table 23 that the BLyS chimeric antibody could block the binding of BLyS protein to its receptor BAFF R.

**Table 23 The binding of BLyS protein to its receptor BAFF R was blocked by BLyS chimeric antibody**

| OD₄₅₀ₙₘ | Antibody Cone. (nM) | | | | | | | |
|---|---|---|---|---|---|---|---|---|
| Clone No. | 100 | 20 | 4 | 0.8 | 0.16 | 0.032 | 0.0064 | 0 |
| c97A3C2H4 | 0.0799 | 0.2137 | 0.7615 | 1.3773 | 1.9786 | 1.9078 | 1.8547 | 2.1380 |
| c111D10D6G3 | 1.0556 | 0.9201 | 1.0798 | 0.9776 | 1.4512 | 1.6438 | 2.0103 | 2.0924 |
| hIgG Control | 2.2001 | 2.0120 | 2.1457 | 1.8270 | 2.0417 | 1.9870 | 2.0126 | 2.3674 |

The results were shown in Figure 14 and Table 24, and it was indicated in Figure 24 that BLyS chimeric antibody could inhibit mouse B lymphocyte proliferation which was simulated by hBLyS-ECD.

**Table 24 Inhibition of BLyS chimeric antibody on mouse B lmphocyte proliferation stimulated by hBLyS-ECD**

| OD₄₅₀ₙₘ | Antibody Cone. (nM) | | | | | | | | | |
|---|---|---|---|---|---|---|---|---|---|---|
| Clone No. | 100 | 20 | 4 | 0.8 | 0.16 | 0.032 | 0.0064 | 0.00128 | 0.000256 | 0 |
| c8E7D9C7F5 | 345.07 | 367.38 | 488.27 | 2069.27 | 4109.20 | 4616.25 | 5282.92 | 5154.99 | 4971.89 | 4754.75 |
| c97A3C2H4 | 443.52 | 423.67 | 577.74 | 1464.77 | 3739.53 | 4450.32 | 5280.16 | 4874.00 | 5015.22 | 4443.32 |
| c67A2E1D10 | 1103.30 | 2611.53 | 3496.52 | 3974.22 | 3896.76 | 4338.08 | 5169.07 | 4805.22 | 4720.71 | 4055.21 |
| c93C6F10D3 | 389.24 | 433.52 | 526.75 | 1091.97 | 4304.93 | 4521.70 | 4922.59 | 4515.87 | 4602.11 | 4315.42 |
| hIgG Control | 4884.43 | 4925.19 | 5096.35 | 5036.96 | 5366.47 | 5117.30 | 5066.07 | 4691.15 | 4943.82 | 4394.25 |

### Embodiment 8 Detection of in vivo neutralizing activity of BLyS antibody in mouse

Female BALB/c mice (aged 8-9 weeks, purchased from Shanghai LinChang Systems Co., Ltd.) were feed under SPF conditions, and the experiment was started after one week of acclimatization. On day 1 and day 3 mice were injected intravenously at a dose of 3 mg/kg (1 hour before immunogen injection) with human or human-mouse chimeric BLyS monoclonal antibodies prepared in Embodiments 2 and 6 with clone numbers of 2-1G11, L1D12, c8E7D9C7F5, c20D1B6E9E5, c35E6F7C3, c97A3C2H4, c93C6F10D3 and c111D10D6G3. On day 1 to day 4 mice were stimulated daily by subcutaneous injection with the immunogen prepared in Embodiment 1 (hBLyS-ECD) at a dose of 0.3 mg/kg. On day 5 all animals were sacrificed, and the weight of mice spleen, the proportion of B lymphocytes to splenocytes, and concentration of IgA in serum were measured.

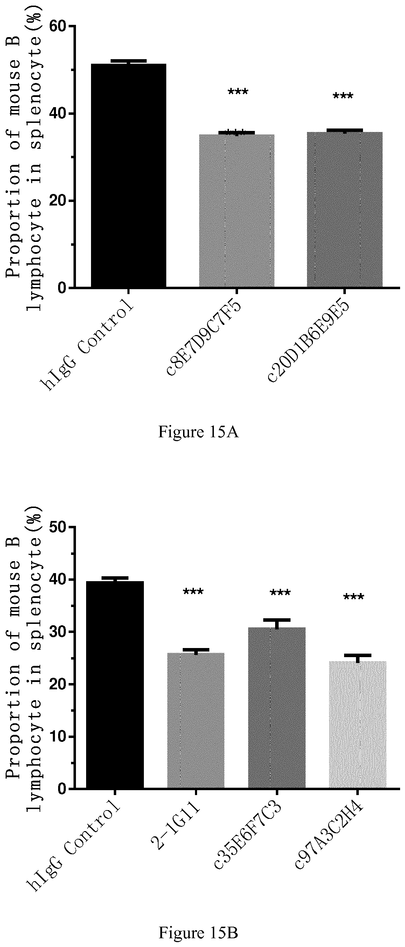

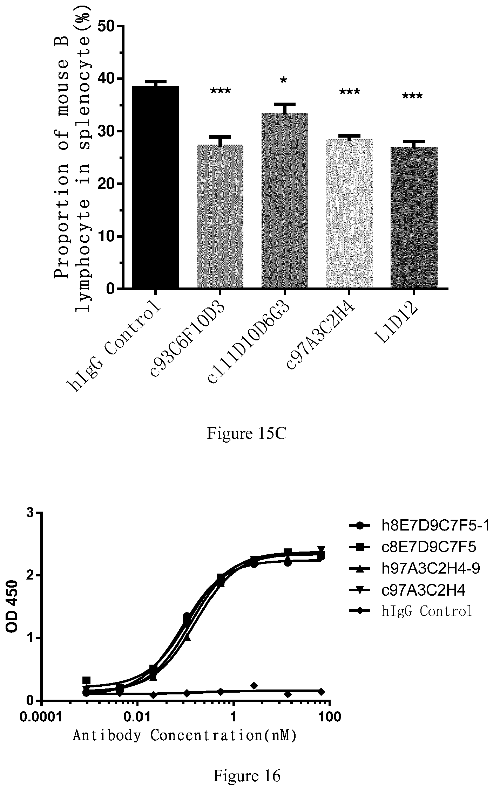

Some results were shown in Figures 15A-15C and Table 25. IgG control herein is human IgG. It was shown in the results that the BLyS antibody could reduce the increase proportion of B lymphocytes to mouse splenocytes which was stimulated by hBLyS-ECD.

**Table 25-1 The effect of BLyS antibody on the proportion of mouse B lymphocytes to splenocytes after hBLyS-ECD stimulation**

| Clone No. | Proportion of B lymphocytes in splenocytes (%) |
|---|---|
| c8E7D9C7F5 | 34.80 |
| c20D1B6E9E5 | 35.36 |
| hIgG Control | 51.05 |

**Table 25-2 The effect of BLyS antibody on the proportion of mouse B lymphocytes to splenocytes after hBLyS-ECD stimulation**

| Clone No. | Proportion of B lymphocytes in splenocytes (%) |
|---|---|
| 2-1G11 | 25.64 |
| c35E6F7C3 | 30.51 |
| c97A3C2H4 | 24.10 |
| hIgG Control | 39.36 |

**Table 25-3 The effect of BLyS antibody on the proportion of mouse B lymphocytes to splenocytes after hBLyS-ECD stimulation**

| Clone No. | Proportion of B lymphocytes in splenocytes (%) |
|---|---|
| L1D12 | 26.75 |
| c93C6F10D3 | 27.11 |
| c111D10D6G3 | 33.24 |
| c97A3C2H4 | 28.19 |
| hIgG Control | 38.29 |

### Embodiment 9 Preparation and identification of humanized BLyS antibody

The templates of human antibody heavy and light chain variable region that best match with the non-CDR regions of the above chimeric antibody c8E7D9C7F5 or c97A3C2H4 were selected from the Germline database. The sequences of the humanized BLyS antibody were selected from the sequences of human V_{H}, J_{H}, V_{K} and J_{K} exons. Among them, the template of the heavy chain variable region of antibody c8E7D9C7F5 is V_{H}1-18 of V_{H} exon and J_{H}-6 of J_{H} exon from a human antibody heavy chain, and the template of the light chain variable region is B3 of V_{K} exon, J_{K}-4 of the J_{K} exon from a human antibody light chain. The template of the heavy chain variable region of antibody c97A3C2H4 is V_{H}3-7 of V_{H} exon and J_{H}-6 of J_{H} exon from a human antibody heavy chain, and the template of the light chain variable region is A10 of V_{K} exon, J_{K}-4 of the J_{K} exon from a human antibody light chain.

The heavy chain CDRs and light chain CDRs of the chimeric antibody c8E7D9C7F5 or c97A3C2H4 determined according to Kabat definition were transplanted into the CDR regions of selected human templates, respectively. Then, a humanized antibody was obtained by reverse mutating buried residues, residues directly interacting with the CDR regions, and framework residues having a significant influence on the conformations of VH and VL based on the three-dimensional structure of mouse antibody.

The amino acid sequences alignment of heavy and light chain variable region of the humanized BLyS antibody variants and the heavy and light chain variable region of the chimeric antibody were shown in Table 26. The sequences of heavy chain variable region of the humanized BLyS antibody h8E7D9C7F5 variants were shown in SEQ ID No. 140, SEQ ID No. 141, SEQ ID No. 142, SEQ ID No. 143, SEQ ID No. 144, SEQ ID No. 144, No.145 and SEQ ID No.146, respectively, and the sequences of light chain variable region were shown in SEQ ID No.147 and SEQ ID No.148, respectively. The sequences of heavy chain variable region of the humanized BLyS antibody h97A3C2H4 variant were shown in SEQ ID No. 149, SEQ ID No. 150, SEQ ID No. 151 and SEQ ID No. 152, respectively, and the sequences of light chain variable region were shown in SEQ ID No.153, SEQ ID No.154, SEQ ID No.155, SEQ ID No.156, SEQ ID No.157, respectively.

Template of human heavy chain variable region V_{H}1-18/J_{H}6 (SEQ ID NO.136)
V_{H}1-18:

QVQLVQSGAEVKKPGASVKVSCKASGYTFTSYGISWVRQAPGQGLEWMGWI
SAYNGNTNYAQKLQGRVTMTTDTSTSTAYMELRSLRSDDTAVYYCAR;

J_{H}6:WGQGTTVTVSS.

Template of human light chain variable region B3/JK4 (SEQ ID NO. 137)
B3:

DIVMTQSPDSLAVSLGERATINCKSSQSVLYSSNNKNYLAWYQQKPGQPPKLL
IYWASTRESGVPDRFSGSGSGTDFTLTISSLQAEDVAVYYC;

J_{K}4:FGGGTKVEIK.

Template of human heavy chain variable region V_{H}3-7/JH6 (SEQ ID NO.138)
V_{H}3-7:

EVQLVESGGGLVQPGGSLRLSCAASGFTFSSYWMSWVRQAPGKGLEWVANI
KQDGSEKYYVDSVKGRFTISRDNAKNSLYLQMNSLRAEDTAVYYCAR;

J_{H}6:WGQGTTVTVSS.

Template of human light chain variable region A10/JK4 (SEQ ID NO.139)
A10:

EIVLTQSPDFQSVTPKEKVTITCRASQSIGSSLHWYQQKPDQSPKLLIKYASQSF
SGVPSRFSGSGSGTDFTLTINSLEAEDAATYYC;

J_{K}4:FGGGTKVEIK.

**Table 26-1 Sequence alignment of variable region of humanized BLyS antibody h8E7D9C7F5 and variable region of chimeric antibody c8E7D9C7F5**

| Antibody | Heavy Chain | V_{H} template of heavy chain variable region of human antibody | Donor framework residues and reverse mutation | SEQ ID NO. | Light Chain | V_{K} template of light chain variable region of human antibody | Donor framework residues and reverse mutation | SEQ ID NO. |
|---|---|---|---|---|---|---|---|---|
| c8E7D9C7F5 | V_{H} | None | CDR | 33 | Vₖ | None | CDR | 37 |
| h8E7D9C7F5-1 | V_{H}.1 | V_{H}1-18/J_{H}6 | CDR-grafted, Q1E | 140 | Vₖ.1 | B3/J_{K}4 | CDR-grafted, Y49K | 147 |
| h8E7D9C7F5-2 | V_{H}.1 | V_{H}1-18/J_{H}6 | CDR-grafted, Q1E | 140 | Vₖ.1a | B3/J_{K}4 | CDR-grafted, M4L, Y49K | 148 |
| h8E7D9C7F5-3 | V_{H}.1a | V_{H}1-18/J_{H}6 | CDR-grafted, Q1E, T71A | 141 | Vₖ.1 | B3/J_{K}4 | CDR-grafted, Y49K | 147 |
| h8E7D9C7F5-4 | V_{H}.1a | V_{H}1-18/J_{H}6 | CDR-grafted, Q1E, T71A | 141 | Vₖ.1a | B3/J_{K}4 | CDR-grafted, M4L,Y49K | 148 |
| h8E7D9C7F5-5 | V_{H}.1b | V_{H}1-18/J_{H}6 | CDR-grafted, Q1E, T71A, M69L | 142 | Vₖ.1 | B3/J_{K}4 | CDR-grafted, Y49K | 147 |
| h8E7D9C7F5-6 | V_{H}.1b | V_{H}1-18/J_{H}6 | CDR-grafted, Q1E, T71A, M69L | 142 | Vₖ.1a | B3/J_{K}4 | CDR-grafted, M4L, Y49K | 148 |
| h8E7D9C7F5-7 | V_{H}.1c | V_{H}1-18/J_{H}6 | CDR-grafted, Q1E, T71A, M69L, M48I | 143 | Vₖ.1 | B3/J_{K}4 | CDR-grafted, Y49K | 147 |
| h8E7D9C7F5-8 | V_{H}.1c | V_{H}1-18/J_{H}6 | CDR-grafted, Q1E, T71A, M69L, M48I | 143 | Vₖ.1a | B3/J_{K}4 | CDR-grafted, M4L, Y49K | 148 |
| h8E7D9C7F5-9 | V_{H}.1d | V_{H}1-18/J_{H}6 | CDR-grafted, Q1E, T71A, M69L, M48I, R38K | 144 | Vₖ.1 | B3/J_{K}4 | CDR-grafted, Y49K | 147 |
| h8E7D9C7F5-10 | V_{H}.1d | V_{H}1-18/J_{H}6 | CDR-grafted, Q1E, T71A, M69L, M48I, R38K | 144 | Vₖ.1a | B3/J_{K}4 | CDR-grafted, M4L, Y49K | 148 |
| h8E7D9C7F5-11 | V_{H}.1e | V_{H}1-18/J_{H}6 | CDR-grafted, Q1E, T71A, M69L, M48I, R38K, R66K, V67A | 145 | Vₖ.1 | B3/J_{K}4 | CDR-grafted, Y49K | 147 |
| h8E7D9C7F5-12 | V_{H}.1e | V_{H}1-18/J_{H}6 | CDR-grafted, Q1E, T71A, M69L, M48I, R38K, R66K, V67A | 145 | Vₖ.1a | B3/J_{K}4 | CDR-grafted, M4L, Y49K | 148 |
| h8E7D9C7F5-13 | V_{H}.1f | V_{H}1-18/J_{H}6 | CDR-grafted, Q1E, T71A, M69L, M48I, R38K, R66K, V67A, G44A, T73K, Y91F | 146 | Vₖ.1 | B3/J_{K}4 | CDR-grafted, Y49K | 147 |
| h8E7D9C7F5-14 | V_{H}.1f | V_{H}1-18/J_{H}6 | CDR-grafted, Q1E, T71A, M69L, M48I, R38K, R66K, V67A, G44A, T73K, Y91F | 146 | Vₖ.1a | B3/J_{K}4 | CDR-grafted, M4L, Y49K | 148 |

**Table 26-2 Sequence alignment of variable region of humanized BLyS antibody h97A3C2H4 and variable region of chimeric antibody c97A3C2H4**

| Antibod y | Heavy Chain | V_{H} template of heavy chain variable region of human antibody | Donor framework residues and reverse mutation | SEQ ID NO. | Light Chain | V_{K} template of light chain variable region of human antibody | Donor framework residues and reverse mutation | SEQ ID NO. |
|---|---|---|---|---|---|---|---|---|
| c97A3C2H4 | V_{H} | None | CDR | 73 | Vₖ | None | CDR | 77 |
| h97A3C2H4-1 | V_{H}.1 | V_{H}3-7/J_{H}6 | CDR-grafted | 149 | Vₖ.1 | A10/J_{K}4 | CDR-grafted | 153 |
| h97A3C2H4-2 | V_{H}.1 | V_{H}3-7/J_{H}6 | CDR-grafted | 149 | Vₖ.1a | A10/J_{K}4 | CDR-grafted, V58T | 154 |
| h97A3C2H4-3 | V_{H}.1a | V_{H}3-7/J_{H}6 | CDR-grafted, A93T | 150 | Vₖ.1 | A10/J_{K}4 | CDR-grafted | 153 |
| h97A3C2H4-4 | V_{H}.1a | V_{H}3-7/J_{H}6 | CDR-grafted, A93T | 150 | V_{k.}1a | A10/J_{K}4 | CDR-grafted, V58T | 154 |
| h97A3C2H4-5 | V_{H}.1b | V_{H}3-7/J_{H}6 | CDR-grafted, A93T, G44R | 151 | Vₖ.1 | A10/J_{K}4 | CDR-grafted | 153 |
| h97A3C2H4-6 | V_{H}.1b | V_{H}3-7/J_{H}6 | CDR-grafted, A93T, G44R | 151 | Vₖ.1a | A10/J_{K}4 | CDR-grafted, V58T | 154 |
| h97A3C2H4-7 | V_{H}.1b | V_{H}3-7/J_{H}6 | CDR-grafted, A93T, G44R | 151 | Vₖ.1b | A10/J_{K}4 | CDR-grafted, V58T, E1D, V3L | 155 |
| h97A3C2H4-8 | V_{H}.1b | V_{H}3-7/J_{H}6 | CDR-grafted, A93T, G44R | 151 | Vₖ.1c | A10/J_{K}4 | CDR-grafted, V58T, P40T | 156 |
| h97A3C2H4-9 | V_{H}.1b | V_{H}3-7/J_{H}6 | CDR-grafted, A93T, G44R | 151 | Vₖ.1d | A10/J_{K}4 | CDR-grafted, V58T, E1D, V3L, P40T | 157 |
| h97A3C2H4-10 | V_{H}.1c | V_{H}3-7/J_{H}6 | CDR-grafted, A93T, G44R, G42E | 152 | Vₖ.1b | A10/J_{K}4 | CDR-grafted, V58T, E1D, V3L | 155 |
| h97A3C2H4-11 | V_{H}.1c | V_{H}3-7/J_{H}6 | CDR-grafted, A93T, G44R, G42E | 152 | Vₖ.1c | A10/J_{K}4 | CDR-grafted, V58T, P40T | 156 |
| h97A3C2H4-12 | V_{H}.1c | V_{H}3-7/J_{H}6 | CDR-grafted, A93T, G44R, G42E | 152 | Vₖ.1d | A10/J_{K}4 | CDR-grafted, V58T, E1D, V3L, P40T | 157 |

The nucleotide sequences of heavy and light chain variable region of the humanized BLyS antibody variants were shown in Table 27. The nucleotide sequences of the heavy chain variable region of the humanized BLyS antibody h8E7D9C7F5 variant were shown in SEQ ID No. 158, SEQ ID No. 159, SEQ ID No. 160, SEQ ID No. 161, SEQ ID No. 162, SEQ ID No.163, SEQ ID No.164, respectively, and the nucleotide sequences of the light chain variable region were shown in SEQ ID No.165 and SEQ ID No.166, respectively. The nucleotide sequences of heavy chain variable region of the humanized BLyS antibody h97A3C2H4 variants were shown in SEQ ID No. 167, SEQ ID No. 168, SEQ ID No. 169, SEQ ID No. 170, respectively, and the nucleotide sequences of light chain variable region were shown in SEQ ID No. 171, SEQ ID No. 172, SEQ ID No. 173, SEQ ID No. 174, SEQ ID No. 175, respectively.

**Table 27-1 Nucleotide SEQ ID NO. of variable region of humanized BLyS antibody h8E7D9C7F5**

| Antibody | Heavy Chain variable region | Light Chain variable region |
|---|---|---|
| h8E7D9C7F5-1 | 158 | 165 |
| h8E7D9C7F5-2 | 158 | 166 |
| h8E7D9C7F5-3 | 159 | 165 |
| h8E7D9C7F5-4 | 159 | 166 |
| h8E7D9C7F5-5 | 160 | 165 |
| h8E7D9C7F5-6 | 160 | 166 |
| h8E7D9C7F5-7 | 161 | 165 |
| h8E7D9C7F5-8 | 161 | 166 |
| h8E7D9C7F5-9 | 162 | 165 |
| h8E7D9C7F5-10 | 162 | 166 |
| h8E7D9C7F5-11 | 163 | 165 |
| h8E7D9C7F5-12 | 163 | 166 |
| h8E7D9C7F5-13 | 164 | 165 |
| h8E7D9C7F5-14 | 164 | 166 |

**Table 27-2 Nucleotide SEQ ID NO. of variable region of humanized BLyS antibody h97A3C2H4**

| Antibody | Heavy Chain variable region | Light Chain variable region |
|---|---|---|
| h97A3C2H4-1 | 167 | 171 |
| h97A3C2H4-2 | 167 | 172 |
| h97A3C2H4-3 | 168 | 171 |
| h97A3C2H4-4 | 168 | 172 |
| h97A3C2H4-5 | 169 | 171 |
| h97A3C2H4-6 | 169 | 172 |
| h97A3C2H4-7 | 169 | 173 |
| h97A3C2H4-8 | 169 | 174 |
| h97A3C2H4-9 | 169 | 175 |
| h97A3C2H4-10 | 170 | 173 |
| h97A3C2H4-11 | 170 | 174 |
| h97A3C2H4-12 | 170 | 175 |

Each domain was assembled using overlap extension PCR using overlapping oligonucleotides synthesized from humanized V_{H} or V_{L} domains. The V_{H} domain was directionally cloned into the expression vector comprising the signal peptide and the heavy chain IgG1 constant region of human antibody using the restriction sites incorportated into the PCR product (wherein the expression vector was purchased from Invitrogen and the recombination process was performed by Shanghai ChemPartner Co., Ltd.), the V_{L} domain was directionally cloned into the expression vector comprising the signal peptide and light chain kappa constant region of human antibody (wherein the expression vector was purchased from Invitrogen and the recombination process was performed by Shanghai ChemPartner Co., Ltd.). The harvested recombinant plasmid was confirmed by sequencing, then high purity of recombinant plasmid was extracted and filtered through a 0.22µm filter membrane for following transfection. Production and preparation of humanized BLyS antibody referred to step (iii) of Embodiment 2, and the harvested BLyS antibody was characterized (see Embodiments 3-5 and 7).

The results were shown in Figure 16 and Table 28, and it was shown in Table 28 that the humanized antibody could bind to BLyS recombinant protein in ELISA assay. Data in the table is the OD value at 450nm.

**Table 28 ELISA detection of the binding of humanized BLyS antibody to biotinylated hBLyS-ECD**

| OD₄₅₀ₙₘ | Antibody Cone. (nM) | | | | | | | |
|---|---|---|---|---|---|---|---|---|
| Clone No. | 66.7 | 13.3 | 2.67 | 0.534 | 0.107 | 0.0213 | 0.00427 | 0 |
| h8E7D9C7F5-1 | 2.2986 | 2.2019 | 2.1808 | 1.8797 | 1.3505 | 0.4991 | 0.1950 | 0.1285 |
| c8E7D9C7F5 | 2.3233 | 2.3700 | 2.2093 | 1.9660 | 1.2801 | 0.5083 | 0.1978 | 0.3222 |
| h97A3C2H4-9 | 2.3711 | 2.2738 | 2.2379 | 1.8774 | 1.0192 | 0.3716 | 0.1794 | 0.1978 |
| c97A3C2H4 | 2.4051 | 2.3430 | 2.2493 | 1.9071 | 1.1888 | 0.3745 | 0.1937 | 0.1653 |
| hIgG Control | 0.1401 | 0.1035 | 0.2374 | 0.1414 | 0.1171 | 0.0887 | 0.1212 | 0.1194 |

The binding and dissociation between antibody and antigen was detected by Biacore. The results were shown in Table 29, indicating that the humanized BLyS antibody has high affinity for hBLyS-ECD.

**Table 29 Affinity constant of humanized BLyS antibody for hBLyS-ECD**

| Clone No. | Affinity Constant K_{D} (nM) | Binding Constant kₐ (1/Ms) | Dissociation Constant k_{d}(1/s) |
|---|---|---|---|
| c8E7D9C7F5 | 2.941 | 2.427×10⁵ | 7.138×10⁻⁴ |
| h8E7D9C7F5-1 | 4.329 | 2.177×10⁵ | 9.423×10⁻⁴ |
| c97A3C2H4 | 1.488 | 1.206×10⁵ | 1.794×10⁻⁴ |
| h97A3C2H4-9 | 1.878 | 1.150×10⁵ | 2.160×10⁻⁴ |

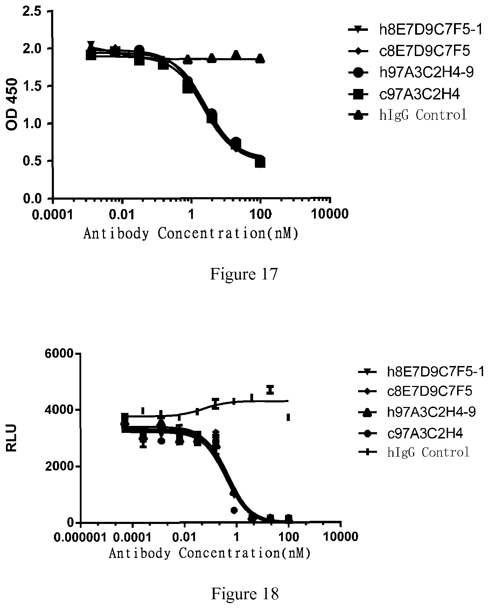

The results were shown in Figures 17 and Table 30. Table 30 showed that the humanized BLyS antibody could block the binding of BLyS protein to its receptor BAFF R. The data in the table is OD₄₅₀ₙₘ value.

**Table 30 The binding of BLyS protein to its receptor BAFF R that blocked by humanized BLyS antibody**

| OD₄₅₀ₙₘ | Antibody Cone. (nM) | | | | | | | |
|---|---|---|---|---|---|---|---|---|
| Clone No. | 100 | 20 | 4 | 0.8 | 0.16 | 0.032 | 0.0064 | 0 |
| h8E7D9C7F5-1 | 0.4501 | 0.7213 | 1.1150 | 1.5421 | 1.7835 | 1.8727 | 1.9808 | 2.0499 |
| c8E7D9C7F5 | 0.5296 | 0.6604 | 1.0354 | 1.5597 | 1.8533 | 1.9748 | 2.0080 | 1.9519 |
| h97 A3C2H4-9 | 0.5060 | 0.7477 | 1.1276 | 1.5564 | 1.8094 | 1.9797 | 1.9601 | 1.9430 |
| c97A3C2H4 | 0.4740 | 0.7166 | 1.0687 | 1.4683 | 1.7871 | 1.8425 | 1.9609 | 1.9144 |
| hIgG Control | 1.8655 | 1.9140 | 1.8696 | 1.8512 | 1.7914 | 1.8435 | 1.9850 | 1.9992 |

The results were shown in Figure 18 and Table 31. It was shown in Table 31 that humanized BLyS antibody could inhibit the proliferation of mouse B lymphocytes that stimulated by hBLyS-ECD.

**Table 31 Inhibition of humanized BLyS antibody on mouse B lmphocyte proliferation stimulated by hBLyS-ECD**

| OD₄₅₀ₙₘ | Antibody Cone. (nM) | | | | | | | | | |
|---|---|---|---|---|---|---|---|---|---|---|
| Clone No. | 100 | 20 | 4 | 0.8 | 0.16 | 0.032 | 0.0064 | 0.00128 | 0.000256 | 0 |
| h8E7D9C7F5-1 | 131.23 | 151.51 | 208.78 | 950.84 | 2726.07 | 2973.03 | 3199.70 | 3120.96 | 2995.69 | 3481.26 |
| c8E7D9C7F5 | 116.00 | 187.02 | 187.02 | 408.36 | 3195.83 | 2949.63 | 3021.83 | 3357.99 | 3305.91 | 3639.69 |
| h97A3C2H4-9 | 126.73 | 137.39 | 216.75 | 1108.61 | 2772.70 | 2964.58 | 2965.76 | 3541.38 | 3084.20 | 3591.13 |
| c97A3C2H4 | 169.37 | 158.71 | 222.67 | 428.76 | 2892.33 | 2964.58 | 3053.41 | 2900.62 | 3107.89 | 3451.37 |
| hIgG Control | 3721.21 | 4701.60 | 4430.12 | 4291.42 | 4201.33 | 3921.55 | 3733.06 | 3861.09 | 3967.79 | 3573.02 |

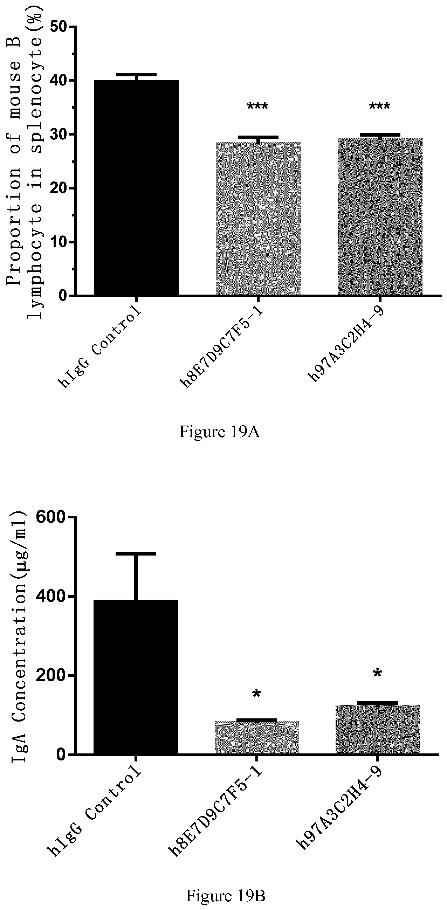

Meanwhile, *in vivo* neutralizing activity of BLyS antibody was detected in mouse. The results were shown in Figures 19A-19B and Table 32. It was shown in the results that humanized BLyS antibody could reduce the increase proportion of B lymphocytes to mouse splenocytes stimulated by hBLyS-ECD and effectively inhibit the level of IgA in serum at the same time.

**Table 32-1 The effect of humanized BLyS antibody on the proportion of mouse B lymphocytes to splenocytes after hBLyS-ECD stimulation**

| Clone No. | Proportion of B lymphocytes in splenocytes (%) |
|---|---|
| h8E7D9C7F5-1 | 28.29 |
| h97A3C2H4-9 | 28.98 |
| hIgG Control | 39.74 |

**Table 32-2 Effect of humanized BLyS antibody on IgA concentration in mouse serum**

| Clone No. | IgA Conc. (µg/mL) |
|---|---|
| h8E7D9C7F5-1 | 80.89 |
| h97A3C2H4-9 | 121.33 |
| hIgG Control | 387.02 |

All references mentioned in the present invention are incorporated herein by reference, as if each reference was individually incorporated by reference. In addition, it is to be understood that after reading the foregoing contents of the present invention, those skilled in the art can make various changes or modifications to the present invention, and these equivalent forms also fall within the scope of the appended claims of the present application.

## Claims

1. An isolated protein, wherein said protein comprises complementary determining regions (CDRs) of BLyS antibody: one or more of the heavy chain CDR1, heavy chain CDR2 and heavy chain CDR3, and/or one or more of the light chain CDR1, light chain CDR2, and light chain CDR3, wherein the heavy chain CDR1 contains amino acid sequences represented by SEQ ID No.2, SEQ ID No. 10, SEQ ID No. 18, SEQ ID No.26, SEQ ID No.34, SEQ ID No.42, SEQ ID No.50, SEQ ID No.58, SEQ ID No.66, SEQ ID No.74, SEQ ID No.82, SEQ ID No.90 or SEQ ID No.98; the heavy chain CDR2 contains amino acid sequences represented by SEQ ID No.3, SEQ ID No.11, SEQ ID No.19, SEQ ID No.27, SEQ ID No.35, SEQ ID No.43, SEQ ID No.51, SEQ ID No.59, SEQ ID No.67, SEQ ID No.75, SEQ ID No.83, SEQ ID No.91 or SEQ ID No.99; the heavy chain CDR3 contains amino acid sequences represented by SEQ ID No.4, SEQ ID No.12, SEQ ID No.20, SEQ ID No.28, SEQ ID No.36, SEQ ID No.44, SEQ ID No.52, SEQ ID No.60, SEQ ID No.68, SEQ ID No.76, SEQ ID No.84, SEQ ID No.92 or SEQ ID No.100; the light chain CDR1 contains amino acid sequences represented by SEQ ID No.6, SEQ ID No. 14, SEQ ID No.22, SEQ ID No.30, SEQ ID No.38, SEQ ID No.46, SEQ ID No.54, SEQ ID No.62, SEQ ID No.70, SEQ ID No.78, SEQ ID No.86, SEQ ID No.94 or SEQ ID No.102; the light chain CDR2 contains amino acid sequences represented by SEQ ID No.7, SEQ ID No. 15, SEQ ID No.23, SEQ ID No.31, SEQ ID No.39, SEQ ID No.47, SEQ ID No.55, SEQ ID No.63, SEQ ID No.71, SEQ ID No.79, SEQ ID No.87, SEQ ID No.95 or SEQ ID No.103; the light chain CDR3 contains amino acid sequences represented by SEQ ID No.8, SEQ ID No. 16, SEQ ID No.24, SEQ ID No.32, SEQ ID No.40, SEQ ID No.48, SEQ ID No.56, SEQ ID No.64, SEQ ID No.72, SEQ ID No.80, SEQ ID No.88, SEQ ID No.96 or SEQ ID No.104;
Or, the amino acid sequences of the heavy chain CDR1 is at least 80% identical to the amino acid sequences represented by SEQ ID No.2, SEQ ID No.10, SEQ ID No. 18, SEQ ID No.26, SEQ ID No.34, SEQ ID No.42, SEQ ID No.50, SEQ ID No.58, SEQ ID No.66, SEQ ID No.74, SEQ ID No.82, SEQ ID No.90 or SEQ ID No.98; the amino acid sequences of the heavy chain CDR2 is at least 80% identical to the amino acid sequences represented by SEQ ID No.3, SEQ ID No.11, SEQ ID No.19, SEQ ID No.27, SEQ ID No.35, SEQ ID No.43, SEQ ID No.51, SEQ ID No.59, SEQ ID No.67, SEQ ID No.75, SEQ ID No.83, SEQ ID No.91 or SEQ ID No.99; the amino acid sequences of the heavy chain CDR3 is at least 80% identical to the amino acid sequences represented by SEQ ID No.4, SEQ ID No.12, SEQ ID No.20, SEQ ID No.28, SEQ ID No.36, SEQ ID No.44, SEQ ID No.52, SEQ ID No.60, SEQ ID No.68, SEQ ID No.76, SEQ ID No.84, SEQ ID No.92 or SEQ ID No.100; the amino acid sequences of the light chain CDR1 is at least 80% identical to the amino acid sequences represented by SEQ ID No.6, SEQ ID No.14, SEQ ID No.22, SEQ ID No.30, SEQ ID No.38, SEQ ID No.46, SEQ ID No.54, SEQ ID No.62, SEQ ID No.70, SEQ ID No.78, SEQ ID No.86, SEQ ID No.94 or SEQ ID No.102; the amino acid sequences of the light chain CDR2 is at least 80% identical to the amino acid sequences represented by SEQ ID No.7, SEQ ID No. 15, SEQ ID No.23, SEQ ID No.31, SEQ ID No.39, SEQ ID No.47, SEQ ID No.55, SEQ ID No.63, SEQ ID No.71, SEQ ID No.79, SEQ ID No.87, SEQ ID No.95 or SEQ ID No.103; the amino acid sequences of the light chain CDR3 is at least 80% identical to the amino acid sequences represented by SEQ ID No.8, SEQ ID No.16, SEQ ID No.24, SEQ ID No.32, SEQ ID No.40, SEQ ID No.48, SEQ ID No.56, SEQ ID No.64, SEQ ID No.72, SEQ ID No.80, SEQ ID No.88, SEQ ID No.96 or SEQ ID No.104.

2. The protein of claim 1, wherein a heavy chain CDR1 comprises amino acid sequence of SEQ ID No.2, a heavy chain CDR2 comprises amino acid sequence of SEQ ID No.3 and a heavy chain CDR3 comprises amino acid sequence of SEQ ID No.4; a heavy chain heavy chain CDR1 comprises amino acid sequence of SEQ ID No. 10, a heavy chain CDR2 comprises amino acid sequence of SEQ ID No. 11 and a CDR3 comprises amino acid sequence of SEQ ID No.12; a heavy chain CDR1 comprises amino acid sequence of SEQ ID No.18, a heavy chain CDR2 comprises amino acid sequence of SEQ ID No.19 and a heavy chain CDR3 comprises amino acid sequence of SEQ ID No.20; a heavy chain CDR1 comprises amino acid sequence of SEQ ID No.26, a heavy chain CDR2 comprises amino acid sequence of SEQ ID No.27 and a heavy chain CDR3 comprises amino acid sequence of SEQ ID No.28; a heavy chain CDR1 comprises amino acid sequence of SEQ ID No.34, a heavy chain CDR2 comprises amino acid sequence of SEQ ID No.35 and a CDR3 comprises amino acid sequence of SEQ ID No.36; a heavy chain CDR1 comprises amino acid sequence of SEQ ID No.42, a heavy chain CDR2 comprises amino acid sequence of SEQ ID No.43 and a heavy chain CDR3 comprises amino acid sequence of SEQ ID No.44; a heavy chain CDR1 comprises amino acid sequence of SEQ ID No.50, a heavy chain CDR2 comprises amino acid sequence of SEQ ID No.51 and a heavy chain CDR3 comprises amino acid sequence of SEQ ID No.52; a heavy chain CDR1 comprises amino acid sequence of SEQ ID No.58, a heavy chain CDR2 comprises amino acid sequence of SEQ ID No.59 and a heavy chain CDR3 comprises amino acid sequence of SEQ ID No.60; a heavy chain CDR1 comprises amino acid sequence of SEQ ID No.66, a heavy chain CDR2 comprises amino acid sequence of SEQ ID No.67 and a heavy chain CDR3 comprises amino acid sequence of SEQ ID No.68; a heavy chain CDR1 comprises amino acid sequence of SEQ ID No.74, a heavy chain CDR2 comprises amino acid sequence of SEQ ID No.75 and a heavy chain CDR3 comprises amino acid sequence of SEQ ID No.76; a heavy chain CDR1 comprises amino acid sequence of SEQ ID No.82, a heavy chain CDR2 comprises amino acid sequence of SEQ ID No.83 and a heavy chain CDR3 comprises amino acid sequence of SEQ ID No.84; a heavy chain CDR1 comprises amino acid sequence of SEQ ID No.90, a heavy chain CDR2 comprises amino acid sequence of SEQ ID No.91 and a heavy chain CDR3 comprises amino acid sequence of SEQ ID No.92; a heavy chain CDR1 comprises amino acid sequence of SEQ ID No.98, a CDR2 comprises amino acid sequence of SEQ ID No.99 and a heavy chain CDR3 comprises amino acid sequence of SEQ ID No.100;
a light chain CDR1 comprises amino acid sequence of SEQ ID No.6, a light chain CDR2 comprises amino acid sequence of SEQ ID No.7 and a light chain CDR3 comprises amino acid sequence of SEQ ID No.8; a light chain CDR1 comprises amino acid sequence of SEQ ID No.14, a light chain CDR2 comprises amino acid sequence of SEQ ID No.15 and a light chain CDR3 comprises amino acid sequence of SEQ ID No.16; a light chain CDR1 comprises amino acid sequence of SEQ ID No.22, a light chain CDR2 comprises amino acid sequence of SEQ ID No.23 and a light chain CDR3 comprises amino acid sequence of SEQ ID No.24; a light chain CDR1 comprises amino acid sequence of SEQ ID No.30, a light chain CDR2 comprises amino acid sequence of SEQ ID No.31 and a light chain CDR3 comprises amino acid sequence of SEQ ID No.32; a light chain CDR1 comprises amino acid sequence of SEQ ID No.38, a light chain CDR2 comprises amino acid sequence of SEQ ID No.39 and a light chain CDR3 comprises amino acid sequence of SEQ ID No.40; a light chain CDR1 comprises amino acid sequence of SEQ ID No.46, a light chain CDR2 comprises amino acid sequence of SEQ ID No.47 and a light chain CDR3 comprises amino acid sequence of SEQ ID No.48; a light chain CDR1 comprises amino acid sequence of SEQ ID No.54, a light chain CDR2 comprises amino acid sequence of SEQ ID No.55 and a light chain CDR3 comprises amino acid sequence of SEQ ID No.56; a light chain CDR1 comprises amino acid sequence of SEQ ID No.62, a light chain CDR2 comprises amino acid sequence of SEQ ID No.63 and a light chain CDR3 comprises amino acid sequence of SEQ ID No.64; a light chain CDR1 comprises amino acid sequence of SEQ ID No.70, a light chain CDR2 comprises amino acid sequence of SEQ ID No.71 and a light chain CDR3 comprises amino acid sequence of SEQ ID No.72; a light chain CDR1 comprises amino acid sequence of SEQ ID No.78, a light chain CDR2 comprises amino acid sequence of SEQ ID No.79 and a light chain CDR3 comprises amino acid sequence of SEQ ID No.80; a light chain CDR1 comprises amino acid sequence of SEQ ID No.86, a light chain CDR2 comprises amino acid sequence of SEQ ID No.87 and a light chain CDR3 comprises amino acid sequence of SEQ ID No.88; a light chain CDR1 comprises amino acid sequence of SEQ ID No.94, a light chain CDR2 comprises amino acid sequence of SEQ ID No.95 and a CDR3 comprises amino acid sequence of SEQ ID No.96; or, a light chain light chain CDR1 comprises amino acid sequence of SEQ ID No.102, a light chain CDR2 comprises amino acid sequence of SEQ ID No.103 and a light chain CDR3 comprises amino acid sequence of SEQ ID No.104.

3. The protein of any one of claim 1-2, wherein said protein further comprises a framework region of BLyS antibody, the framework region comprises a framework region of heavy chain and/or a framework region of light chain; and preferably, the framework region of heavy chain is a framework region of human or mouse antibody heavy chain, and/or the framework region of light chain is a framework region of human or mouse antibody light chain; more preferably, the framework region of heavy chain is a framework region of human antibody heavy chain, prefer V_{H}1-18 of V_{H} exon, V_{H}3-7 of V_{H} exon or J_{H}-6 of J_{H} exon from a human antibody heavy chain, and the light chain framework region is a framework region of human antibody light chain, prefer B3 of V_{K} exon, J_{K}-4 of the J_{K} exon or A10 of the V_{K} exon from a human antibody light chain.

4. The protein of claim 3, wherein said protein comprises the variable region of BLyS antibody heavy chain and/or variable region of BLyS antibody light chain forming by CDR and framework, wherein the amino acid sequences of heavy chain variable region are represented by SEQ ID No.1, SEQ ID No.9, SEQ ID No.17, SEQ ID No.25, SEQ ID No.33, SEQ ID No.41, SEQ ID No.49, SEQ ID No.57, SEQ ID No.65, SEQ ID No.73, SEQ ID No.81, SEQ ID No.89, SEQ ID No.97, SEQ ID NO. 140, SEQ ID NO. 141, SEQ ID NO. 142, SEQ ID NO. 143, SEQ ID NO. 144, SEQ ID NO. 145, SEQ ID NO. 146, SEQ ID NO. 149, SEQ ID NO. 150, SEQ ID NO. 151 or SEQ ID NO. 152; the amino acid sequences of light chain variable region are represented by SEQ ID No.5, SEQ ID No.13, SEQ ID No.21, SEQ ID No.29, SEQ ID No.37, SEQ ID No.45, SEQ ID No.53, SEQ ID No.61, SEQ ID No.69, SEQ ID No.77, SEQ ID No.85, SEQ ID No.93, SEQ ID No.101, SEQ ID No.147, SEQ ID No.148, SEQ ID No.153, SEQ ID No.154, SEQ ID No.155, SEQ ID No.156 or SEQ ID No.157.

5. The protein of claim 4, wherein an amino acid sequence of the heavy chain variable region is represented by SEQ ID No.1 and an amino acid sequence of the light chain variable region is represented by SEQ ID No.5; an amino acid sequence of the heavy chain variable region is represented by SEQ ID No.9 and an amino acid sequence of the light chain variable region is represented by SEQ ID No.13; an amino acid sequence of the heavy chain variable region is represented by SEQ ID No.17 and an amino acid sequence of the light chain variable region is represented by SEQ ID No.21; an amino acid sequence of the heavy chain variable region is represented by SEQ ID No.25 and an amino acid sequence of the light chain variable region is represented by SEQ ID No.29; an amino acid sequence of the heavy chain variable region is represented by SEQ ID No.33 and an amino acid sequence of the light chain variable region is represented by SEQ ID No.37; an amino acid sequence of the heavy chain variable region is represented by SEQ ID No.41 and an amino acid sequence of the light chain variable region is represented by SEQ ID No.45; an amino acid sequence of the heavy chain variable region is represented by SEQ ID No.49 and an amino acid sequence of the light chain variable region is represented by SEQ ID No.53; an amino acid sequence of the heavy chain variable region is represented by SEQ ID No.57 and an amino acid sequence of the light chain variable region is represented by SEQ ID No.61; an amino acid sequence of the heavy chain variable region is represented by SEQ ID No.65 and an amino acid sequence of the light chain variable region is represented by SEQ ID No.69; an amino acid sequence of the heavy chain variable region is represented by SEQ ID No.73 and an amino acid sequence of the light chain variable region is represented by SEQ ID No.77; an amino acid sequence of the heavy chain variable region is represented by SEQ ID No.81 and an amino acid sequence of the light chain variable region is represented by SEQ ID No.85; an amino acid sequence of the heavy chain variable region is represented by SEQ ID No.89 and an amino acid sequence of the light chain variable region is represented by SEQ ID No.93; an amino acid sequence of the heavy chain variable region is represented by SEQ ID No.97 and an amino acid sequence of the light chain variable region is represented by SEQ ID No.101; an amino acid sequence of the heavy chain variable region is represented by SEQ ID No.140 and an amino acid sequence of the light chain variable region is represented by SEQ ID No.147; an amino acid sequence of the heavy chain variable region is represented by SEQ ID No.140 and an amino acid sequence of the light chain variable region is represented by SEQ ID No.148; an amino acid sequence of the heavy chain variable region is represented by SEQ ID No.141 and an amino acid sequence of the light chain variable region is represented by SEQ ID No.147; an amino acid sequence of the heavy chain variable region is represented by SEQ ID No.141 and an amino acid sequence of the light chain variable region is represented by SEQ ID No.148; an amino acid sequence of the heavy chain variable region is represented by SEQ ID No.142 and an amino acid sequence of the light chain variable region is represented by SEQ ID No.147; an amino acid sequence of the heavy chain variable region is represented by SEQ ID No.142 and an amino acid sequence of the light chain variable region is represented by SEQ ID No.148; an amino acid sequence of the heavy chain variable region is represented by SEQ ID No.143 and an amino acid sequence of the light chain variable region is represented by SEQ ID No.147; an amino acid sequence of the heavy chain variable region is represented by SEQ ID No.143 and an amino acid sequence of the light chain variable region is represented by SEQ ID No.148; an amino acid sequence of the heavy chain variable region is represented by SEQ ID No.144 and an amino acid sequence of the light chain variable region is represented by SEQ ID No.147; an amino acid sequence of the heavy chain variable region is represented by SEQ ID No.144 and an amino acid sequence of the light chain variable region is represented by SEQ ID No.148; an amino acid sequence of the heavy chain variable region is represented by SEQ ID No.145 and an amino acid sequence of the light chain variable region is represented by SEQ ID No.147; an amino acid sequence of the heavy chain variable region is represented by SEQ ID No.145 and an amino acid sequence of the light chain variable region is represented by SEQ ID No.148; a heavy chain variable region is represented by SEQ ID No.146 and an amino acid sequence of the light chain variable region is represented by SEQ ID No.147; an amino acid sequence of the heavy chain variable region is represented by SEQ ID No.146 and an amino acid sequence of the light chain variable region is represented by SEQ ID No.148; an amino acid sequence of the heavy chain variable region is represented by SEQ ID No.149 and an amino acid sequence of the light chain variable region is represented by SEQ ID No.153; an amino acid sequence of the heavy chain variable region is represented by SEQ ID No.149 and an amino acid sequence of the light chain variable region is represented by SEQ ID No.154; an amino acid sequence of the heavy chain variable region is represented by SEQ ID No.150 and an amino acid sequence of the light chain variable region is represented by SEQ ID No.153; an amino acid sequence of the heavy chain variable region is represented by SEQ ID No.150 and an amino acid sequence of the light chain variable region is represented by SEQ ID No.154; an amino acid sequence of the heavy chain variable region is represented by SEQ ID No.151 and an amino acid sequence of the light chain variable region is represented by SEQ ID No.153; an amino acid sequence of the heavy chain variable region is represented by SEQ ID No.151 and an amino acid sequence of the light chain variable region is represented by SEQ ID No.154; an amino acid sequence of the heavy chain variable region is represented by SEQ ID No.151 and an amino acid sequence of the light chain variable region is represented by SEQ ID No.155; an amino acid sequence of the heavy chain variable region is represented by SEQ ID No.151 and an amino acid sequence of the light chain variable region is represented by SEQ ID No.156; an amino acid sequence of the heavy chain variable region is represented by SEQ ID No.151 and an amino acid sequence of the light chain variable region is represented by SEQ ID No.157; an amino acid sequence of the heavy chain variable region is represented by SEQ ID No.152 and an amino acid sequence of the light chain variable region is represented by SEQ ID No.155; an amino acid sequence of the heavy chain variable region is represented by SEQ ID No.152 and an amino acid sequence of the light chain variable region is represented by SEQ ID No.156; an amino acid sequence of the heavy chain variable region is represented by SEQ ID No.152 and an amino acid sequence of the light chain variable region is represented by SEQ ID No.157.

6. The protein of any of claims 1-5, wherein said protein further comprises heavy chain constant region of antibody and/or light chain constant region of antibody.

7. The protein of claim 6, wherein the heavy chain constant region of antibody is a heavy chain constant region of human or mouse antibody, the light chain constant region of antibody is a light chain constant region of human or mouse antibody.

8. The protein of claim 7, wherein the heavy chain constant region of antibody is a heavy chain constant region of human antibody, the light chain constant region of antibody is a light chain constant region of human antibody.

9. The protein of any of claims 1-5, wherein the protein is monoclonal antibody, full-length antibody protein, antibody-antigen binding domain protein fragment, bispecific antibody, multispecific antibody, single-chain antibody fragment, single-domain or single-region antibody of BLyS antibody.

10. A nucleic acid, wherein said nucleic acid encodes the protein of any of claims 1-9.

11. The nucleic acid of claim 10, wherein the nucleotide sequences of the nucleic acid encoding the heavy chain variable region are shown in SEQ ID No.105, SEQ ID No.107, SEQ ID No.109, SEQ ID No.111, SEQ ID No.113, SEQ ID No.115, SEQ ID No.117, SEQ ID No.119, SEQ ID No.121, SEQ ID No.123, SEQ ID No.125, SEQ ID No.127, SEQ ID No.129, SEQ ID NO. 158, SEQ ID NO. 159, SEQ ID NO. 160, SEQ ID NO. 161, SEQ ID NO. 162, SEQ ID NO. 163, SEQ ID NO. 164, SEQ ID NO. 167, SEQ ID NO. 168, SEQ ID NO. 169 or SEQ ID NO. 170; and/or, the nucleotide sequences of the nucleic acid encoding the light chain variable region are shown in SEQ ID No.106, SEQ ID No.108, SEQ ID No.110, SEQ ID No.112, SEQ ID No.114, SEQ ID No.116, SEQ ID No.118, SEQ ID No.120, SEQ ID No.122, SEQ ID No.124, SEQ ID No.126, SEQ ID No.128, SEQ ID No.130, SEQ ID No.165, SEQ ID No.166, SEQ ID No.171, SEQ ID No.172, SEQ ID No.173, SEQ ID No.174 or SEQ ID No.175.

12. The nucleic acid of claim 11, wherein a nucleotide sequence of the nucleic acid encoding the heavy chain variable region is shown in SEQ ID No.105, and a nucleotide sequence of the nucleic acid encoding the light chain variable region is shown in SEQ ID No.106; a nucleotide sequence of the nucleic acid encoding the heavy chain variable region is shown in SEQ ID No.107, and a nucleotide sequence of the nucleic acid encoding the light chain variable region is shown in SEQ ID No.108; a nucleotide sequence of the nucleic acid encoding the heavy chain variable region is shown in SEQ ID No.109, and a nucleotide sequence of the nucleic acid encoding the light chain variable region is shown in SEQ ID No.110; a nucleotide sequence of the nucleic acid encoding the heavy chain variable region is shown in SEQ ID No.111, and a nucleotide sequence of the nucleic acid encoding the light chain variable region is shown in SEQ ID No.112; a nucleotide sequence of the nucleic acid encoding the heavy chain variable region is shown in SEQ ID No.113, and a nucleotide sequence of the nucleic acid encoding the light chain variable region is shown in SEQ ID No.114; a nucleotide sequence of the nucleic acid encoding the heavy chain variable region is shown in SEQ ID No.115, and a nucleotide sequence of the nucleic acid encoding the light chain variable region is shown in SEQ ID No.116; a nucleotide sequence of the nucleic acid encoding the heavy chain variable region is shown in SEQ ID No.117, and a nucleotide sequence of the nucleic acid encoding the light chain variable region is shown in SEQ ID No.118; a nucleotide sequence of the nucleic acid encoding the heavy chain variable region is shown in SEQ ID No.119, and a nucleotide sequence of the nucleic acid encoding the light chain variable region is shown in SEQ ID No.120; a nucleotide sequence of the nucleic acid encoding the heavy chain variable region is shown in SEQ ID No.121, and a nucleotide sequence of the nucleic acid encoding the light chain variable region is shown in SEQ ID No.122; a nucleotide sequence of the nucleic acid encoding the heavy chain variable region is shown in SEQ ID No.123, and a nucleotide sequence of the nucleic acid encoding the light chain variable region is shown in SEQ ID No.124; a nucleotide sequence of the nucleic acid encoding the heavy chain variable region is shown in SEQ ID No.125, and a nucleotide sequence of the nucleic acid encoding the light chain variable region is shown in SEQ ID No.126; a nucleotide sequence of the nucleic acid encoding the heavy chain variable region is shown in SEQ ID No.127, and a nucleotide sequence of the nucleic acid encoding the light chain variable region is shown in SEQ ID No.128; a nucleotide sequence of the nucleic acid encoding the heavy chain variable region is shown in SEQ ID No.129, and a nucleotide sequence of the nucleic acid encoding the light chain variable region is shown in SEQ ID No.130; a nucleotide sequence of the nucleic acid encoding the heavy chain variable region is shown in SEQ ID No.158, and a nucleotide sequence of the nucleic acid encoding the light chain variable region is shown in SEQ ID No.165; a nucleotide sequence of the nucleic acid encoding the heavy chain variable region is shown in SEQ ID No.158, and a nucleotide sequence of the nucleic acid encoding the light chain variable region is shown in SEQ ID No.166; a nucleotide sequence of the nucleic acid encoding the heavy chain variable region is shown in SEQ ID No.159, and a nucleotide sequence of the nucleic acid encoding the light chain variable region is shown in SEQ ID No.165; a nucleotide sequence of the nucleic acid encoding the heavy chain variable region is shown in SEQ ID No.159, and a nucleotide sequence of the nucleic acid encoding the light chain variable region is shown in SEQ ID No.166; a nucleotide sequence of the nucleic acid encoding the heavy chain variable region is shown in SEQ ID No.160, and a nucleotide sequence of the nucleic acid encoding the light chain variable region is shown in SEQ ID No.165; a nucleotide sequence of the nucleic acid encoding the heavy chain variable region is shown in SEQ ID No.160, and a nucleotide sequence of the nucleic acid encoding the light chain variable region is shown in SEQ ID No.166; a nucleotide sequence of the nucleic acid encoding the heavy chain variable region is shown in SEQ ID No.161, and a nucleotide sequence of the nucleic acid encoding the light chain variable region is shown in SEQ ID No.165; a nucleotide sequence of the nucleic acid encoding the heavy chain variable region is shown in SEQ ID No.161, and a nucleotide sequence of the nucleic acid encoding the light chain variable region is shown in SEQ ID No.166; a nucleotide sequence of the nucleic acid encoding the heavy chain variable region is shown in SEQ ID No.162, and a nucleotide sequence of the nucleic acid encoding the light chain variable region is shown in SEQ ID No.165; a nucleotide sequence of the nucleic acid encoding the heavy chain variable region is shown in SEQ ID No.162, and a nucleotide sequence of the nucleic acid encoding the light chain variable region is shown in SEQ ID No.166; a nucleotide sequence of the nucleic acid encoding the heavy chain variable region is shown in SEQ ID No.163, and a nucleotide sequence of the nucleic acid encoding the light chain variable region is shown in SEQ ID No.165; a nucleotide sequence of the nucleic acid encoding the heavy chain variable region is shown in SEQ ID No.163, and a nucleotide sequence of the nucleic acid encoding the light chain variable region is shown in SEQ ID No.166; a nucleotide sequence of the nucleic acid encoding the heavy chain variable region is shown in SEQ ID No.164, and a nucleotide sequence of the nucleic acid encoding the light chain variable region is shown in SEQ ID No.165; a nucleotide sequence of the nucleic acid encoding the heavy chain variable region is shown in SEQ ID No.164, and a nucleotide sequence of the nucleic acid encoding the light chain variable region is shown in SEQ ID No.166; a nucleotide sequence of the nucleic acid encoding the heavy chain variable region is shown in SEQ ID No.167, and a nucleotide sequence of the nucleic acid encoding the light chain variable region is shown in SEQ ID No.171; a nucleotide sequence of the nucleic acid encoding the heavy chain variable region is shown in SEQ ID No.167, and a nucleotide sequence of the nucleic acid encoding the light chain variable region is shown in SEQ ID No.172; a nucleotide sequence of the nucleic acid encoding the heavy chain variable region is shown in SEQ ID No.168, and a nucleotide sequence of the nucleic acid encoding the light chain variable region is shown in SEQ ID No.171; a nucleotide sequence of the nucleic acid encoding the heavy chain variable region is shown in SEQ ID No.168, and a nucleotide sequence of the nucleic acid encoding the light chain variable region is shown in SEQ ID No.172; a nucleotide sequence of the nucleic acid encoding the heavy chain variable region is shown in SEQ ID No.169, and a nucleotide sequence of the nucleic acid encoding the light chain variable region is shown in SEQ ID No.171; a nucleotide sequence of the nucleic acid encoding the heavy chain variable region is shown in SEQ ID No.169, and a nucleotide sequence of the nucleic acid encoding the light chain variable region is shown in SEQ ID No.172; a nucleotide sequence of the nucleic acid encoding the heavy chain variable region is shown in SEQ ID No.169, and a nucleotide sequence of the nucleic acid encoding the light chain variable region is shown in SEQ ID No.173; a nucleotide sequence of the nucleic acid encoding the heavy chain variable region is shown in SEQ ID No.169, and a nucleotide sequence of the nucleic acid encoding the light chain variable region is shown in SEQ ID No.174; a nucleotide sequence of the nucleic acid encoding the heavy chain variable region is shown in SEQ ID No.169, and a nucleotide sequence of the nucleic acid encoding the light chain variable region is shown in SEQ ID No.175; a nucleotide sequence of the nucleic acid encoding the heavy chain variable region is shown in SEQ ID No.170, and a nucleotide sequence of the nucleic acid encoding the light chain variable region is shown in SEQ ID No.173; a nucleotide sequence of the nucleic acid encoding the heavy chain variable region is shown in SEQ ID No.170, and a nucleotide sequence of the nucleic acid encoding the light chain variable region is shown in SEQ ID No.174; a nucleotide sequence of the nucleic acid encoding the heavy chain variable region is shown in SEQ ID No.170, and a nucleotide sequence of the nucleic acid encoding the light chain variable region is shown in SEQ ID No.175.

13. A recombinant expression vector comprising the nucleic acid of any one of claims 10-12.

14. A recombinant expression transformant comprising the recombinant expression vector of claim 13.

15. A method for preparing BLyS antibody, comprising following steps: culture the recombinant expression transformant of claim 14, harvest BLyS antibody from the culture.

16. A method for detecting BLyS protein-overexpressing cells, comprising following steps: contact a protein according to any one of claims 1-9 with a sample to be tested *in vitro,* detect the combination between the protein according to any one of claims 1-9 and the sample to be tested.

17. A composition for detecting BLyS protein-overexpressing cells, wherein said composition comprises the protein according to any one of claims 1-9 as an active ingredient.

18. A pharmaceutical composition, wherein said composition comprises the protein according to any one of claims 1-9 as an active ingredient and a pharmaceutically acceptable carrier.

19. A pharmaceutical composition of claim 18, wherein said pharmaceutical composition comprises 0.01-99.99% of the protein according to any one of claims 1-9 and 0.01-99.99% of pharmaceutical carrier, the percentage is the mass percentage of the pharmaceutical composition.

20. The use of a protein according to any one of claims 1-9 or a pharmaceutical composition according to any one of claims 18-19 in the preparation of drugs for preventing or treating diseases associated with abnormal expression or dysfunction of BLyS; preferably, the diseases associated with abnormal expression or dysfunction of BLyS are autoimmune diseases, inflammatory diseases, infectious diseases or proliferative diseases.


SEQUENCE LISTING

<110>  Pharmaexplorer Limited

<120>  BlyS Antibody, Method Of Its Preparation And Use Thereof

<130>  P21283WOEP

<150>  201610527996.0
<151>  2016-07-06

<160>  187

<170>  PatentIn version 3.5

<210>  1
<211>  125
<212>  PRT
<213>  Artificial sequence

<220>
<223>  1

<400>  1

Glu Val Gln Leu Val Glu Ser Arg Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Ala Ala Val Tyr Tyr Cys
                85                  90                  95

Ala Arg Asp Arg Val Asp Ile Leu Thr Gly Tyr Ser Ile Tyr Gly Met
            100                 105                 110

Asp Val Trp Gly Gln Gly Thr Thr Val Thr Val Ser Ser
        115                 120                 125

<210>  2
<211>  10
<212>  PRT
<213>  Artificial sequence

<220>

<223>  2

<400>  2

Gly Phe Thr Phe Ser Ser Tyr Gly Met His
1               5                   10


<210>  3
<211>  17
<212>  PRT
<213>  Artificial sequence

<220>
<223>  3

<400>  3

Val Ile Ser Tyr Asp Gly Ser Asn Lys Tyr Tyr Ala Asp Ser Val Lys
1               5                   10                  15


Gly




<210>  4
<211>  16
<212>  PRT
<213>  Artificial sequence

<220>
<223>  4

<400>  4

Asp Arg Val Asp Ile Leu Thr Gly Tyr Ser Ile Tyr Gly Met Asp Val
1               5                   10                  15


<210>  5
<211>  107
<212>  PRT
<213>  Homo sapiens

<400>  5

Glu Ile Val Met Thr Gln Ser Pro Ala Thr Leu Ser Leu Ser Pro Gly
1               5                   10                  15


Glu Arg Ala Thr Leu Ser Cys Arg Ala Ser Gln Ser Val Ser Ser Tyr
            20                  25                  30


Leu Ala Trp Tyr Gln Gln Lys Pro Gly Gln Ala Pro Arg Leu Leu Ile
        35                  40                  45


Tyr Asp Ala Ser Lys Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Leu
                85                  90                  95

Thr Phe Gly Gly Gly Thr Lys Val Glu Ile Lys
            100                 105

<210>  6
<211>  11
<212>  PRT
<213>  Artificial sequence

<220>
<223>  6

<400>  6

Arg Ala Ser Gln Ser Val Ser Ser Tyr Leu Ala
1               5                   10

<210>  7
<211>  7
<212>  PRT
<213>  Artificial sequence

<220>
<223>  7

<400>  7

Asp Ala Ser Lys Arg Ala Thr
1               5

<210>  8
<211>  9
<212>  PRT
<213>  Artificial sequence

<220>
<223>  8

<400>  8

Gln Gln Arg Ser Asn Trp Pro Leu Thr
1               5

<210>  9
<211>  125
<212>  PRT
<213>  Artificial sequence

<220>
<223>  9

<400>  9

Gln Ile Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Met
        35                  40                  45

Gly Trp Ile Ser Ala Tyr Asn Gly Asn Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Thr Tyr Tyr Asp Ile Leu Thr Gly Tyr Tyr Tyr Tyr Gly Met
            100                 105                 110

Asp Val Trp Gly Gln Gly Thr Thr Val Thr Val Ser Ser
        115                 120                 125

<210>  10
<211>  10
<212>  PRT
<213>  Artificial sequence

<220>
<223>  10

<400>  10

Gly Tyr Thr Phe Thr Ser Tyr Gly Ile Ser
1               5                   10

<210>  11
<211>  17
<212>  PRT
<213>  Artificial sequence

<220>
<223>  11

<400>  11

Trp Ile Ser Ala Tyr Asn Gly Asn Thr Asn Tyr Ala Gln Lys Leu Gln
1               5                   10                  15

Gly

<210>  12
<211>  16
<212>  PRT
<213>  Artificial sequence

<220>
<223>  12

<400>  12

Thr Tyr Tyr Asp Ile Leu Thr Gly Tyr Tyr Tyr Tyr Gly Met Asp Val
1               5                   10                  15


<210>  13
<211>  107
<212>  PRT
<213>  Artificial sequence

<220>
<223>  13

<400>  13

Glu Ile Val Leu Thr Gln Ser Pro Gly Thr Leu Ser Leu Ser Pro Gly
1               5                   10                  15


Glu Arg Ala Thr Leu Ser Cys Arg Thr Ser Gln Ser Val Asp Thr Tyr
            20                  25                  30


Leu Ala Trp Tyr Gln Gln Lys Pro Gly Gln Ala Pro Arg Leu Leu Ile
        35                  40                  45


Tyr Asp Ala Ser Asn Arg Ala Thr Gly Ile Pro Ala Arg Phe Ser Gly
    50                  55                  60


Ser Gly Ser Gly Thr Asp Phe Thr Leu Thr Ile Ser Ser Leu Glu Pro
65                  70                  75                  80


Glu Asp Phe Ala Val Tyr Tyr Cys Gln Gln Arg Ser Asn Trp Pro Leu
                85                  90                  95


Thr Phe Gly Gly Gly Thr Lys Val Glu Ile Lys
            100                 105


<210>  14
<211>  11
<212>  PRT
<213>  Artificial sequence

<220>
<223>  14

<400>  14

Arg Thr Ser Gln Ser Val Asp Thr Tyr Leu Ala
1               5                   10

<210>  15
<211>  7
<212>  PRT
<213>  Artificial sequence

<220>
<223>  15

<400>  15

Asp Ala Ser Asn Arg Ala Thr
1               5

<210>  16
<211>  9
<212>  PRT
<213>  Artificial sequence

<220>
<223>  16

<400>  16

Gln Gln Arg Ser Asn Trp Pro Leu Thr
1               5

<210>  17
<211>  121
<212>  PRT
<213>  Artificial sequence

<220>
<223>  17

<400>  17

Gln Val Gln Leu Val Gln Ser Gly Gly Gly Leu Val Gln Pro Gly Gly
1               5                   10                  15

Ser Leu Arg Leu Ser Cys Ala Ala Ser Gly Phe Thr Val Ser Ser Asn
            20                  25                  30

Tyr Met Ser Trp Val Arg Gln Ala Pro Gly Lys Gly Leu Glu Trp Val
        35                  40                  45

Ser Val Ile Tyr Ser Gly Gly Ser Thr Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Arg Gly Phe His Asp Ile Leu Thr Gly Tyr Gly Met Asp Val Trp Gly
            100                 105                 110

Gln Gly Thr Thr Val Thr Val Ser Ser
        115                 120

<210>  18
<211>  10
<212>  PRT
<213>  Artificial sequence

<220>
<223>  18

<400>  18

Gly Phe Thr Val Ser Ser Asn Tyr Met Ser
1               5                   10

<210>  19
<211>  16
<212>  PRT
<213>  Artificial sequence

<220>
<223>  19

<400>  19

Val Ile Tyr Ser Gly Gly Ser Thr Tyr Tyr Ala Asp Ser Val Lys Gly
1               5                   10                  15

<210>  20
<211>  13
<212>  PRT
<213>  Artificial sequence

<220>
<223>  20

<400>  20

Gly Phe His Asp Ile Leu Thr Gly Tyr Gly Met Asp Val
1               5                   10

<210>  21
<211>  109
<212>  PRT
<213>  Artificial sequence

<220>
<223>  21

<400>  21

Ser Ser Glu Leu Thr Gln Asp Pro Ala Val Ser Val Ala Leu Gly Gln
1               5                   10                  15

Thr Val Arg Ile Thr Cys Gln Gly Asp Ser Leu Arg Asn Tyr Tyr Ala
            20                  25                  30

Gly Trp Tyr Gln Gln Lys Pro Gly Gln Ala Pro Val Leu Val Ile Tyr
        35                  40                  45

Gly Lys Asn Asn Arg Pro Ser Gly Ile Pro Asp Arg Phe Ser Gly Ser
    50                  55                  60

Ser Ser Gly Asn Thr Ala Ser Leu Thr Ile Thr Gly Thr Gln Ala Glu
65                  70                  75                  80

Asp Glu Ala Asp Tyr Tyr Cys Ser Ser Arg Asp Asn Ser Gly Thr His
                85                  90                  95

Leu Val Glu Phe Gly Gly Gly Thr Lys Leu Thr Val Leu
            100                 105

<210>  22
<211>  11
<212>  PRT
<213>  Artificial sequence

<220>
<223>  22

<400>  22

Gln Gly Asp Ser Leu Arg Asn Tyr Tyr Ala Gly
1               5                   10

<210>  23
<211>  7
<212>  PRT
<213>  Artificial sequence

<220>
<223>  23

<400>  23

Gly Lys Asn Asn Arg Pro Ser
1               5

<210>  24
<211>  12
<212>  PRT
<213>  Artificial sequence

<220>

<223>  24

<400>  24

Ser Ser Arg Asp Asn Ser Gly Thr His Leu Val Glu
1               5                   10


<210>  25
<211>  118
<212>  PRT
<213>  Artificial sequence

<220>
<223>  25

<400>  25

Glu Val Gln Leu Gln Gln Ser Gly Ala Glu Leu Val Lys Pro Gly Ala
1               5                   10                  15


Ser Val Lys Leu Ser Cys Thr Ala Ala Gly Phe Asn Ile Gln Asp Thr
            20                  25                  30


Tyr Ile His Trp Val Lys Gln Arg Pro Glu Gln Gly Leu Glu Trp Ile
        35                  40                  45


Gly Arg Ile Asp Pro Ala Asn Ala Asn Thr Val Tyr Asp Pro Lys Phe
    50                  55                  60


Gln Gly Lys Ala Thr Ile Thr Ala Asp Thr Ser Ser Asn Thr Ala Tyr
65                  70                  75                  80


Leu Gln Val Ser Ser Leu Thr Ser Glu Asp Thr Ala Val Tyr Tyr Cys
                85                  90                  95


Ala Ser Pro Val Ile Thr Ala Gly Phe Ala Tyr Trp Gly Gln Gly Thr
            100                 105                 110


Leu Val Thr Val Ser Ala
        115


<210>  26
<211>  10
<212>  PRT
<213>  Artificial sequence

<220>
<223>  26

<400>  26

Gly Phe Asn Ile Gln Asp Thr Tyr Ile His
1               5                   10

<210>  27
<211>  17
<212>  PRT
<213>  Artificial sequence

<220>
<223>  27

<400>  27

Arg Ile Asp Pro Ala Asn Ala Asn Thr Val Tyr Asp Pro Lys Phe Gln
1               5                   10                  15

Gly

<210>  28
<211>  9
<212>  PRT
<213>  Artificial sequence

<220>
<223>  28

<400>  28

Pro Val Ile Thr Ala Gly Phe Ala Tyr
1               5

<210>  29
<211>  106
<212>  PRT
<213>  Artificial sequence

<220>
<223>  29

<400>  29

Gln Ile Val Leu Thr Gln Ser Pro Ala Ile Met Ser Ala Ser Pro Gly
1               5                   10                  15

Glu Lys Val Thr Met Thr Cys Gly Ala Ser Ser Ser Val Asn Tyr Val
            20                  25                  30

His Trp Tyr Gln Gln Lys Ser Gly Thr Ser Pro Lys Arg Trp Ile Tyr
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys Gln Gln Trp Thr Arg Ser Pro Leu Thr
                85                  90                  95

Phe Gly Ala Gly Thr Lys Val Glu Leu Lys
            100                 105

<210>  30
<211>  10
<212>  PRT
<213>  Artificial sequence

<220>
<223>  30

<400>  30

Gly Ala Ser Ser Ser Val Asn Tyr Val His
1               5                   10

<210>  31
<211>  7
<212>  PRT
<213>  Artificial sequence

<220>
<223>  31

<400>  31

Asp Thr Ser Lys Leu Ala Ser
1               5

<210>  32
<211>  9
<212>  PRT
<213>  Artificial sequence

<220>
<223>  32

<400>  32

Gln Gln Trp Thr Arg Ser Pro Leu Thr
1               5

<210>  33
<211>  125
<212>  PRT
<213>  Artificial sequence

<220>
<223>  33

<400>  33

Gln Val Gln Leu Gln Gln Ser Gly Val Glu Leu Ala Arg Pro Gly Ala
1               5                   10                  15

Ser Val Lys Leu Ser Cys Lys Ala Ser Gly Tyr Thr Phe Thr Ser Tyr
            20                  25                  30

Gly Ile Ser Trp Val Lys Gln Arg Thr Gly Gln Ala Leu Glu Trp Ile
        35                  40                  45

Gly Glu Ile Tyr Pro Arg Ser Gly Asn Thr Tyr Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Glu Leu Arg Ser Leu Thr Ser Glu Asp Ser Ala Val Tyr Phe Cys
                85                  90                  95

Ala Arg Ser Gly Pro Ile Thr Thr Leu Val Ala Thr Asp Tyr Gly Met
            100                 105                 110

Asp Tyr Trp Gly Gln Gly Thr Ser Val Thr Val Ser Ser
        115                 120                 125

<210>  34
<211>  10
<212>  PRT
<213>  Artificial sequence

<220>
<223>  34

<400>  34

Gly Tyr Thr Phe Thr Ser Tyr Gly Ile Ser
1               5                   10

<210>  35
<211>  17
<212>  PRT
<213>  Artificial sequence

<220>
<223>  35

<400>  35

Glu Ile Tyr Pro Arg Ser Gly Asn Thr Tyr Tyr Asn Glu Lys Phe Lys
1               5                   10                  15

Gly

<210>  36
<211>  16

<212>  PRT
<213>  Artificial sequence

<220>
<223>  36

<400>  36

Ser Gly Pro Ile Thr Thr Leu Val Ala Thr Asp Tyr Gly Met Asp Tyr
1               5                   10                  15


<210>  37
<211>  111
<212>  PRT
<213>  Artificial sequence

<220>
<223>  37

<400>  37

Asp Ile Val Leu Thr Gln Ser Pro Ala Ser Leu Ala Val Ser Leu Gly
1               5                   10                  15


Gln Arg Ala Thr Ile Ser Cys Arg Ala Ser Lys Ser Val Ser Thr Ser
            20                  25                  30


Ser Tyr Ser Tyr Met His Trp Tyr Gln Gln Lys Pro Gly Gln Pro Pro
        35                  40                  45


Lys Leu Leu Ile Lys Phe Ala Ser Asn Leu Glu Ser Gly Val Pro Ala
    50                  55                  60


Arg Phe Ser Gly Ser Gly Ser Gly Thr Asp Phe Thr Leu Asn Ile His
65                  70                  75                  80


Pro Val Glu Glu Glu Asp Ala Ala Thr Tyr Tyr Cys Gln His Ser Arg
                85                  90                  95


Glu Phe Pro Tyr Thr Phe Gly Gly Gly Thr Lys Leu Glu Ile Lys
            100                 105                 110


<210>  38
<211>  15
<212>  PRT
<213>  Artificial sequence

<220>
<223>  38

<400>  38

Arg Ala Ser Lys Ser Val Ser Thr Ser Ser Tyr Ser Tyr Met His
1               5                   10                  15

<210>  39
<211>  7
<212>  PRT
<213>  Artificial sequence

<220>
<223>  39

<400>  39

Phe Ala Ser Asn Leu Glu Ser
1               5


<210>  40
<211>  9
<212>  PRT
<213>  Artificial sequence

<220>
<223>  40

<400>  40

Gln His Ser Arg Glu Phe Pro Tyr Thr
1               5


<210>  41
<211>  124
<212>  PRT
<213>  Artificial sequence

<220>
<223>  41

<400>  41

Glu Val Gln Leu Gln Gln Ser Val Ala Glu Leu Val Arg Pro Gly Ala
1               5                   10                  15


Ser Val Lys Leu Ser Cys Thr Ala Ser Asp Phe Asn Ile Lys Asn Thr
            20                  25                  30


Tyr Ile His Trp Val Lys Gln Arg Pro Glu Gln Gly Leu Glu Trp Ile
        35                  40                  45


Gly Arg Ile Asp Pro Val Asn Gly Asn Thr Lys Tyr Ala Pro Lys Phe
    50                  55                  60


Gln Asp Lys Ala Thr Ile Ile Val Asp Thr Tyr Ser Asn Thr Ala Tyr
65                  70                  75                  80


Leu Gln Leu Ser Ser Leu Ala Ser Glu Asp Thr Ala Val Tyr Tyr Cys
                85                  90                  95

Ala Arg Trp Pro Ile Thr Thr Ile Val Ala Thr Asp Trp Tyr Phe Asp
            100                 105                 110

Val Trp Gly Thr Gly Thr Thr Val Thr Val Ser Ser
        115                 120

<210>  42
<211>  10
<212>  PRT
<213>  Artificial sequence

<220>
<223>  42

<400>  42

Asp Phe Asn Ile Lys Asn Thr Tyr Ile His
1               5                   10

<210>  43
<211>  17
<212>  PRT
<213>  Artificial sequence

<220>
<223>  43

<400>  43

Arg Ile Asp Pro Val Asn Gly Asn Thr Lys Tyr Ala Pro Lys Phe Gln
1               5                   10                  15

Asp

<210>  44
<211>  15
<212>  PRT
<213>  Artificial sequence

<220>
<223>  44

<400>  44

Trp Pro Ile Thr Thr Ile Val Ala Thr Asp Trp Tyr Phe Asp Val
1               5                   10                  15

<210>  45
<211>  112
<212>  PRT
<213>  Artificial sequence

<220>
<223>  45

<400>  45

Asp Val Leu Met Thr Gln Thr Gln Leu Ala Leu Pro Val Arg Leu Gly
1               5                   10                  15

Glu Gln Ala Ser Ile Ser Cys Arg Ser Ser Gln Ser Ile Val Asn Ser
            20                  25                  30

Asn Gly Asn Thr Tyr Leu Glu Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Phe Gln Gly
                85                  90                  95

Ser His Val Pro Trp Thr Phe Gly Gly Gly Thr Lys Leu Glu Ile Lys
            100                 105                 110


<210>  46
<211>  16
<212>  PRT
<213>  Artificial sequence

<220>
<223>  46

<400>  46

Arg Ser Ser Gln Ser Ile Val Asn Ser Asn Gly Asn Thr Tyr Leu Glu
1               5                   10                  15


<210>  47
<211>  7
<212>  PRT
<213>  Artificial sequence

<220>
<223>  47

<400>  47

Lys Val Ser Asn Arg Phe Ser
1               5


<210>  48
<211>  7
<212>  PRT
<213>  Artificial sequence

<220>

<223>  48

<400>  48

Phe Gln Gly Ser His Val Pro
1               5


<210>  49
<211>  116
<212>  PRT
<213>  Artificial sequence

<220>
<223>  49

<400>  49

Gln Val Gln Leu Gln Gln Ser Gly Ala Glu Leu Val Arg Pro Gly Thr
1               5                   10                  15


Ser Val Lys Ile Ser Cys Lys Ala Ser Gly Tyr Ala Phe Thr Asn Tyr
            20                  25                  30


Trp Leu Gly Trp Val Lys Gln Arg Pro Gly His Gly Leu Glu Trp Ile
        35                  40                  45


Gly Asp Ile Tyr Pro Gly Ser Gly Asn Thr Tyr Tyr Asn Glu Lys Phe
    50                  55                  60


Lys Gly Lys Ala Thr Leu Thr Ala Asp Lys Ser Ser Ser Thr Ala Tyr
65                  70                  75                  80


Met Gln Leu Ser Ser Leu Thr Ser Glu Asp Ser Ala Val Phe Phe Cys
                85                  90                  95


Ala Ala Tyr Leu Tyr Ala Met Asp Tyr Trp Gly Gln Gly Thr Ser Val
            100                 105                 110


Thr Val Ser Ser
        115


<210>  50
<211>  7
<212>  PRT
<213>  Artificial sequence

<220>
<223>  50

<400>  50

Gly Tyr Ala Phe Thr Asn Tyr
1               5

<210>  51
<211>  17
<212>  PRT
<213>  Artificial sequence

<220>
<223>  51

<400>  51

Asp Ile Tyr Pro Gly Ser Gly Asn Thr Tyr Tyr Asn Glu Lys Phe Lys
1               5                   10                  15

Gly

<210>  52
<211>  7
<212>  PRT
<213>  Artificial sequence

<220>
<223>  52

<400>  52

Tyr Leu Tyr Ala Met Asp Tyr
1               5

<210>  53
<211>  106
<212>  PRT
<213>  Artificial sequence

<220>
<223>  53

<400>  53

Gln Ile Val Leu Thr Gln Ser Pro Ala Ile Met Ser Ala Ser Pro Gly
1               5                   10                  15

Glu Lys Val Thr Ile Thr Cys Ser Ala Ser Ser Ser Val Ser Tyr Met
            20                  25                  30

His Trp Phe Gln Gln Lys Pro Gly Thr Ser Pro Lys Leu Trp Ile Tyr
        35                  40                  45

Ser Ala Ser Asn Leu Ala Ser Gly Val Pro Ala Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Arg Gly Ser Tyr Pro Phe Thr
                85                  90                  95

Phe Gly Ser Gly Thr Lys Leu Glu Ile Lys
            100                 105

<210>  54
<211>  10
<212>  PRT
<213>  Artificial sequence

<220>
<223>  54

<400>  54

Ser Ala Ser Ser Ser Val Ser Tyr Met His
1               5                   10

<210>  55
<211>  7
<212>  PRT
<213>  Artificial sequence

<220>
<223>  55

<400>  55

Ser Ala Ser Asn Leu Ala Ser
1               5

<210>  56
<211>  9
<212>  PRT
<213>  Artificial sequence

<220>
<223>  56

<400>  56

Gln Gln Arg Gly Ser Tyr Pro Phe Thr
1               5

<210>  57
<211>  120
<212>  PRT
<213>  Artificial sequence

<220>
<223>  57

<400>  57

Gln Val Gln Leu Lys Glu Ser Gly Pro Gly Leu Val Ala Pro Ser Gln
1               5                   10                  15

Ser Leu Ser Ile Thr Cys Thr Val Ser Gly Leu Ser Leu Asn Ser His
            20                  25                  30

Asp Ile Ser Trp Ile Arg Gln Pro Pro Gly Lys Gly Leu Glu Trp Leu
        35                  40                  45

Gly Val Ile Trp Ser Gly Gly Gly Thr Lys Tyr Asn Ser Ala Phe Met
    50                  55                  60

Ser Arg Leu Ser Ile Ser Lys Asp Asn Ser Lys Ser Gln Val Phe Leu
65                  70                  75                  80

Lys Met Asn Ser Leu His Thr Asp Asp Thr Ala Ile Tyr Tyr Cys Val
                85                  90                  95

Arg Thr Pro Ser Met Ile Thr Tyr Ser Ala Met Asp Tyr Trp Gly Gln
            100                 105                 110

Gly Thr Ser Val Thr Val Ser Ser
        115                 120

<210>  58
<211>  10
<212>  PRT
<213>  Artificial sequence

<220>
<223>  58

<400>  58

Gly Leu Ser Leu Asn Ser His Asp Ile Ser
1               5                   10

<210>  59
<211>  16
<212>  PRT
<213>  Artificial sequence

<220>
<223>  59

<400>  59

Val Ile Trp Ser Gly Gly Gly Thr Lys Tyr Asn Ser Ala Phe Met Ser
1               5                   10                  15

<210>  60
<211>  12
<212>  PRT
<213>  Artificial sequence

<220>

<223>  60

<400>  60

Thr Pro Ser Met Ile Thr Tyr Ser Ala Met Asp Tyr
1               5                   10


<210>  61
<211>  108
<212>  PRT
<213>  Artificial sequence

<220>
<223>  61

<400>  61

Glu Ile Val Leu Thr Gln Ser Pro Ala Leu Met Ala Ala Ser Pro Gly
1               5                   10                  15


Glu Lys Val Thr Ile Thr Cys Ser Val Ser Ser Ser Ile Ser Ser Arg
            20                  25                  30


Asn Leu His Trp Tyr Gln Gln Lys Ser Gln Thr Ser Pro Lys Ala Trp
        35                  40                  45


Ile His Ala Thr Ser Asn Leu Ala Ser Gly Val Pro Val Arg Phe Ser
    50                  55                  60


Gly Ser Gly Ser Gly Thr Ser Tyr Ser Leu Thr Ile Ser Ser Met Glu
65                  70                  75                  80


Ala Glu Asp Ala Ala Thr Tyr Tyr Cys Gln Gln Trp Ser Ser Tyr Pro
                85                  90                  95


Leu Thr Phe Gly Ser Gly Thr Lys Leu Glu Ile Lys
            100                 105


<210>  62
<211>  12
<212>  PRT
<213>  Artificial sequence

<220>
<223>  62

<400>  62

Ser Val Ser Ser Ser Ile Ser Ser Arg Asn Leu His
1               5                   10


<210>  63
<211>  7
<212>  PRT

<213>  Artificial sequence

<220>
<223>  63

<400>  63

Ala Thr Ser Asn Leu Ala Ser
1               5


<210>  64
<211>  9
<212>  PRT
<213>  Artificial sequence

<220>
<223>  64

<400>  64

Gln Gln Trp Ser Ser Tyr Pro Leu Thr
1               5


<210>  65
<211>  118
<212>  PRT
<213>  Artificial sequence

<220>
<223>  65

<400>  65

Glu Val Gln Leu Gln Gln Ser Gly Pro Glu Leu Val Lys Ser Gly Ala
1               5                   10                  15

Ser Val Lys Ile Ser Cys Lys Ala Ser Gly Tyr Thr Phe Thr Asp His
            20                  25                  30

Lys Met His Trp Val Lys Gln Ser His Gly Lys Ser Leu Glu Trp Ile
        35                  40                  45

Gly Tyr Ile Ser Pro Tyr Asn Gly Gly Thr Gly Tyr Asn Gln Lys Phe
    50                  55                  60

Lys Ser Lys Val Thr Leu Thr Val Asp Ile Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Ala Arg Trp Gly Phe Tyr Asp Val Met Asp Ser Trp Gly Gln Gly Thr
            100                 105                 110

Ser Val Thr Val Ser Ser
        115


<210>  66
<211>  10
<212>  PRT
<213>  Artificial sequence

<220>
<223>  66

<400>  66

Gly Tyr Thr Phe Thr Asp His Lys Met His
1               5                   10


<210>  67
<211>  17
<212>  PRT
<213>  Artificial sequence

<220>
<223>  67

<400>  67

Tyr Ile Ser Pro Tyr Asn Gly Gly Thr Gly Tyr Asn Gln Lys Phe Lys
1               5                   10                  15


Ser




<210>  68
<211>  9
<212>  PRT
<213>  Artificial sequence

<220>
<223>  68

<400>  68

Trp Gly Phe Tyr Asp Val Met Asp Ser
1               5


<210>  69
<211>  112
<212>  PRT
<213>  Artificial sequence

<220>
<223>  69

<400>  69

Asp Val Leu Leu Thr Gln Thr Pro Leu Ser Leu Pro Val Ser Leu Gly
1               5                   10                  15

Asp Gln Ala Ser Ile Ser Cys Arg Ser Ser Gln Thr Ile Val His Ile
            20                  25                  30

Ile Gly Asp Thr Tyr Leu Glu Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ala Gly Thr Asp Phe Thr Leu Lys Ile
65                  70                  75                  80

Ser Arg Val Glu Ala Glu Asp Leu Gly Val Tyr Tyr Cys Phe Gln Gly
                85                  90                  95

Ser His Val Pro Arg Thr Phe Gly Gly Gly Thr Lys Leu Glu Ile Lys
            100                 105                 110

<210>  70
<211>  16
<212>  PRT
<213>  Artificial sequence

<220>
<223>  70

<400>  70

Arg Ser Ser Gln Thr Ile Val His Ile Ile Gly Asp Thr Tyr Leu Glu
1               5                   10                  15

<210>  71
<211>  7
<212>  PRT
<213>  Artificial sequence

<220>
<223>  71

<400>  71

Lys Val Ser Asn Arg Phe Ser
1               5

<210>  72
<211>  9
<212>  PRT
<213>  Artificial sequence

<220>
<223>  72

<400>  72

Phe Gln Gly Ser His Val Pro Arg Thr
1               5

<210>  73
<211>  120
<212>  PRT
<213>  Artificial sequence

<220>
<223>  73

<400>  73

Glu Val Lys Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser Thr Tyr
            20                  25                  30

Thr Met Ser Trp Val Arg Gln Thr Pro Glu Lys Arg Leu Glu Trp Val
        35                  40                  45

Ala Tyr Ile Ser Tyr Gly Gly Gly Ser Thr Ser Tyr Pro Asp Thr Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Ile Tyr Tyr Cys
                85                  90                  95

Thr Arg His Val Thr Val Val Glu Asn Ser Met Asp Tyr Trp Gly Gln
            100                 105                 110

Gly Thr Ser Val Thr Val Ser Ser
        115                 120

<210>  74
<211>  10
<212>  PRT
<213>  Artificial sequence

<220>
<223>  74

<400>  74

Gly Phe Thr Phe Ser Thr Tyr Thr Met Ser
1               5                   10

<210>  75
<211>  17
<212>  PRT

<213>  Artificial sequence

<220>
<223>  75

<400>  75

Tyr Ile Ser Tyr Gly Gly Gly Ser Thr Ser Tyr Pro Asp Thr Val Lys
1               5                   10                  15

Gly

<210>  76
<211>  11
<212>  PRT
<213>  Artificial sequence

<220>
<223>  76

<400>  76

His Val Thr Val Val Glu Asn Ser Met Asp Tyr
1               5                   10

<210>  77
<211>  107
<212>  PRT
<213>  Artificial sequence

<220>
<223>  77

<400>  77

Asp Ile Leu Leu Thr Gln Ser Pro Asp Ile Leu Ser Val Ser Pro Gly
1               5                   10                  15

Glu Arg Val Ser Phe Ser Cys Arg Ala Ser Gln Ser Ile Gly Thr Ser
            20                  25                  30

Ile His Trp Tyr Gln Gln Arg Thr Asn Gly Ser Pro Arg Leu Leu Ile
        35                  40                  45

Lys Tyr Val Ser Glu Ser Phe Ser Gly Thr Pro Ser Arg Phe Ser Gly
    50                  55                  60

Ser Gly Ser Gly Thr Ala Phe Thr Leu Thr Ile Asn Ser Val Glu Ser
65                  70                  75                  80

Glu Asp Ile Ala Asp Tyr Tyr Cys Gln Gln Thr His Ser Trp Pro Trp
                85                  90                  95

Thr Phe Gly Gly Gly Thr Lys Leu Glu Ile Lys
            100                 105

<210>  78
<211>  11
<212>  PRT
<213>  Artificial sequence

<220>
<223>  78

<400>  78

Arg Ala Ser Gln Ser Ile Gly Thr Ser Ile His
1               5                   10

<210>  79
<211>  7
<212>  PRT
<213>  Artificial sequence

<220>
<223>  79

<400>  79

Tyr Val Ser Glu Ser Phe Ser
1               5

<210>  80
<211>  9
<212>  PRT
<213>  Artificial sequence

<220>
<223>  80

<400>  80

Gln Gln Thr His Ser Trp Pro Trp Thr
1               5

<210>  81
<211>  122
<212>  PRT
<213>  Artificial sequence

<220>
<223>  81

<400>  81

Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asn Ile Lys Asn Tyr
            20                  25                  30

Tyr Met His Trp Val Lys Gln Arg Thr Glu Glu Gly Leu Glu Trp Ile
        35                  40                  45

Gly Arg Ile Asp Pro Glu Asp Gly Glu Thr Lys Tyr Ala Pro Lys Phe
    50                  55                  60

Gln Ala Lys Ala Thr Ile Thr Ala Asp Thr Ser Ser Asn Thr Ala Tyr
65                  70                  75                  80

Leu Gln Leu Ser Ser Leu Thr Ser Glu Asp Thr Ala Val Tyr Phe Cys
                85                  90                  95

Ala Arg Lys Gly Tyr Tyr Gly Asn Tyr Gly Ala Trp Phe Ala Tyr Trp
            100                 105                 110

Gly Gln Gly Thr Leu Val Thr Val Ser Ala
        115                 120

<210>  82
<211>  10
<212>  PRT
<213>  Artificial sequence

<220>
<223>  82

<400>  82

Gly Phe Asn Ile Lys Asn Tyr Tyr Met His
1               5                   10

<210>  83
<211>  17
<212>  PRT
<213>  Artificial sequence

<220>
<223>  83

<400>  83

Arg Ile Asp Pro Glu Asp Gly Glu Thr Lys Tyr Ala Pro Lys Phe Gln
1               5                   10                  15

Ala

<210>  84
<211>  13
<212>  PRT
<213>  Artificial sequence

<220>

<223>  84

<400>  84

Lys Gly Tyr Tyr Gly Asn Tyr Gly Ala Trp Phe Ala Tyr
1               5                   10


<210>  85
<211>  107
<212>  PRT
<213>  Artificial sequence

<220>
<223>  85

<400>  85

Asp Ile Gln Met Thr Gln Ser Pro Ala Ser Leu Ser Val Ser Val Gly
1               5                   10                  15


Glu Thr Val Thr Ile Thr Cys Arg Ala Ser Glu Asp Leu Tyr Ser Asn
            20                  25                  30


Leu Ala Trp Tyr Gln Gln Lys Gln Gly Lys Ser Pro Gln Leu Leu Val
        35                  40                  45


Tyr Ala Ala Thr Phe Leu Ala Asp Gly Val Pro Ser Arg Phe Ser Gly
    50                  55                  60


Ser Gly Ser Gly Thr Gln Tyr Ser Leu Lys Ile Asn Ser Leu Gln Ser
65                  70                  75                  80


Glu Asp Phe Gly Asn Tyr Tyr Cys Gln His Phe Trp Gly Thr Pro Pro
                85                  90                  95


Thr Phe Gly Gly Gly Thr Lys Leu Glu Ile Lys
            100                 105


<210>  86
<211>  11
<212>  PRT
<213>  Artificial sequence

<220>
<223>  86

<400>  86

Arg Ala Ser Glu Asp Leu Tyr Ser Asn Leu Ala
1               5                   10


<210>  87
<211>  7
<212>  PRT

<213>  Artificial sequence

<220>
<223>  87

<400>  87

Ala Ala Thr Phe Leu Ala Asp
1               5


<210>  88
<211>  9
<212>  PRT
<213>  Artificial sequence

<220>
<223>  88

<400>  88

Gln His Phe Trp Gly Thr Pro Pro Thr
1               5


<210>  89
<211>  114
<212>  PRT
<213>  Artificial sequence

<220>
<223>  89

<400>  89

Gln Val Gln Leu Gln Gln Pro Gly Ala Glu Leu Val Lys Pro Gly Ala
1               5                   10                  15


Ser Val Lys Met Ser Cys Lys Ala Ser Gly Tyr Thr Phe Thr Arg His
            20                  25                  30


Trp Ile Thr Trp Val Lys Gln Arg Pro Gly Gln Gly Leu Glu Trp Ile
        35                  40                  45


Gly Asp Ile Phe Pro Gly Ser Gly Ser Ile Asn Tyr Asn Glu Lys Phe
    50                  55                  60


Lys Ser Glu Ala Thr Leu Thr Val Glu Lys Ser Ser Ser Thr Ala Tyr
65                  70                  75                  80


Met Gln Leu Ser Ser Leu Thr Ser Glu Asp Ser Ala Val Tyr Tyr Cys
                85                  90                  95


Ala Arg Gly Asp Val Asp Val Trp Gly Thr Gly Thr Thr Val Thr Val
            100                 105                 110

Ser Ser

<210> 90
<211> 7
<212> PRT
<213> Artificial sequence

<220>
<223> 90

<400> 90

Gly Tyr Thr Phe Thr Arg His
1               5

<210> 91
<211> 17
<212> PRT
<213> Artificial sequence

<220>
<223> 91

<400> 91

Asp Ile Phe Pro Gly Ser Gly Ser Ile Asn Tyr Asn Glu Lys Phe Lys
1               5                   10                  15

Ser

<210> 92
<211> 5
<212> PRT
<213> Artificial sequence

<220>
<223> 92

<400> 92

Gly Asp Val Asp Val
1               5

<210> 93
<211> 107
<212> PRT
<213> Artificial sequence

<220>
<223> 93

<400> 93

Asp Ile Gln Met Thr Gln Ser Pro Ser Ser Leu Ser Ala Ser Leu Gly
1               5                   10                  15

Glu Arg Val Ser Leu Thr Cys Arg Ala Ser Gln Glu Ile Arg Gly Tyr
            20                  25                  30

Leu Ser Trp Leu Gln Gln Lys Pro Asn Gly Thr Ile Lys Arg Leu Ile
        35                  40                  45

Tyr Ala Ala Ser Thr Leu Asp Ser Gly Val Pro Lys Arg Phe Ser Ala
    50                  55                  60

Thr Arg Ser Gly Ser Asp Phe Ser Leu Thr Ile Ser Ser Leu Glu Ser
65                  70                  75                  80

Glu Asp Phe Ala Asp Tyr Tyr Cys Leu Gln Tyr Ala Ser Tyr Pro Phe
                85                  90                  95

Thr Phe Gly Ser Gly Thr Lys Leu Glu Ile Lys
            100                 105

<210>  94
<211>  11
<212>  PRT
<213>  Artificial sequence

<220>
<223>  94

<400>  94

Arg Ala Ser Gln Glu Ile Arg Gly Tyr Leu Ser
1               5                   10

<210>  95
<211>  7
<212>  PRT
<213>  Artificial sequence

<220>
<223>  95

<400>  95

Ala Ala Ser Thr Leu Asp Ser
1               5

<210>  96
<211>  9
<212>  PRT
<213>  Artificial sequence

<220>
<223>  96

<400>  96

Leu Gln Tyr Ala Ser Tyr Pro Phe Thr
1               5

<210>  97
<211>  123
<212>  PRT
<213>  Artificial sequence

<220>
<223>  97

<400>  97

Glu Val Gln Leu Val Glu Ser Gly Gly Gly Leu Val Thr Pro Gly Gly
1               5                   10                  15

Ser Leu Lys Leu Ser Cys Val Ala Ser Gly Phe Ala Phe Ser Thr Tyr
            20                  25                  30

Gly Met Ser Trp Val Arg Gln Thr Pro Glu Arg Arg Leu Glu Trp Val
        35                  40                  45

Ala Tyr Ile Ser Gly Gly Gly Gly Ser Ile Tyr Tyr Pro Asp Ser Val
    50                  55                  60

Lys Gly Arg Phe Thr Ile Ser Arg Asp Asn Ala Lys Asn Ile Leu Tyr
65                  70                  75                  80

Leu Gln Met Ser Ser Leu Lys Ser Glu Asp Thr Ala Met Tyr His Cys
                85                  90                  95

Thr Arg His Pro Asp Leu Leu Leu Arg Tyr Asp Tyr Ala Leu Asp His
            100                 105                 110

Trp Gly Gln Gly Thr Ser Val Thr Val Ser Ser
        115                 120

<210>  98
<211>  10
<212>  PRT
<213>  Artificial sequence

<220>
<223>  98

<400>  98

Gly Phe Ala Phe Ser Thr Tyr Gly Met Ser
1               5                   10

<210>  99
<211>  17
<212>  PRT

<213>  Artificial sequence

<220>
<223>  99

<400>  99

Tyr Ile Ser Gly Gly Gly Gly Ser Ile Tyr Tyr Pro Asp Ser Val Lys
1               5                   10                  15

Gly

<210>  100
<211>  14
<212>  PRT
<213>  Artificial sequence

<220>
<223>  100

<400>  100

His Pro Asp Leu Leu Leu Arg Tyr Asp Tyr Ala Leu Asp His
1               5                   10

<210>  101
<211>  107
<212>  PRT
<213>  Artificial sequence

<220>
<223>  101

<400>  101

Asp Ile Gln Met Thr Gln Ser Pro Ala Ser Leu Ser Ala Ser Val Gly
1               5                   10                  15

Glu Thr Val Thr Ile Thr Cys Arg Ala Ser Glu Asn Ile His Ile Tyr
            20                  25                  30

Leu Ala Trp Tyr Gln Gln Lys Gln Gly Lys Ser Pro Gln Leu Leu Val
        35                  40                  45

Tyr Asn Gly Lys Thr Leu Ala Asp Gly Val Pro Ser Arg Phe Ser Gly
    50                  55                  60

Ser Gly Ser Gly Thr Gln Tyr Ser Leu Lys Ile Asn Thr Leu Gln Pro
65                  70                  75                  80

Glu Asp Phe Gly Asn Tyr Tyr Cys Gln His Phe Trp Asn Ser Thr Trp
                85                  90                  95

Thr Phe Gly Gly Gly Thr Lys Leu Glu Ile Lys
            100                 105

<210>  102
<211>  11
<212>  PRT
<213>  Artificial sequence

<220>
<223>  102

<400>  102

Arg Ala Ser Glu Asn Ile His Ile Tyr Leu Ala
1               5                   10

<210>  103
<211>  7
<212>  PRT
<213>  Artificial sequence

<220>
<223>  103

<400>  103

Asn Gly Lys Thr Leu Ala Asp
1               5

<210>  104
<211>  9
<212>  PRT
<213>  Artificial sequence

<220>
<223>  104

<400>  104

Gln His Phe Trp Asn Ser Thr Trp Thr
1               5

<210>  105
<211>  375
<212>  DNA
<213>  Artificial sequence

<220>
<223>  105

<400>  105
gaggtgcagc tggtggagtc ccggggaggc gtggtccagc ctgggaggtc cctgagactc      60

tcctgtgcag cgtctggatt caccttcagt agctatggca tgcactgggt ccgccaggct     120

ccaggcaagg ggctggagtg ggtggcagtt atatcatatg atggaagtaa taaatactat     180

gcagactccg tgaagggccg attcaccatc tccagagaca attccaagaa cacgctgtat     240

ctgcaaatga acagcctgag agctgaggac gcggccgtgt attactgtgc gagagatcgg 300

gtagatattt tgactggtta ttctatctac ggtatggacg tctggggcca agggaccacg 360

gtcaccgtct cctca 375

<210> 106
<211> 321
<212> DNA
<213> Artificial sequence

<220>
<223> 106

<400> 106
gaaattgtga tgactcagtc tccagccacc ctgtctttgt ctccagggga aagagccacc 60

ctctcctgca gggccagtca gagtgttagc agctacttag cctggtacca acagaaacct 120

ggccaggctc ccaggctcct catctatgat gcatccaaga gggccactgg catcccagcc 180

aggttcagtg gcagtgggtc tgggacagac ttcactctca ccatcagcag cctagagcct 240

gaagattttg cagtttatta ctgtcagcag cgtagcaact ggcctctcac tttcggcgga 300

gggaccaagg tggagatcaa a 321

<210> 107
<211> 375
<212> DNA
<213> Artificial sequence

<220>
<223> 107

<400> 107
caaatccagc tggtacaatc tggagctgag gtgaagaagc ctggggcctc agtgaaggtc 60

tcctgcaagg cttctggtta cacctttacc agctatggta tcagctgggt gcgacaggcc 120

cctggacaag ggcttgagtg gatgggatgg atcagcgctt acaatggtaa cacaaactat 180

gcacagaagc tccagggcag agtcaccatg accacagaca catccacgag cacagcctac 240

atggagctga ggagcctgag atctgacgac acggccgtgt attactgtgc gagaacgtat 300

tacgatattt tgactggtta ttactactac ggtatggacg tctggggcca agggaccacg 360

gtcaccgtct cctca 375

<210> 108
<211> 321
<212> DNA
<213> Artificial sequence

<220>
<223> 108

<400> 108
gaaattgtgt tgacgcagtc tccaggcacc ctgtctttgt ctccagggga gagagccacc 60

ctctcctgca ggaccagtca gagtgttgac acctacttgg cctggtacca acagaagcct 120

ggccaggctc ccaggctcct catctatgat gcatccaaca gggccactgg catcccagcc 180

aggttcagtg gcagtgggtc tgggacagac ttcactctca ccatcagcag cctagagcct 240

gaagattttg cagtttatta ctgtcagcag cgtagcaact ggccactcac tttcggcgga 300

gggaccaagg tggagatcaa a 321

<210> 109
<211> 363
<212> DNA
<213> Artificial sequence

<220>
<223> 109

<400> 109
caggtccagc tggtgcagtc tgggggaggc ttggtccagc ctggggggtc cctgagactc 60

tcctgtgcag cctctggatt caccgtcagt agcaactaca tgagctgggt ccgccaggct 120

ccagggaagg ggctggagtg ggtctcagtt atttatagcg gtggtagcac atactacgca 180

gactccgtga agggccgatt caccatctcc agagacaatt ccaagaacac gctgtatctg 240

caaatgaaca gcctgagagc tgaggacacg gctgtgtatt actgtgcgag aggtttccac 300

gatattttga ctggttacgg tatggacgtc tggggccaag ggaccacggt caccgtctcc 360

tca 363

<210> 110
<211> 327
<212> DNA
<213> Artificial sequence

<220>
<223> 110

<400> 110
tcttctgagc tgactcagga ccctgctgtg tctgtggcct tgggacagac agtcaggatc 60

acatgccaag gagacagcct cagaaactat tatgcaggct ggtaccaaca gaagccagga 120

caggcccctg tacttgtcat ctatggaaag aacaaccggc cctcaggaat cccagaccga 180

ttctctggct ccagctcagg aaacacagct tccttgacca tcactgggac tcaggcggaa 240

gatgaggctg actattactg tagctcccgg gacaacagtg gtacccatct cgtggaattc 300

ggcggaggga ccaagttgac cgtccta 327

<210> 111
<211> 354
<212> DNA
<213> Artificial sequence

<220>
<223>  111

<400>  111
gaggttcagc tgcagcagtc tggggcagag cttgtgaagc caggggcctc agtcaagttg      60

tcctgcacag ctgctggctt caacattcaa gacacctata ttcattgggt gaagcagagg     120

cctgagcagg gcctggagtg gattggaagg attgatcctg cgaatgctaa cactgtatat     180

gacccgaagt tccagggcaa ggccactata acagcagaca catcctccaa cacagcctac     240

ctgcaggtca gcagcctgac atctgaggac actgccgtct attactgtgc cagtcccgtt     300

attactgcgg gatttgctta ctggggccaa gggactctgg tcactgtctc tgca           354


<210>  112
<211>  318
<212>  DNA
<213>  Artificial sequence

<220>
<223>  112

<400>  112
caaattgttc tcacccagtc tccagcaatc atgtctgctt ctccagggga gaaggtcacc      60

atgacctgcg gtgccagctc aagtgtaaat tacgtgcact ggtaccagca gaagtcaggg     120

acctccccca aaagatggat ttatgacaca tccaaattgg cttctggagt ccctgctcgc     180

ttcagtggca gtgggtctgg gacctcttac tctctcacaa tcagcagcat ggaggctgaa     240

gatgctgcca cttattactg ccagcagtgg actagatccc cgctcacgtt cggtgctggg     300

accaaggtgg agctgaaa                                                   318


<210>  113
<211>  375
<212>  DNA
<213>  Artificial sequence

<220>
<223>  113

<400>  113
caggttcagc tgcagcagtc tggagttgag ctggcgaggc ctggggcttc agtgaagctg      60

tcctgcaagg cttctggcta caccttcaca agctatggta taagctgggt gaagcagaga     120

actggacagg gccttgagtg gattggagag atttatccta gaagtggtaa tacttactac     180

aatgagaagt tcaagggcaa ggccacactg actgcagaca aatcctccag cacagcgtac     240

atggagctcc gcagcctgac atctgaggac tctgcggtct atttctgtgc aagatcgggg     300

cctattacta cgttagtagc tacggactat ggtatggact actggggtca aggaacctca     360

gtcaccgtct cctca                                                      375

<210>  114
<211>  333
<212>  DNA
<213>  Artificial sequence

<220>
<223>  114

<400>  114
gacattgtgc tgacacagtc tcctgcttcc ttagctgtat ctctggggca gagggccacc      60

atctcctgca gggccagcaa aagtgtcagt acatctagct atagttacat gcactggtac      120

caacagaaac caggacagcc gcccaaactc ctcatcaagt ttgcatccaa cctagaatct      180

ggggtccctg ccaggttcag tggcagtggg tctgggacag acttcaccct caacatccat      240

cctgtggagg aggaggatgc tgcaacatat tactgtcagc acagtaggga gtttccgtac      300

acgttcggag gggggaccaa gctggaaata aaa                                  333


<210>  115
<211>  372
<212>  DNA
<213>  Artificial sequence

<220>
<223>  115

<400>  115
gaggttcagc tgcagcagtc tgtggcagag cttgtgaggc caggggcctc agtcaagttg      60

tcctgcacag cttctgactt caatattaaa aacacctata ttcactgggt gaaacagagg      120

cctgaacagg gcctggagtg gattggaagg attgatcctg tgaatggtaa tactaaatat      180

gccccgaagt tccaggacaa ggccactata attgtagaca catattccaa cacagcctat      240

ctgcagctca gcagcctggc atctgaggac actgccgtct attactgtgc tagatggccc      300

ataactacga ttgtagccac ggactggtac ttcgatgtct ggggcacagg gaccacggtc      360

accgtctcct ca                                                         372


<210>  116
<211>  336
<212>  DNA
<213>  Artificial sequence

<220>
<223>  116

<400>  116
gatgttttga tgacccaaac tcaactcgcc ctgcctgtca ggcttggaga gcaagcctcc      60

atttcttgca gatctagtca gagcattgta aatagtaatg gaaacaccta tttagaatgg      120

tacctgcaga aaccaggcca gtctccaaag ctcctgatct acaaagtttc caaccgattt      180

tctggggtcc cagacaggtt cagtggcagt ggatcaggga cagattttac actcaagatc      240

agcagagtgg aggctgagga tctgggagtt tattactgct ttcaaggttc acatgttccg 300

tggacgttcg gtggaggcac caagctggaa atcaaa 336

<210> 117
<211> 348
<212> DNA
<213> Artificial sequence

<220>
<223> 117

<400> 117
caggtccagt tgcagcagtc tggagctgag ctggtaaggc ctgggacttc agtgaagata 60

tcctgcaagg cttctggata cgccttcact aactactggc taggttgggt aaagcagagg 120

cctggacatg gacttgagtg gattggagat atttaccctg gaagtggtaa tacttactac 180

aatgagaagt tcaagggcaa agccacactg actgcagaca aatcctcgag cacagcctat 240

atgcagctca gtagcctgac atctgaggac tctgctgtct ttttctgtgc agcttacctc 300

tatgctatgg actactgggg tcaaggaacc tcagtcaccg tctcctca 348

<210> 118
<211> 318
<212> DNA
<213> Artificial sequence

<220>
<223> 118

<400> 118
caaattgttc tcacccagtc tccagcaatc atgtctgcat ctccagggga gaaggtcacc 60

ataacctgca gtgccagctc aagtgtaagt tacatgcact ggttccagca gaagccaggc 120

acttctccca aactctggat ttatagcgca tccaacctgg cttctggagt ccctgctcgc 180

ttcagtggca gtggatctgg gacctcttac tctctcacaa tcagccgaat ggaggctgaa 240

gatgctgcca cttattactg ccagcaaagg ggtagttacc cattcacgtt cggctcgggg 300

acaaagttgg aaataaaa 318

<210> 119
<211> 360
<212> DNA
<213> Artificial sequence

<220>
<223> 119

<400> 119
caggtgcaat tgaaggagtc aggacctggc ctggtggcgc cctcgcagag cctgtccatt 60

acctgcactg tctctgggct ctcattaaac agccatgata taagctggat tcgccagcca 120

ccaggaaagg gtctggagtg gcttggagta atatggagtg gtggaggcac aaaatataat 180

tcagctttca tgtccagact gagcatcagc aaggacaact ccaagagcca agtgttctta 240

aaaatgaaca gtctgcacac tgatgacaca gccatatatt actgtgtaag aaccccctct 300

atgattactt actctgctat ggactactgg ggtcaaggaa cctcagtcac cgtctcctca 360

<210> 120
<211> 324
<212> DNA
<213> Artificial sequence

<220>
<223> 120

<400> 120
gaaattgtac tcacccagtc tccagcactc atggctgcat ctccagggga gaaggtcacc 60

atcacctgca gtgtcagctc aagtataagt tcccgcaact tacactggta ccagcagaag 120

tcacaaacct cccccaaagc ctggattcat gccacatcca acctggcttc tggagtccct 180

gttcgcttca gtggcagtgg atctgggacc tcttattctc tcacaatcag cagcatggag 240

gctgaagatg ctgccactta ttactgtcaa cagtggagta gttacccact cacgttcggc 300

tcggggacaa agttggaaat aaaa 324

<210> 121
<211> 354
<212> DNA
<213> Artificial sequence

<220>
<223> 121

<400> 121
gaggtccagc ttcagcagtc aggacctgag ctggtgaaat ccggggcctc agtgaagata 60

tcctgcaagg cttctggata cacattcact gaccacaaaa tgcactgggt gaagcagagc 120

catggaaaga gccttgagtg gattggatat atttctcctt acaatggtgg cactggctac 180

aatcagaagt tcaagagcaa ggtcacattg actgtagaca tatcctccag tacagcctat 240

atggaactcc gcagcctgac atctgaagac tctgcagtct attactgtgc aagatggggt 300

ttctacgatg taatggactc ctggggtcaa gggacctcag tcaccgtctc ctca 354

<210> 122
<211> 336
<212> DNA
<213> Artificial sequence

<220>
<223> 122

<400> 122
gatgttttgt tgacccaaac tccactctcc ctgcctgtca gtcttggaga tcaagcctcc 60

atctcttgca gatctagtca gaccattgtt catattattg gagacaccta tttagagtgg 120

tacctgcaga aaccaggcca gtctccaaag ctcctgatct acaaagtttc caaccgattt 180

tctggggtcc cagacaggtt cagtggcagt ggagcaggga cagatttcac actcaagatc 240

agcagagtgg aggctgagga tctgggagtt tattactgct ttcaaggttc acatgttcct 300

cggacgttcg gtggaggcac caagctggaa atcaaa 336

<210> 123
<211> 360
<212> DNA
<213> Artificial sequence

<220>
<223> 123

<400> 123
gaagtgaaac tggtggagtc tgggggaggt ttagtgcagc ctggagggtc cctgaaactc 60

tcctgtgcag cctctggatt cacttttagt acttatacca tgtcttgggt tcgccagact 120

ccagagaaga ggctggagtg ggtcgcatac attagttatg gtggtggtag cacctcctat 180

ccagacactg taaagggccg attcaccatc tccagagaca atgccaagaa caccctttac 240

ctacaaatga gcagtctgaa gtctgaggac acggccattt attattgtac aagacacgtt 300

acggtagttg agaattctat ggactactgg ggtcaaggaa cctcagtcac cgtctcctca 360

<210> 124
<211> 321
<212> DNA
<213> Artificial sequence

<220>
<223> 124

<400> 124
gacatcttgc tgactcagtc tccagacatc ctgtctgtga gtccaggaga aagagtcagt 60

ttctcctgca gggccagcca gagcattggc acaagcatac actggtatca gcaaagaacg 120

aatggttctc caaggcttct cataaaatat gtttctgagt ctttctctgg gaccccttcc 180

agatttagtg gcagtggatc agggacagct tttactctta ccatcaacag tgtggagtct 240

gaagatattg cagattatta ctgtcaacaa actcatagct ggccgtggac gttcggtgga 300

ggcaccaagc tggagatcaa a 321

<210> 125
<211> 366
<212> DNA
<213> Artificial sequence

<220>
<223> 125

<400> 125
gaggttcagc tgcagcagtc tggggcagag cttgtgaagc caggggcctc agtcaagttg 60

tcctgcacag cttctggctt caacattaaa aactactata tgcactgggt gaagcagagg 120

actgaagagg gcctggagtg gattggaagg attgatcctg aggatggtga aactaaatat 180

gccccgaaat tccaggccaa ggccactata acagcagaca catcctccaa cacagcctac 240

ctgcagctca gcagcctgac atctgaggac actgccgtct atttctgtgc tagaaagggc 300

tactatggta actacggggc ctggtttgct tactggggcc aagggactct ggtcactgtc 360

tctgca 366


<210> 126
<211> 321
<212> DNA
<213> Artificial sequence

<220>
<223> 126

<400> 126
gacatccaga tgactcagtc tccagcctcc ctatctgtat ctgtgggaga aactgtcacc 60

atcacatgtc gagcaagtga agatctttac agtaatttag catggtatca gcagaaacag 120

ggaaaatctc ctcagctcct ggtctatgct gcaacattct tagcagatgg tgtgccatca 180

aggttcagtg gcagtggatc aggcacacag tattccctca agatcaacag cctgcagtct 240

gaagattttg ggaattatta ctgtcaacat ttttggggta ctcctccgac gttcggtgga 300

ggcaccaagc tggaaatcaa a 321


<210> 127
<211> 342
<212> DNA
<213> Artificial sequence

<220>
<223> 127

<400> 127
caggtccaac tgcagcagcc tggggctgag cttgtgaagc ctggggcttc agtgaagatg 60

tcctgcaagg cttctggcta caccttcacc aggcactgga taacctgggt gaagcagagg 120

cctggacaag gccttgagtg gattggagat atttttcctg gtagtggtag tattaactac 180

aatgagaagt tcaagagcga ggccacactg actgtagaaa aatcctccag cacagcctac 240

atgcagctca gcagcctgac atctgaggac tctgcggtct attactgtgc aagaggggac 300

gtcgatgtct ggggcacagg gaccacggtc accgtctcct ca 342


<210> 128
<211> 321
<212> DNA

<213>  Artificial sequence

<220>
<223>  128

<400>  128
gacatccaga tgacccagtc tccatcgtcc ttatctgcct ctctgggaga aagagtcagt      60

ctcacttgtc gggcaagtca ggaaattcgt ggttacttaa gctggcttca gcagaaacca     120

aatggaacta ttaaacgcct gatctacgcc gcatccactt tagattctgg tgtcccaaaa     180

aggttcagtg ccactaggtc tgggtcagat ttttctctca ccatcagcag ccttgagtct     240

gaagattttg cagactatta ctgtctccaa tatgctagtt atccattcac gttcggctcg     300

gggacaaagt tggaaataaa a                                               321


<210>  129
<211>  369
<212>  DNA
<213>  Artificial sequence

<220>
<223>  129

<400>  129
gaagtgcagt tggtggagtc tgggggaggc ttagtgacgc ctggagggtc cctgaaactc      60

tcctgtgtcg cctctggatt cgctttcagt acctatggca tgtcttgggt tcgccagact     120

ccggagagga ggctggagtg ggtcgcatat attagtggtg gtggtggaag tatctactat     180

ccagacagtg tgaagggccg attcaccatc tccagagaca atgccaagaa catcctgtac     240

cttcaaatga gcagtctgaa gtctgaggac acagccatgt atcactgtac cagacacccc     300

gatttattac tacggtatga ttatgcattg gaccactggg gtcaaggaac ctcagtcacc     360

gtctcctca                                                             369


<210>  130
<211>  321
<212>  DNA
<213>  Artificial sequence

<220>
<223>  130

<400>  130
gacatccaga tgactcagtc tccagcctcc ctatctgcat ctgtgggaga aactgtcacc      60

atcacatgtc gagcaagtga aaatattcac atttatttag catggtatca gcagaaacag     120

ggaaaatctc ctcagctcct ggtctataat ggaaaaacct tagcagatgg tgtgccatca     180

aggttcagtg gcagtggatc aggaacacaa tattctctca agatcaatac cctgcagcct     240

gaagattttg ggaattatta ctgtcaacat ttttggaata gtacgtggac gttcggtgga     300

ggcaccaagc tggaaatcaa a                                               321

<210>  131
<211>  456
<212>  DNA
<213>  Homo sapiens

<400>  131
gccgtccagg gccccgagga gaccgtgacc caggactgtc tccagctcat cgctgactcc      60

gaaaccccca ccatccagaa gggcagctac accttcgtgc cttggctgct gagcttcaag     120

agaggcagcg ccctggagga gaaggagaac aagattctcg tgaaggagac cggctacttc     180

ttcatctacg gccaggtgct ctacaccgac aagacctatg ccatgggaca cctcatccag     240

aggaagaagg tccacgtctt cggcgacgag ctcagcctgg tcaccctgtt caggtgcatc     300

cagaatatgc ccgagaccct gcccaataac agctgctaca gcgctggcat tgccaaactg     360

gaggagggcg acgaactgca gctcgccatc cccagggaga acgcccagat cagcctcgac     420

ggcgatgtga ccttcttcgg cgccctcaag ctcctg                               456


<210>  132
<211>  858
<212>  DNA
<213>  Homo sapiens

<400>  132
atggacgact ctacagagcg cgagcagtct aggctcacat catgtctcaa gaagcgggaa      60

gagatgaagc tcaaagaatg cgtcagcatc ctgcctagaa aggaatcacc ttccgtccgg     120

tcttccaagg atggaaagct gctcgccgct acactgctcc tcgcactcct gtcttgttgc     180

ctcacagtcg tgtcatttta ccaggtggcc gcactacagg gcgatctggc atcactgcgc     240

gcagagctac aaggccacca cgctgagaag ctcccagccg gggctggggc accaaaggcc     300

ggactcgaag aggcacccgc agtgaccgcc ggactgaaaa tttttgagcc acccgccccc     360

ggcgagggca attctagcca gaactcacac aacaagagag cagtgcaggg ccccgaggaa     420

accgtgacac aggattgtct ccagctgatt gccgatagcg agaccccaac tattcagaag     480

ggatcataca catttgtgcc ttggctcctg agcttcaagc gcgggtccgc cttagaggaa     540

aaggaaaata agattctggt caaagaaacc gggtacttct ttatctacgg acaggtcctg     600

tacaccgata agacatacgc tatgggacac ctgatccagc ggaagaaggt gcacgtcttc     660

ggcgatgagc tatctctggt gactctgttt cggtgtattc agaatatgcc tgagaccctc     720

cctaacaatt catgttactc cgctggaatt gctaagctcg aagaggggga tgaactacag     780

ctggctatcc cacgcgagaa cgcacagatt agcctcgacg gcgacgtgac tttcttcggc     840

gccctcaagc tgctgtga                                                   858


<210>  133

<211>  858
<212>  DNA
<213>  Macaca fascicularis

<400>  133
atggacgact ctacagagcg cgagcagtca cggctgacat catgtctcaa gaagagagaa      60

gagatgaagc tgaaagaatg cgtctcaatt ctgccacaga aggaatcccc atccgtgcgg     120

tctagcaagg atagaaagct cctcgcagcc gccctgctgc tcgccctgct gtcatgttgc     180

ctgatggtcg tgtccttcta ccaggtggcc gctctccagg gcgatctggc atcactgcgc     240

gccgagttgc agtcccacca cgcagagaag ctcccagccc gagcacgggc cccaaaggca     300

ggcctcggcg aggcacccgc agtgaccgcc ggactcaaaa tctttgagcc acccgcacct     360

ggcgagggaa attctagcca gtcttcacac aataagcgcg ctatccaggg ggccgacgag     420

accgtgattc aggattgtct ccagctgatt gccgatagcg agaccccaac tattcagaag     480

ggatcataca catttgtgcc ttggctcctg agcttcaagc gcgggtccgc cttagaggaa     540

aaggaaaata agattctggt caaagaaacc gggtacttct ttatctacgg acaggtcctg     600

tacaccgata agacatacgc tatgggacac ctgatccagc ggaagaaggt gcacgtcttc     660

ggcgatgagc tatctctggt gactctgttt cggtgtattc agaatatgcc tgagaccctc     720

cctaacaatt catgttactc cgctggaatt gctaagctcg aagaggggga tgaactacag     780

ctggctatcc cacgcgagaa cgcacagatt agcctcgacg gcgacgtgac tttcttcggc     840

gccctcaagc tgctgtga                                                   858


<210>  134
<211>  18
<212>  DNA
<213>  Artificial sequence

<220>
<223>  pTT-EF1a-F

<400>  134
gccctttttg agtttgga                                                    18


<210>  135
<211>  18
<212>  DNA
<213>  Artificial sequence

<220>
<223>  pSV40

<400>  135
cactgcattc tagttgtg                                                    18


<210>  136
<211>  109
<212>  PRT

<213>  Homo sapiens

<400>  136

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Met
        35                  40                  45

Gly Trp Ile Ser Ala Tyr Asn Gly Asn Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Trp Gly Gln Gly Thr Thr Val Thr Val Ser Ser
            100                 105


<210>  137
<211>  104
<212>  PRT
<213>  Homo sapiens

<400>  137

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Val Leu Tyr Ser
            20                  25                  30

Ser Asn Asn Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Phe Gly
                85                  90                  95

Gly Gly Thr Lys Val Glu Ile Lys
            100

<210>  138
<211>  109
<212>  PRT
<213>  Homo sapiens

<400>  138

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Trp Gly Gln Gly Thr Thr Val Thr Val Ser Ser
            100                 105

<210>  139
<211>  98
<212>  PRT
<213>  Homo sapiens

<400>  139

Glu Ile Val Leu Thr Gln Ser Pro Asp Phe Gln Ser Val Thr Pro Lys
1               5                   10                  15

Glu Lys Val Thr Ile Thr Cys Arg Ala Ser Gln Ser Ile Gly Ser Ser
            20                  25                  30

Leu His Trp Tyr Gln Gln Lys Pro Asp Gln Ser Pro Lys Leu Leu Ile
        35                  40                  45

Lys Tyr Ala Ser Gln Ser Phe Ser Gly Val Pro Ser Arg Phe Ser Gly
    50                  55                  60

Ser Gly Ser Gly Thr Asp Phe Thr Leu Thr Ile Asn Ser Leu Glu Ala

65                  70                  75                  80

Glu Asp Ala Ala Thr Tyr Tyr Cys Phe Gly Gly Gly Thr Lys Val Glu
                85                  90                  95

Ile Lys

<210>  140
<211>  125
<212>  PRT
<213>  Artificial sequence

<220>
<223>  140

<400>  140

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Met
        35                  40                  45

Gly Glu Ile Tyr Pro Arg Ser Gly Asn Thr Tyr Tyr Asn Glu Lys Phe
    50                  55                  60

Lys Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Ser Gly Pro Ile Thr Thr Leu Val Ala Thr Asp Tyr Gly Met
            100                 105                 110

Asp Tyr Trp Gly Gln Gly Thr Thr Val Thr Val Ser Ser
        115                 120                 125

<210>  141
<211>  125
<212>  PRT
<213>  Artificial sequence

<220>
<223>  141

<400>  141

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Met
        35                  40                  45

Gly Glu Ile Tyr Pro Arg Ser Gly Asn Thr Tyr Tyr Asn Glu Lys Phe
    50                  55                  60

Lys Gly Arg Val Thr Met Thr Ala Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Ser Gly Pro Ile Thr Thr Leu Val Ala Thr Asp Tyr Gly Met
            100                 105                 110

Asp Tyr Trp Gly Gln Gly Thr Thr Val Thr Val Ser Ser
        115                 120                 125

<210>  142
<211>  125
<212>  PRT
<213>  Artificial sequence

<220>
<223>  142

<400>  142

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Met
        35                  40                  45

Gly Glu Ile Tyr Pro Arg Ser Gly Asn Thr Tyr Tyr Asn Glu Lys Phe
    50                  55                  60

Lys Gly Arg Val Thr Leu Thr Ala Asp Thr Ser Thr Ser Thr Ala Tyr
65                  70                  75                  80

Met Glu Leu Arg Ser Leu Arg Ser Asp Asp Thr Ala Val Tyr Tyr Cys

85                  90                  95

Ala Arg Ser Gly Pro Ile Thr Thr Leu Val Ala Thr Asp Tyr Gly Met
            100                 105                 110

Asp Tyr Trp Gly Gln Gly Thr Thr Val Thr Val Ser Ser
        115                 120                 125

<210>  143
<211>  125
<212>  PRT
<213>  Artificial sequence

<220>
<223>  143

<400>  143

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Ile
        35                  40                  45

Gly Glu Ile Tyr Pro Arg Ser Gly Asn Thr Tyr Tyr Asn Glu Lys Phe
    50                  55                  60

Lys Gly Arg Val Thr Leu Thr Ala Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Ser Gly Pro Ile Thr Thr Leu Val Ala Thr Asp Tyr Gly Met
            100                 105                 110

Asp Tyr Trp Gly Gln Gly Thr Thr Val Thr Val Ser Ser
        115                 120                 125

<210>  144
<211>  125
<212>  PRT
<213>  Artificial sequence

<220>
<223>  144

<400>  144

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Lys Gln Ala Pro Gly Gln Gly Leu Glu Trp Ile
        35                  40                  45

Gly Glu Ile Tyr Pro Arg Ser Gly Asn Thr Tyr Tyr Asn Glu Lys Phe
    50                  55                  60

Lys Gly Arg Val Thr Leu Thr Ala Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Ser Gly Pro Ile Thr Thr Leu Val Ala Thr Asp Tyr Gly Met
            100                 105                 110

Asp Tyr Trp Gly Gln Gly Thr Thr Val Thr Val Ser Ser
        115                 120                 125

<210>  145
<211>  125
<212>  PRT
<213>  Artificial sequence

<220>
<223>  145

<400>  145

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Lys Gln Ala Pro Gly Gln Gly Leu Glu Trp Ile
        35                  40                  45

Gly Glu Ile Tyr Pro Arg Ser Gly Asn Thr Tyr Tyr Asn Glu Lys Phe
    50                  55                  60

Lys Gly Lys Ala Thr Leu Thr Ala Asp Thr Ser Thr Ser Thr Ala Tyr
65                  70                  75                  80

Met Glu Leu Arg Ser Leu Arg Ser Asp Asp Thr Ala Val Tyr Tyr Cys

85                  90                  95

Ala Arg Ser Gly Pro Ile Thr Thr Leu Val Ala Thr Asp Tyr Gly Met
            100                 105                 110

Asp Tyr Trp Gly Gln Gly Thr Thr Val Thr Val Ser Ser
        115                 120                 125

<210>  146
<211>  125
<212>  PRT
<213>  Artificial sequence

<220>
<223>  146

<400>  146

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Lys Gln Ala Pro Gly Gln Ala Leu Glu Trp Ile
        35                  40                  45

Gly Glu Ile Tyr Pro Arg Ser Gly Asn Thr Tyr Tyr Asn Glu Lys Phe
    50                  55                  60

Lys Gly Lys Ala Thr Leu Thr Ala Asp Lys Ser Thr Ser Thr Ala Tyr
65                  70                  75                  80

Met Glu Leu Arg Ser Leu Arg Ser Asp Asp Thr Ala Val Tyr Phe Cys
                85                  90                  95

Ala Arg Ser Gly Pro Ile Thr Thr Leu Val Ala Thr Asp Tyr Gly Met
            100                 105                 110

Asp Tyr Trp Gly Gln Gly Thr Thr Val Thr Val Ser Ser
        115                 120                 125

<210>  147
<211>  111
<212>  PRT
<213>  Artificial sequence

<220>
<223>  147

<400>  147

Asp Ile Val Met Thr Gln Ser Pro Asp Ser Leu Ala Val Ser Leu Gly
1               5                   10                  15

Glu Arg Ala Thr Ile Asn Cys Arg Ala Ser Lys Ser Val Ser Thr Ser
            20                  25                  30

Ser Tyr Ser Tyr Met His Trp Tyr Gln Gln Lys Pro Gly Gln Pro Pro
        35                  40                  45

Lys Leu Leu Ile Lys Phe Ala Ser Asn Leu Glu Ser Gly Val Pro Asp
    50                  55                  60

Arg Phe Ser Gly Ser Gly Ser Gly Thr Asp Phe Thr Leu Thr Ile Ser
65                  70                  75                  80

Ser Leu Gln Ala Glu Asp Val Ala Val Tyr Tyr Cys Gln His Ser Arg
                85                  90                  95

Glu Phe Pro Tyr Thr Phe Gly Gly Gly Thr Lys Val Glu Ile Lys
            100                 105                 110

<210>  148
<211>  111
<212>  PRT
<213>  Artificial sequence

<220>
<223>  148

<400>  148

Asp Ile Val Leu Thr Gln Ser Pro Asp Ser Leu Ala Val Ser Leu Gly
1               5                   10                  15

Glu Arg Ala Thr Ile Asn Cys Arg Ala Ser Lys Ser Val Ser Thr Ser
            20                  25                  30

Ser Tyr Ser Tyr Met His Trp Tyr Gln Gln Lys Pro Gly Gln Pro Pro
        35                  40                  45

Lys Leu Leu Ile Lys Phe Ala Ser Asn Leu Glu Ser Gly Val Pro Asp
    50                  55                  60

Arg Phe Ser Gly Ser Gly Ser Gly Thr Asp Phe Thr Leu Thr Ile Ser
65                  70                  75                  80

Ser Leu Gln Ala Glu Asp Val Ala Val Tyr Tyr Cys Gln His Ser Arg
                85                  90                  95

Glu Phe Pro Tyr Thr Phe Gly Gly Gly Thr Lys Val Glu Ile Lys

            100                 105                 110

<210> 149
<211> 120
<212> PRT
<213> Artificial sequence

<220>
<223> 149

<400> 149

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Thr Met Ser Trp Val Arg Gln Ala Pro Gly Lys Gly Leu Glu Trp Val
        35                  40                  45

Ala Tyr Ile Ser Tyr Gly Gly Gly Ser Thr Ser Tyr Pro Asp Thr Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg His Val Thr Val Val Glu Asn Ser Met Asp Tyr Trp Gly Gln
            100                 105                 110

Gly Thr Thr Val Thr Val Ser Ser
        115                 120

<210> 150
<211> 120
<212> PRT
<213> Artificial sequence

<220>
<223> 150

<400> 150

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Thr Met Ser Trp Val Arg Gln Ala Pro Gly Lys Gly Leu Glu Trp Val
        35                  40                  45

Ala Tyr Ile Ser Tyr Gly Gly Gly Ser Thr Ser Tyr Pro Asp Thr Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Thr Arg His Val Thr Val Val Glu Asn Ser Met Asp Tyr Trp Gly Gln
            100                 105                 110

Gly Thr Thr Val Thr Val Ser Ser
        115                 120

<210>  151
<211>  120
<212>  PRT
<213>  Artificial sequence

<220>
<223>  151

<400>  151

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Thr Met Ser Trp Val Arg Gln Ala Pro Gly Lys Arg Leu Glu Trp Val
        35                  40                  45

Ala Tyr Ile Ser Tyr Gly Gly Gly Ser Thr Ser Tyr Pro Asp Thr Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Thr Arg His Val Thr Val Val Glu Asn Ser Met Asp Tyr Trp Gly Gln
            100                 105                 110

Gly Thr Thr Val Thr Val Ser Ser

115                 120

<210>  152
<211>  120
<212>  PRT
<213>  Artificial sequence

<220>
<223>  152

<400>  152

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Thr Met Ser Trp Val Arg Gln Ala Pro Glu Lys Arg Leu Glu Trp Val
        35                  40                  45

Ala Tyr Ile Ser Tyr Gly Gly Gly Ser Thr Ser Tyr Pro Asp Thr Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Thr Arg His Val Thr Val Val Glu Asn Ser Met Asp Tyr Trp Gly Gln
            100                 105                 110

Gly Thr Thr Val Thr Val Ser Ser
        115                 120

<210>  153
<211>  107
<212>  PRT
<213>  Artificial sequence

<220>
<223>  153

<400>  153

Glu Ile Val Leu Thr Gln Ser Pro Asp Phe Gln Ser Val Thr Pro Lys
1               5                   10                  15

Glu Lys Val Thr Ile Thr Cys Arg Ala Ser Gln Ser Ile Gly Thr Ser
            20                  25                  30

Ile His Trp Tyr Gln Gln Lys Pro Asp Gln Ser Pro Lys Leu Leu Ile
        35                  40                  45

Lys Tyr Val Ser Glu Ser Phe Ser Gly Val Pro Ser Arg Phe Ser Gly
    50                  55                  60

Ser Gly Ser Gly Thr Asp Phe Thr Leu Thr Ile Asn Ser Leu Glu Ala
65                  70                  75                  80

Glu Asp Ala Ala Thr Tyr Tyr Cys Gln Gln Thr His Ser Trp Pro Trp
                85                  90                  95

Thr Phe Gly Gly Gly Thr Lys Val Glu Ile Lys
            100                 105

<210>  154
<211>  107
<212>  PRT
<213>  Artificial sequence

<220>
<223>  154

<400>  154

Glu Ile Val Leu Thr Gln Ser Pro Asp Phe Gln Ser Val Thr Pro Lys
1               5                   10                  15

Glu Lys Val Thr Ile Thr Cys Arg Ala Ser Gln Ser Ile Gly Thr Ser
            20                  25                  30

Ile His Trp Tyr Gln Gln Lys Pro Asp Gln Ser Pro Lys Leu Leu Ile
        35                  40                  45

Lys Tyr Val Ser Glu Ser Phe Ser Gly Thr Pro Ser Arg Phe Ser Gly
    50                  55                  60

Ser Gly Ser Gly Thr Asp Phe Thr Leu Thr Ile Asn Ser Leu Glu Ala
65                  70                  75                  80

Glu Asp Ala Ala Thr Tyr Tyr Cys Gln Gln Thr His Ser Trp Pro Trp
                85                  90                  95

Thr Phe Gly Gly Gly Thr Lys Val Glu Ile Lys
            100                 105

<210>  155
<211>  107
<212>  PRT
<213>  Artificial sequence

<220>
<223>  155

<400>  155

Asp Ile Leu Leu Thr Gln Ser Pro Asp Phe Gln Ser Val Thr Pro Lys
1               5                   10                  15

Glu Lys Val Thr Ile Thr Cys Arg Ala Ser Gln Ser Ile Gly Thr Ser
            20                  25                  30

Ile His Trp Tyr Gln Gln Lys Pro Asp Gln Ser Pro Lys Leu Leu Ile
        35                  40                  45

Lys Tyr Val Ser Glu Ser Phe Ser Gly Thr Pro Ser Arg Phe Ser Gly
    50                  55                  60

Ser Gly Ser Gly Thr Asp Phe Thr Leu Thr Ile Asn Ser Leu Glu Ala
65                  70                  75                  80

Glu Asp Ala Ala Thr Tyr Tyr Cys Gln Gln Thr His Ser Trp Pro Trp
                85                  90                  95

Thr Phe Gly Gly Gly Thr Lys Val Glu Ile Lys
            100                 105


<210>  156
<211>  107
<212>  PRT
<213>  Artificial sequence

<220>
<223>  156

<400>  156

Glu Ile Val Leu Thr Gln Ser Pro Asp Phe Gln Ser Val Thr Pro Lys
1               5                   10                  15

Glu Lys Val Thr Ile Thr Cys Arg Ala Ser Gln Ser Ile Gly Thr Ser
            20                  25                  30

Ile His Trp Tyr Gln Gln Lys Thr Asp Gln Ser Pro Lys Leu Leu Ile
        35                  40                  45

Lys Tyr Val Ser Glu Ser Phe Ser Gly Thr Pro Ser Arg Phe Ser Gly
    50                  55                  60

Ser Gly Ser Gly Thr Asp Phe Thr Leu Thr Ile Asn Ser Leu Glu Ala
65                  70                  75                  80

Glu Asp Ala Ala Thr Tyr Tyr Cys Gln Gln Thr His Ser Trp Pro Trp
                85                  90                  95

Thr Phe Gly Gly Gly Thr Lys Val Glu Ile Lys
            100                 105

<210>  157
<211>  107
<212>  PRT
<213>  Artificial sequence

<220>
<223>  157

<400>  157

Asp Ile Leu Leu Thr Gln Ser Pro Asp Phe Gln Ser Val Thr Pro Lys
1               5                   10                  15

Glu Lys Val Thr Ile Thr Cys Arg Ala Ser Gln Ser Ile Gly Thr Ser
            20                  25                  30

Ile His Trp Tyr Gln Gln Lys Thr Asp Gln Ser Pro Lys Leu Leu Ile
        35                  40                  45

Lys Tyr Val Ser Glu Ser Phe Ser Gly Thr Pro Ser Arg Phe Ser Gly
    50                  55                  60

Ser Gly Ser Gly Thr Asp Phe Thr Leu Thr Ile Asn Ser Leu Glu Ala
65                  70                  75                  80

Glu Asp Ala Ala Thr Tyr Tyr Cys Gln Gln Thr His Ser Trp Pro Trp
                85                  90                  95

Thr Phe Gly Gly Gly Thr Lys Val Glu Ile Lys
            100                 105

<210>  158
<211>  375
<212>  DNA
<213>  Artificial sequence

<220>
<223>  158

<400>  158
gaagtgcagc tggtgcagag cggagcagaa gtgaagaagc caggcgcaag cgtgaaggtg      60

tcttgcaagg ccagcggcta caccttcacc agctacggca tctcttgggt gcggcaggcc     120

ccaggacagg gactcgagtg gatgggagaa atctaccccc ggagcggcaa cacctactac     180

aacgagaagt tcaagggccg cgtgaccatg acaaccgata caagcaccag caccgcctac     240

atggagctga gaagcctgag gagcgacgac acagccgtgt actattgcgc caggagcgga 300

cctatcacca cactggtggc caccgactac ggcatggact attggggcca gggaaccaca 360

gtgaccgtgt ctagc 375

<210> 159
<211> 375
<212> DNA
<213> Artificial sequence

<220>
<223> 159

<400> 159
gaagtgcagc tggtgcagag cggagcagaa gtgaagaagc caggcgcaag cgtgaaggtg 60

tcttgcaagg ccagcggcta caccttcacc agctacggca tctcttgggt gcggcaggcc 120

ccaggacagg gactcgagtg gatgggagaa atctaccccc ggagcggcaa cacctactac 180

aacgagaagt tcaagggccg cgtgaccatg acagccgata caagcaccag caccgcctac 240

atggagctga gaagcctgag gagcgacgac acagccgtgt actattgcgc caggagcgga 300

cctatcacca cactggtggc caccgactac ggcatggact attggggcca gggaaccaca 360

gtgaccgtgt ctagc 375

<210> 160
<211> 375
<212> DNA
<213> Artificial sequence

<220>
<223> 160

<400> 160
gaagtgcagc tggtgcagag cggagcagaa gtgaagaagc caggcgcaag cgtgaaggtg 60

tcttgcaagg ccagcggcta caccttcacc agctacggca tctcttgggt gcggcaggcc 120

ccaggacagg gactcgagtg gatgggagaa atctaccccc ggagcggcaa cacctactac 180

aacgagaagt tcaagggccg cgtgaccctg acagccgata caagcaccag caccgcctac 240

atggagctga gaagcctgag gagcgacgac acagccgtgt actattgcgc caggagcgga 300

cctatcacca cactggtggc caccgactac ggcatggact attggggcca gggaaccaca 360

gtgaccgtgt ctagc 375

<210> 161
<211> 375
<212> DNA
<213> Artificial sequence

<220>
<223> 161

<400>  161
gaagtgcagc tggtgcagag cggagcagaa gtgaagaagc caggcgcaag cgtgaaggtg      60

tcttgcaagg ccagcggcta caccttcacc agctacggca tctcttgggt gcggcaggcc     120

ccaggacagg gactcgagtg gatcggagaa atctaccccc ggagcggcaa cacctactac     180

aacgagaagt tcaagggccg cgtgaccctg acagccgata caagcaccag caccgcctac     240

atggagctga gaagcctgag gagcgacgac acagccgtgt actattgcgc caggagcgga     300

cctatcacca cactggtggc caccgactac ggcatggact attggggcca gggaaccaca     360

gtgaccgtgt ctagc                                                      375


<210>  162
<211>  375
<212>  DNA
<213>  Artificial sequence

<220>
<223>  162

<400>  162
gaagtgcagc tggtgcagag cggagcagaa gtgaagaagc caggcgcaag cgtgaaggtg      60

tcttgcaagg ccagcggcta caccttcacc agctacggca tctcttgggt gaagcaggcc     120

ccaggacagg gactcgagtg gatcggagaa atctaccccc ggagcggcaa cacctactac     180

aacgagaagt tcaagggccg cgtgaccctg acagccgata caagcaccag caccgcctac     240

atggagctga gaagcctgag gagcgacgac acagccgtgt actattgcgc caggagcgga     300

cctatcacca cactggtggc caccgactac ggcatggact attggggcca gggaaccaca     360

gtgaccgtgt ctagc                                                      375


<210>  163
<211>  375
<212>  DNA
<213>  Artificial sequence

<220>
<223>  163

<400>  163
gaagtgcagc tggtgcagag cggagcagaa gtgaagaagc caggcgcaag cgtgaaggtg      60

tcttgcaagg ccagcggcta caccttcacc agctacggca tctcttgggt gaagcaggcc     120

ccaggacagg gactcgagtg gatcggagaa atctaccccc ggagcggcaa cacctactac     180

aacgagaagt tcaagggcaa ggccaccctg acagccgata caagcaccag caccgcctac     240

atggagctga gaagcctgag gagcgacgac acagccgtgt actattgcgc caggagcgga     300

cctatcacca cactggtggc caccgactac ggcatggact attggggcca gggaaccaca     360

gtgaccgtgt ctagc                                                      375

<210>  164
<211>  375
<212>  DNA
<213>  Artificial sequence

<220>
<223>  164

<400>  164
gaagtgcagc tggtgcagag cggagcagaa gtgaagaagc caggcgcaag cgtgaaggtg      60

tcttgcaagg ccagcggcta caccttcacc agctacggca tctcttgggt gaagcaggcc     120

ccaggacagg cactcgagtg gatcggagaa atctaccccc ggagcggcaa cacctactac     180

aacgagaagt tcaagggcaa ggccaccctg acagccgata agagcaccag caccgcctac     240

atggagctga gaagcctgag gagcgacgac acagccgtgt acttttgcgc caggagcgga     300

cctatcacca cactggtggc caccgactac ggcatggact attggggcca gggaaccaca     360

gtgaccgtgt ctagc                                                      375


<210>  165
<211>  333
<212>  DNA
<213>  Artificial sequence

<220>
<223>  165

<400>  165
gacatcgtga tgacccagag cccagacagc ctggcagtgt ctctgggaga gagagccaca      60

atcaattgcc gggccagcaa gagcgtgtcc accagcagct acagctacat gcattggtat     120

cagcagaaac caggccagcc tcctaagctg ctgatcaagt tcgccagcaa cctggagagc     180

ggcgtgccag atagattcag cggaagcgga agcggcaccg atttcaccct gaccatcagc     240

tctctgcagg ccgaagacgt ggccgtgtat tattgccagc acagccggga gttcccctac     300

acatttggcg gcggcaccaa ggtggagatc aag                                  333


<210>  166
<211>  333
<212>  DNA
<213>  Artificial sequence

<220>
<223>  166

<400>  166
gacatcgtgc tgacccagag cccagacagc ctggcagtgt ctctgggaga gagagccaca      60

atcaattgcc gggccagcaa gagcgtgtcc accagcagct acagctacat gcattggtat     120

cagcagaaac caggccagcc tcctaagctg ctgatcaagt tcgccagcaa cctggagagc     180

ggcgtgccag atagattcag cggaagcgga agcggcaccg atttcaccct gaccatcagc     240

tctctgcagg ccgaagacgt ggccgtgtat tattgccagc acagccggga gttcccctac 300

acatttggcg gcggcaccaa ggtggagatc aag 333

<210> 167
<211> 360
<212> DNA
<213> Artificial sequence

<220>
<223> 167

<400> 167
gaggtgcagc tggtggaatc aggaggagga ctggtgcagc caggaggatc tctgagactg 60

tcttgcgccg ccagcggctt tacattcagc acctacacca tgtcttgggt ccggcaggct 120

ccaggcaaag gactcgagtg ggtggcctac atcagctacg gcggcggctc tacaagctac 180

ccagacaccg tgaagggcag gttcaccatc agccgggaca acgccaagaa cagcctgtac 240

ctgcagatga acagcctgag ggccgaggat accgccgtgt actattgcgc taggcacgtg 300

accgtggtgg agaacagcat ggactattgg ggccagggaa ccaccgtgac agtgtcttct 360

<210> 168
<211> 360
<212> DNA
<213> Artificial sequence

<220>
<223> 168

<400> 168
gaggtgcagc tggtggaatc aggaggagga ctggtgcagc caggaggatc tctgagactg 60

tcttgcgccg ccagcggctt tacattcagc acctacacca tgtcttgggt ccggcaggct 120

ccaggcaaag gactcgagtg ggtggcctac atcagctacg gcggcggctc tacaagctac 180

ccagacaccg tgaagggcag gttcaccatc agccgggaca acgccaagaa cagcctgtac 240

ctgcagatga acagcctgag ggccgaggat accgccgtgt actattgcac caggcacgtg 300

accgtggtgg agaacagcat ggactattgg ggccagggaa ccaccgtgac agtgtcttct 360

<210> 169
<211> 360
<212> DNA
<213> Artificial sequence

<220>
<223> 169

<400> 169
gaggtgcagc tggtggaatc aggaggagga ctggtgcagc caggaggatc tctgagactg 60

tcttgcgccg ccagcggctt tacattcagc acctacacca tgtcttgggt ccggcaggct 120

ccaggcaaaa gactcgagtg ggtggcctac atcagctacg gcggcggctc tacaagctac 180

ccagacaccg tgaagggcag gttcaccatc agccgggaca acgccaagaa cagcctgtac 240

ctgcagatga acagcctgag ggccgaggat accgccgtgt actattgcac caggcacgtg 300

accgtggtgg agaacagcat ggactattgg ggccagggaa ccaccgtgac agtgtcttct 360

<210> 170
<211> 360
<212> DNA
<213> Artificial sequence

<220>
<223> 170

<400> 170
gaggtgcagc tggtggaatc aggaggagga ctggtgcagc caggaggatc tctgagactg 60

tcttgcgccg ccagcggctt tacattcagc acctacacca tgtcttgggt ccggcaggct 120

ccagagaaga ggctcgagtg ggtggcctac atcagctacg gcggcggctc tacaagctac 180

ccagacaccg tgaagggcag gttcaccatc agccgggaca acgccaagaa cagcctgtac 240

ctgcagatga acagcctgag ggccgaggat accgccgtgt actattgcac aaggcacgtg 300

accgtggtgg agaacagcat ggactattgg ggccagggaa ccaccgtgac agtgtcttct 360

<210> 171
<211> 321
<212> DNA
<213> Artificial sequence

<220>
<223> 171

<400> 171
gagatcgtgc tgacccagag cccagacttc cagtcagtga cccccaagga gaaggtcacc 60

atcacttgca gagccagcca gagcatcggc accagcatcc attggtatca gcagaagccc 120

gaccagagcc ctaagctgct gatcaagtac gtgtccgaga gctttagcgg cgtgcctagc 180

agattcagcg gcagcggcag cggcacagac ttcaccctga ccatcaacag cctggaggca 240

gaggacgcag ccacctacta ttgccagcag acccactctt ggccttggac cttcggagga 300

ggcaccaagg tggagatcaa g 321

<210> 172
<211> 321
<212> DNA
<213> Artificial sequence

<220>
<223> 172

<400> 172
gagatcgtgc tgacccagag cccagacttc cagtcagtga cccccaagga gaaggtcacc 60

atcacttgca gagccagcca gagcatcggc accagcatcc attggtatca gcagaagccc 120

gaccagagcc ctaagctgct gatcaagtac gtgtccgaga gctttagcgg cacacctagc 180

agattcagcg gcagcggcag cggcacagac ttcaccctga ccatcaacag cctggaggca 240

gaggacgcag ccacctacta ttgccagcag acccactctt ggccttggac cttcggagga 300

ggcaccaagg tggagatcaa g 321

<210> 173
<211> 321
<212> DNA
<213> Artificial sequence

<220>
<223> 173

<400> 173
gacatcttgc tgacccagag cccagacttc cagtcagtga cccccaagga gaaggtcacc 60

atcacttgca gagccagcca gagcatcggc accagcatcc attggtatca gcagaagccc 120

gaccagagcc ctaagctgct gatcaagtac gtgtccgaga gctttagcgg cacccctagc 180

agattcagcg gcagcggcag cggcacagac ttcaccctga ccatcaacag cctggaggca 240

gaggacgcag ccacctacta ttgccagcag acccactctt ggccttggac cttcggagga 300

ggcaccaagg tggagatcaa g 321

<210> 174
<211> 321
<212> DNA
<213> Artificial sequence

<220>
<223> 174

<400> 174
gagatcgtgc tgacccagag cccagacttc cagtcagtga cccccaagga gaaggtcacc 60

atcacttgca gagccagcca gagcatcggc accagcatcc attggtatca gcagaagacc 120

gaccagagcc ctaagctgct gatcaagtac gtgtccgaga gctttagcgg cacccctagc 180

agattcagcg gcagcggcag cggcacagac ttcaccctga ccatcaacag cctggaggca 240

gaggacgcag ccacctacta ttgccagcag acccactctt ggccttggac cttcggagga 300

ggcaccaagg tggagatcaa g 321

<210> 175
<211> 321
<212> DNA
<213> Artificial sequence

<220>
<223> 175

<400>  175
gacatcttgc tgacccagag cccagacttc cagtcagtga cccccaagga gaaggtcacc        60

atcacttgca gagccagcca gagcatcggc accagcatcc attggtatca gcagaagacc       120

gaccagagcc ctaagctgct gatcaagtac gtgtccgaga gctttagcgg cacccctagc       180

agattcagcg gcagcggcag cggcacagac ttcaccctga ccatcaacag cctggaggca       240

gaggacgcag ccacctacta ttgccagcag acccactctt ggccttggac cttcggagga       300

ggcaccaagg tggagatcaa g                                                 321


<210>  176
<211>  42
<212>  DNA
<213>  Artificial sequence

<220>
<223>  2-1G11-VH-forward primer

<400>  176
gattcttaag ggtgtccagt gcgaggtgca gctggtggag tc                           42


<210>  177
<211>  41
<212>  DNA
<213>  Artificial sequence

<220>
<223>  2-1G11-VH-reverse primer

<400>  177
gatgggccct tggtcgaagc tgaggagacg gtgaccgtgg t                            41


<210>  178
<211>  43
<212>  DNA
<213>  Artificial sequence

<220>
<223>  2-1G11-VL-forward primer

<400>  178
tggttccccg gctcgcgatg cgaaattgtg atgactcagt ctc                          43


<210>  179
<211>  45
<212>  DNA
<213>  Artificial sequence

<220>
<223>  2-1G11-VL-reverse primer

<400>  179
cagatggtgc agccaccgta cgtttgatct ccaccttggt ccctc                        45

<210>  180
<211>  44
<212>  DNA
<213>  Artificial sequence

<220>
<223>  L9G7-VH-forward primer

<400>  180
gattcttaag ggtgtccagt gccaaatcca gctggtacaa tctg                        44


<210>  181
<211>  42
<212>  DNA
<213>  Artificial sequence

<220>
<223>  L9G7-VH-reverse primer

<400>  181
gatgggccct tggtcgaagc tgaggagacg gtgaccgtgg tc                          42


<210>  182
<211>  45
<212>  DNA
<213>  Artificial sequence

<220>
<223>  L9G7-VL-forward primer

<400>  182
tggttccccg gctcgcgatg cgaaattgtg ttgacgcagt ctcca                       45


<210>  183
<211>  45
<212>  DNA
<213>  Artificial sequence

<220>
<223>  L9G7-VL-reverse primer

<400>  183
cagatggtgc agccaccgta cgtttgatct ccaccttggt ccctc                       45


<210>  184
<211>  44
<212>  DNA
<213>  Artificial sequence

<220>
<223>  L1D12-VH-forward primer

<400>  184
gattcttaag ggtgtccagt gccaggtcca gctggtgcag tctg                        44


<210>  185
<211>  42
<212>  DNA

<213>  Artificial sequence

<220>
<223>  L1D12-VH-reverse primer

<400>  185
gatgggccct tggtcgaagc tgaggagacg gtgaccgtgg tc                          42


<210>  186
<211>  41
<212>  DNA
<213>  Artificial sequence

<220>
<223>  L1D12-VL-forward primer

<400>  186
actgcacagg gtctctctcc tcttctgagc tgactcagga c                           41


<210>  187
<211>  45
<212>  DNA
<213>  Artificial sequence

<220>
<223>  L1D12-VL-reverse primer

<400>  187
gagggggcag ccttgggttg acctaggacg gtcaacttgg tccct                       45